# FASCICULE DE BREVET EUROPEEN

(11) **EP 1 556 055 B1**
(45) Date de publication et mention de la délivrance du brevet: **29.10.2008**
(21) Numéro de dépôt: 03780257.6
(22) Date de dépôt: 30.10.2003
(51) Int. Cl.: A61K 31/5513, A61K 31/5517, A61P 25/08, A61P 25/16, A61P 25/18, A61P 25/22, A61P 25/24, A61P 25/28, A61P 25/30, A61P 11/06, A61P 21/00, A61P 19/02, A61P 3/04, A61P 37/00, A61P 25/20, C07D 243/14

(54) **INHIBITEURS DES PHOSPHODIESTERASES DES NUCLEOTIDES CYCLIQUES, AYANT UNE STRUCTURE BENZODIAZEPINIQUE, ET LEUR UTILISATION EN THERAPIE**
INHIBITOREN DER PHOSPHODIESTERASE DER ZYKLISCHEN NUCLEOTIDEN, MIT EINER BENZODIAZEPINISCHEN STRUKTUR, UND DEREN VERWENDUNG IN THERAPIE
CYCLIC NUCLEOTIDE PHOSPHODIESTERASE INHIBITORS HAVING A BENZODIAZEPINE STRUCTURE AND THEIR USE IN THERAPY

(30) Priorité: 30.10.2002 FR 0213607; 20.03.2003 US 455874 P
(43) Date de publication de la demande: 27.07.2005
(73) Titulaire: Via Pharmaceuticals, Inc., San Francisco, CA 94111 (US)
(72) Inventeur: BOURGUIGNON, Jean-Jacques, F-67150 Hipsheim (FR); LUGNIER, Claire, F-67000 Strasbourg (FR); ABARGHAZ, Mustapha, F-67200 Strasbourg (FR); LAGOUGE, Yan, F-67000 Strasbourg (FR); WAGNER, Patrick, F-67170 Brumath (FR); MONDADORI, Cesare, CH-4153 Reinach (CH); MACHER, Jean-Paul, F-68500 Bergholtz-Zell (FR); SCHULTZ, Dominique, F-67400 Illkirch (FR); RABOISSON, Pierre, Exton, PA-19341 (US)
(74) Mandataire: Tezier Herman, Béatrice
(86) Numéro de dépôt international: PCT/FR2003/003247
(87) Numéro de publication internationale: WO 2004/041258

(56) Documents cités:
- WO-A-99/58117
- WO-A-02/098865
- DE-A- 1 942 744
- DE-B- 1 136 709
- DE-B- 1 145 626
- GB-A- 1 346 176
- US-A- 3 515 755
- US-A- 3 573 282
- US-A- 3 609 146
- US-A- 3 723 433
- US-A- 3 803 129
- US-A- 4 056 525
- US-A- 5 010 076
- PARFITT, K.: "Martindale. The Complete Drug Reference" 1999, PHARNMACEUTICAL PRESS , LONDON, UK. , XP002245529 page 661 - page 668
- FRYER, R.I., ET AL.: "Quinazolines and 1,4-Benzodiazepins. XLV. 1,4-Benzodiazepines from 4-Isoquinolones." J. ORG. CHEM, vol. 35, no. 7, 1970, pages 2455-249, XP001149447
- KOLBACH, G., ET AL.: "Stereoselective in vitro aromatic-ring oxygenations of chiral 1,4-benzodiazepin-2-ones." HELVETICA CHIMICA ACTA, vol. 60, no. 1, 1977, pages 265-283, XP001149178
- STERNBACH, L.H., ET AL.: "Quinazolines and 1,4-Benzodiazepines. VI. Halo-, Methyl-, and Methoxy-substituted 1,3-dihydro-5-phenyl-2H-1,4-benzodiazepin- 2-ones." J. ORG. CHEM., vol. 27, 1962, pages 3788-3796, XP001149445
- DATABASE CA [Online] CHEMICAL ABSTRACTS SERVICE, COLUMBUS, OHIO, US; NAKAI, HIDEO ET AL: "1,4-Benzodiazepines" XP002245530 extrait de STN Database accession no. 72:79116 HCA & JP 44 026871 B (TANABE SEIYAKU CO., LTD.) 10 novembre 1969 (1969-11-10)
- DATABASE CA [Online] CHEMICAL ABSTRACTS SERVICE, COLUMBUS, OHIO, US; HASEGAWA, HITOSHI: "1,4-Benzodiazepine series compounds" XP002245531 extrait de STN Database accession no. 74:53864 HCA & JP 45 031303 B (JAPAN SYNTHETIC CHEMICAL INDUSTRY CO., LTD.) 9 octobre 1970 (1970-10-09)
- DATABASE WPI Section Ch, Week 198004 Derwent Publications Ltd., London, GB; Class B02, AN 1980-06578C XP002245532 & JP 54 157585 A (TAMURA Y) 12 décembre 1979 (1979-12-12)

## Description

L'invention concerne l'utilisation d'inhibiteurs de PDE2 de formule (I) pour traiter des désordres du système nerveux central et périphérique, un procédé de traitement thérapeutique par administration chez un animal de ces inhibiteurs. Elle concerne plus spécifiquement de nouveaux dérivés de benzodiazépines et leurs applications dans le domaine thérapeutique tout particulièrement pour le traitement de pathologies impliquant l'activité d'une phosphodiestérase de nucléotides cycliques. Elle concerne également des procédés pour leur préparation et de nouveaux intermédiaires de synthèse.

Les composés dont la synthèse est décrite dans la présente invention sont nouveaux et présentent des propriétés pharmacologiques très intéressantes : ce sont des inhibiteurs des phosphodiestérases des nucléotides cycliques et tout particulièrement de de la cGS-PDE (cGMP-Stimulated PDEs ou phosphodiestérase de type 2, PDE2), et à ce titre, ils présentent des applications thérapeutiques très intéressantes.

Les fonctions de la plupart des tissus sont modulées par des substances endogènes (hormones, transmetteurs, etc.) ou exogènes. Certaines de ces substances voient leur effet biologique relayé au niveau intracellulaire par des effecteurs enzymatiques, comme l'adénylate cyclase ou la guanylate cyclase. La stimulation de ces enzymes entraîne une élévation des taux intracellulaire d'AMP cyclique (AMPc) ou de GMP cyclique (GMPc), seconds messagers impliqués dans la régulation de nombreuses activités cellulaires. Ces nucléotides cycliques sont dégradés par une famille d'enzymes, les phosphodiestérases (PDE), divisée en au moins 7 groupes.

L'un d'entre eux, la PDE2, hydrolyse à la fois l'AMPc et le GMPc et est activable par le GMPc. La PDE2 répond sous des conditions physiologiques à des concentrations élevées en GMPc en augmentant le taux d'hydrolyse d'AMPc. Ce groupe est nommé PDE2 et est présent dans de nombreux tissus (adipocytes, surrénale, cerveau, coeur, foie, poumon, vaisseaux sanguins, etc.). Les inhibiteurs de PDE2 sont capables d'augmenter ou de maintenir à la fois les taux intracellulaires d'AMPc et de GMPc et à ce titre trouvent des applications dans le traitement de diverses pathologies.

L'art antérieur décrit des dérivés 1,4-benzodiapéine-2-one à titre de sédatifs, myorelaxants ou anticonvulsifs . Ainsi, DE 11 36 709, DE 11 45 626, US 3 515 755 et US 3 778 433 divulguent de tels dérivés qui sont différents des composés de la présente invention.

La demanderesse a maintenant mis en évidence les effets inhibiteurs de phosphodiestérases de nucléotides cycliques de certaines benzodiazépines, en particulier inhibiteurs de la PDE2. L'invention décrit également de nouveaux composés présentant une puissante activité inhibitrice de la PDE2, et possèdant préférentiellement un excellent profil de sélectivité vis-à-vis des autres isoformes de PDE, notamment une action faible sur la PDE3. Cette sélectivité peut s'étendre également à d'autres enzymes, telles que l'adénosine déaminase. Ainsi, l'invention décrit également de nouveaux composés présentant une puissante activité inhibitrice de la PDE2, et possèdant préférentiellement un excellent profil de sélectivité sur la PDE2 en comparaison avec l'adénosine déaminase. En outre, les composés préférés selon l'invention possèdent des effets centraux importants (anticonvulsivants, anxiolytiques, sédatifs, antidépresseurs) ou périphériques (antirhumatismaux, contre les maladies autoinflammatoires, contre les dysfonctionnement du foie dû à l'âge), et sont avantageusement dénués d'effets perturbateurs de la mémoire.

L'invention décrit l'utilisation d'au moins un inhibiteur de phosphodiestérase 2 selon la formule (I) pour la préparation d'une composition pharmaceutique destinée au traitement de pathologies liées au système nerveux (central et périphérique), en particulier central.

Plus spécifiquement, les pathologies sont celles dues à une dérégulation de la fonction d'un des neurotransmetteurs ou un déficit de libération d'un des neurotransmetteurs (e.g. dopamine, noradrenaline, acetlycholine, ...), en particulier de la dopamine, telles que plus spécifiquement une pathologie choisie parmi la dépression, la schizophrénie, l'anxiété, le désordre bipolaire, les désordres de défaut d'attention, les troubles du sommeil, les troubles obsessionnelles compulsifs (TOC ou en anglais : OCD - obsessive compulsive disorder), la fibromyalgie, syndrome de Tourette, pharmacodépendance (drogue, médicament, alcool, etc.), l'épilepsie, la maladie d'Alzheimer, la maladie de Parkinson, la sclérose latérale amyotrophique, la sclérose multiple, l'obésité et la démence des corps de Lewy (« Lewy body dementia »).

Les inhibiteurs de PDE2 selon la formule (I) peuvent également être utilisés selon l'invention pour traiter d'autres désordres impliquant le système nerveux périphérique et les organes périphériques en général, en particulier les pathologies de type natriurie réduite, insuffisance rénale aigüe, dysfonctionnement du foie, insuffisance hépatique aigüe, notamment dûs à l'âge, et les pathologies dues ou impliquant des dysfonctionnements de la libération de la prolactine, telles que le syndrome de la jambe sans repos, les désordres rhumatismaux, allergiques ou autoinflammatoires, tels que arthrite rhumatoïde, rhinite et asthme.

L'invention réside donc dans l'utilisation des inhibiteurs de PDE2 de formule (I) pour la préparation d'un médicament destiné au traitement de désordres du système nerveux, central ou périphérique, de nature chronique ou aiguë ou l'utilisation périphérique de ces inhibiteurs en tant que vasoconstricteurs.

Les inhibiteurs de PDE2 de formule (I) peuvent aussi être utilisés pour traiter l'anxiété, la dépression ou la schizophrénie.

Les inhibiteurs de l'activité ou de l'expression de phosphodiestérase de type PDE2 particulièrement utiles selon l'invention sont des composés qui présentent une activité inhibitrice sélective de PDE2, c'est à dire qu'ils présentent une activité inhibirice moindre sur les autres phosphodiestérases et notamment les PDE1, PDE3, PDE4 et PDE5. Certains des inhibiteurs de PDE2 sont particulièrement choisis dans le cadre de la présente invention pour leur sélectivité inhibitrice de PDE2 vis-à-vis de l'adénosine déaminase, c'est à dire qu'ils présentent une activité inhibitrice plus élevée pour PDE2 que pour l'adénosine déaminase.

Les inhibiteurs de PDE2 utilisés dans la présente invention peuvent être choisis parmi des composés dérivés de 1,4-benzodiazépine de formule (I).

Dans ce cadre, l'invention décrit également de nouveaux composés présentant une puissante activité inhibitrice de la PDE2. L'invention a ainsi pour objet des composés de formule générale (I) 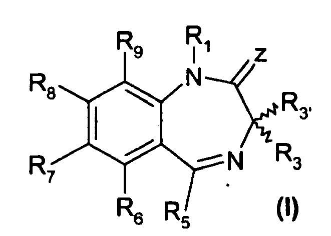 dans laquelle :
- Z représente un atome d'oxygène, de soufre ou un radical NR₂,
- R₁ est l'atome d'hydrogène ou un groupe (C₁-C₆) alkyle non substitué,
- R₂ est un atome d'hydrogène, un groupe (C₁-C₆) alkyle, un groupe (C₆-C₁₈)aryle ou un groupe (C₁-C₆)alkyl(C₆-C₁₈)aryle ou (C₆-C₁₈)aryl(C₁-C₄)alkyle,
   R₁ et R₂ pouvant éventuellement former ensemble une chaîne hydrocarbonée linéaire ou ramifiée ayant de 2 à 6 atomes de carbone, comportant éventuellement une autre ou plusieurs autres doubles liaisons et/ou éventuellement interrompue par un atome d'oxygène, de soufre ou d'azote,
- R₃ et R_{3'}, identiques ou différents, représentent l'atome d'hydrogène, un groupe (C₁-C₁₂) alkyle, (C₃-C₆) cycloalkyle, (C₆-C₁₈) aryle, (C₆-C₁₈)aryl(C₁-C₄)alkyle, (C₁-C₁₂)alkyl(C₆-C₁₈)aryle ou un hétérocycle en (C₅-C₁₈), aromatique ou non, comportant 1 à 3 hétéroatomes, un groupe NO₂, CF₃, CN, NR'R", SR', OR', COOR', CONR'R" ou NHCOR'R", R' et R", indépendamment l'un de l'autre, étant choisis parmi l'atome d'hydrogène, un groupe (C₁-C₆) alkyle, (C₃-C₆) cycloalkyle, (C₆-C₁₂) aryle, et un hétérocycle en (C₅-C₁₂), aromatique ou non, comportant 1 à 3 hétéroatomes ;
- R₅ représente un groupe phényle au moins substitué en position 3, un groupe naphtyle, un hétérocycle en (C₅-C₁₈), aromatique ou non, comportant 1 à 3 hétéroatomes, choisi parmi le radical pyridyle, isoquinolyle, quinolyle et piperazinyle, avec les conditions que lorsque R₅ est un groupe naphtyle substitué en position 6, ce dernier n'est alors pas lié au reste de la molécule en position 2, ou lorsque R5 est un groupement pyridyle, il n'est alors pas lié au reste de la molécule en position 4, ou lorsque R5 est un groupement tetrahydro 1,2,3,4-isoquinolyle, il n'est alors pas lié au reste de la molécule en position 2,
   lorsque R₅ représente un groupe phényle au moins substitué en position 3, ledit substituant étant choisi parmi : un radical alkyle, halogénoalkyle, cycloalkyle, alkényle, alkynyle, aralkyle, aryle, hétérocycle, hétérocycloalkyle, un groupe OH, =O, NO₂, NH₂, CN, CF₃, COR', COOR', (C₁-C₆)alkoxy, (di)(C₁-C₆)alkylamino, NHCOR', CONR'R", dans lesquels R' et R" sont tels que définis ci-avant, CHO, CONH2, phényle éventuellement substitué, notamment par un radical acétyle, par un atome d'halogène (CI), par un groupe CONH2 ou par un groupe CN, prop-1-ynyl éventuellement substitué, notamment par un radical benzyloxy ou tert-butyl carbamate, hex-1-ynyl éventuellement substitué, notamment par un groupe CN ou NH2, pentyle éventuellement substitué, notamment par un groupe CONH2, hexyl, pipéridinyle éventuellement substitué, notamment par un radical prop-1-ynyle, benzylaminométhyl, acétamide (CH3CONH), aminométhyl, NH2CS-, 4-phényl-1, 3-thiazol-2-yl, -CONHBenzyl, -COOEthyl, -CONHiPropyl, -CONH-(CH2)n-CONH2 (n représentant un entier de 1 à 6), -CONR'R", avec R' et R", identiques ou différents, représentant un radical alkyle de C1-C6 ou un atome d'hydrogène, -(4-benzylpypérazin-1-yl)carbonyl, -CONH-(CH2)n-phényl (n représentant un entier de 1 à 6), imidazolyle, pipérazinyle éventuellement substitué, notamment par un radical phényle,
- R₇ et R₈, indépendamment l'un de l'autre, représentent un groupe OR₁₀, dans lequel R₁₀ est un groupe (C₁-C₆) alkyle, de préférence un groupe éthyle ou méthyle, avantageusement méthyle, ou R₇ et R₈ représentent tous deux un groupe éthoxy ou méthoxy, avantageusement méthoxy, ou l'un représente un atome d'hydrogène et l'autre un groupe éthoxy ou méthoxy, avantageusement méthoxy,
- R₆ est choisi parmi l'atome d'hydrogène, un atome d'halogène, un radical alkyle, cycloalkyle, alkényle, alkynyle, un groupe aryle, aralkyle, hétérocycle, hétérocycloalkyle et un groupe OR₁₀, R₁₀ étant tel que défini ci-avant,
le groupe alkyle, cycloalkyle, alkényle, alkynyle, aralkyle, aryle, phényle, naphtyle, hétérocycle, hétérocycloalkyle ou la chaîne hydrocarbonée définie ci-dessus étant éventuellement substitué par un ou plusieurs substituants, identiques ou différents, choisis de préférence parmi un atome d'halogène, un radical alkyle, halogénoalkyle, cycloalkyle, alkényle, alkynyle, aralkyle, aryle, hétérocycle, hétérocycloalkyle, un groupe OH, =O, NO₂, NH₂, CN, CF₃, COR', COOR', (C₁-C₆)alkoxy, (di)(C₁-C₆)alkylamino, NHCOR' et CONR'R", dans lesquels R' et R" sont tels que définis ci-avant, les substituants pouvant être également substitués,
R₉ est un atome d'hydrogène,
et les sels des composés de formule (I),
à l'exclusion des composés de formule (I) dans laquelle.
- R1 est un radical alkyle, R3 et R'3 sont des atomes d'hydrogène, R6 est un atome d'hydrogène, R5 est un radical phényle au moins substitué en position 3 par un groupement méthoxy,
- R1 est un radical alkyle ou un atome d'hydrogène, R3 et R'3 sont des atomes d'hydrogène, R6 est un atome d'hydrogène, R5 est un radical phényle uniquement substitué en position 3 par un atome de chlore ou de brome,
- R1 est un radical alkyle, R3 et R'3 sont des atomes d'hydrogène, R6 est un atome d'hydrogène, R5 est un radical phényle au moins substitué en position 3 par un groupement CH2OH,
- R1 est un atome d'hydrogène, R3 et R'3 sont des atomes d'hydrogène, R6 est un atome d'hydrogène, R5 est un radical phényle uniquement substitué en position 3 par un groupement CF3,
- R1 est un radical alkyle, R3 et R'3 sont des atomes d'hydrogène, R6 est un atome d'hydrogène, R5 est un radical phényle substitué en positions 3 et 5 par un groupement CF3,
- R1 est un radical alkyle, R3 et R'3 sont des atomes d'hydrogène, R6 est un atome d'hydrogène, R7 et R8 sont des radicaux methoxy, R5 est un radical phényle substitué en positions 3 par un groupement phényle,
- R1 est un radical alkyle, R3 et R'3 sont des atomes d'hydrogène, R6 est un atome d'hydrogène, R7 et R8 sont des radicaux methoxy, R5 est un radical phényle substitué en positions 3 par un groupement phényléthynyle.

L'invention concerne également des compositions pharmaceutiques comprenant un ou plusieurs composés de formule générale (I) telle que définie ci-avant, et un véhicule ou un excipient acceptable sur le plan pharmaceutique.

L'invention concerne également l'utilisation des composés de formule générale (I) telle que définie ci-avant pour la préparation d'une composition pharmaceutique destinée à l'inhibition d'une phosphodiestérase des nucléotides cycliques, notamment de la phosphodiestérase 2 (PDE2). L'invention concerne plus particulièrement l'utilisation des composés ci-dessus pour le traitement des pathologies impliquant une dérégulation des taux intracellulaires d'AMP cyclique et/ou de GMP cyclique.

Selon l'invention, le terme "alkyle" désigne un radical hydrocarboné linéaire ou ramifié ayant avantageusement de 1 à 12 atomes de carbone, tel que méthyle, éthyle, propyle, isopropyle, butyle, isobutyle, *tert*-butyle, pentyle, néopentyle, n-hexyle, n-décyle, n-dodécyle, etc. Les groupes en C₁-C₆ sont préférés. Les groupes alkyles peuvent être substitués par un groupe aryle tel que défini ci-après, auquel cas on parle de groupe arylalkyle (ou aralkyle). Des exemples de groupes arylalkyle sont notamment benzyle et phénétyle.

Le terme « cycloalkyle » désigne un système hydrocarboné cyclique, pouvant comprendre avantageusement de 3-6 atomes de carbone et être mono- ou poly-cyclique. On peut citer notamment les groupes cyclopropyle et cyclohexyle.

Les groupes « aryle » sont des systèmes hydrocarbonés aromatiques mono-, bi- ou tri-cycliques, préférentiellement des systèmes hydrocarbonés aromatiques monocycliques ou bi-cycliques ayant de 6 à 18 atomes de carbone, encore plus préférentiellement 6 atomes de carbone. On peut citer par exemple les groupes phényle, naphtyle et bi-phényle.

Les groupes « hétérocycles » désignent des systèmes hydrocarbonés aromatiques ou non comprenant un ou plusieurs hétéroatomes cycliques. Il s'agit préférentiellement de systèmes hydrocarbonés cycliques comportant de 5 à 18 atomes de carbone et 1 ou plusieurs hétéroatomes cycliques, notamment de 1 à 3 ou à 4 hétéroatomes cycliques choisis parmi N, O ou S. Parmi les groupes hétérocycliques aromatiques (hétéroaryles) préférés, on peut citer notamment les groupes thiényle, benzothiényle, benzofuryle, pyridyle, pyrimidinyle, pyridazinyl, isoquinolyle, quinolyle, thiazolyle, furyle, pyranyle, pyrrolyle, 2*H*-pyrrolyle, imidazolyle, benzymidazolyle, pyrazolyle, isothiazolyle, isoxazolyle et indolyle. Parmi les groupes hétérocycliques non-aromatiques préférés, on peut citer notamment les groupes morpholino, pipéridinyle, pipérazinyle et pyrrolidinyle.

Les groupes aryles et hétérocycles peuvent éventuellement être substitués par un groupe alkyle, alkényle ou alkynyle. Dans le cas d'un aryle ou d'un hétérocycle substitué par un groupe alkyle, on parle de groupe alkylaryle ou alkylhétérocycle. Des exemples de groupes alkylaryle sont notamment tolyle, mésythyle et xylyle. Dans le cas, d'un aryle ou d'un hétérocycle substitué par un groupe alkényle, on parle de groupe alkénylaryle ou alkénylhétérocycle. Des exemples de groupes alkénylaryle sont notamment le groupe cinnamyle. Dans le cas, d'un aryle ou d'un hétérocycle substitué par un groupe alkynyle, on parle de groupe alkynylaryle ou alkynylhétérocycle.

Les groupes aryles et hétérocycles peuvent également être substitués par un groupe choisi indépendamment parmi les groupes aryle ou hétérocycle, eux même éventuellement substitués par un ou plusieurs substituants choisis de préférence parmi un atome d'halogène ou un groupe NO₂, CN, CF₃, OR', COR', COOR', alkoxy, NHCOR' et CONR'R", R' et R" étant tels que définis ci-avant.

Des exemples de groupes aryles et hétérocycles substitués par un groupe aryle ou hétérocycle sont notamment les groupes benzothiényle, benzofuryle, furylphényle, benzyloxynaphtyle, pyridylphényle, phénylphényle et thiénylphényle. Comme indiqué, les groupes ci-dessus peuvent être substitués. On peut citer à cet égard les groupes phényle substitués par un groupe phényle lui-même substitué par un atome d'halogène, un groupe NO₂, CF₃, méthoxy ou méthyle.

Les groupes « alkényles » sont des radicaux hydrocarbonés linéaires ou ramifiés comportant une ou plusieurs double-liaisons. Ils comportent avantageusement de 2 à 6 atomes de carbone et, préférentiellement, 1 ou 2 double-liaisons. Les groupes alkényles peuvent être substitués par un groupe aryle tel que défini ci-avant, auquel cas on parle de groupe arylalkényle.

Les groupes « alkynyles » sont des radicaux hydrocarbonés linéaires ou ramifiés comportant une ou plusieurs triple-liaisons. Ils comportent avantageusement de 2 à 6 atomes de carbone et, préférentiellement, 1 ou 2 double-liaisons. Les groupes alkynyles peuvent être substitués par un groupe aryle tel que défini ci-avant, auquel cas on parle de groupe arylalkynyle.

Les groupes « alkoxy » correspondent aux groupes alkyle et cycloalkyle définis ci-dessus reliés au noyau par l'intermédiaire d'une liaison -O- (éther). On préfère tout particulièrement les groupes méthoxy ou éthoxy.

Par « halogène », on entend un atome de fluor, de chlore, de brome ou d'iode.

Par « hétéroatome », on entend un atome choisi parmi O, N et S.

L'invention a tout particulièrement pour objet des composés de formule générale (I) ci-avant dans laquelle R₅ est un radical phényle au moins substitué en position 3 tel que défini ci-avant. De tels composés possèdent des propriétés d'inhibition particulièrement prononcée et préférentielle de la phosphodiestérase 2.

Les groupes substituants peuvent être choisis, par exemple, parmi : CHO, CN, CONH2, NO2, CF3, NH2, atome d'halogène (Cl), (C1-C6)alkyle, phényle éventuellement substitué, notamment par un radical acétyle, par un atome d'halogène (Cl), par un groupe CONH2 ou par un groupe CN, prop-1-ynyl éventuellement substitué, notamment par un radical benzyloxy ou tert-butyl carbamate, hex-1-ynyl éventuellement substitué, notamment par un groupe CN ou NH2, pentyle éventuellement substitué, notamment par un groupe CONH2, hexyl, pipéridinyle éventuellement substitué, notamment par un radical prop-1-ynyle, benzylaminométhyl, acétamide (CH3CONH), aminométhyl, NH2CS-, 4-phényl-1, 3-thiazol-2-yl, -CONHBenzyl, -COOEthyl, -CONHiPropyl, -CONH-(CH2)n-CONH2 (n représentant un entier de 1 à 6), -CONR'R", avec R' et R", identiques ou différents, représentant un radical alkyle de C1-C6 ou un atome d'hydrogène, -(4-benzylpypérazin-1-yl)carbonyl, -CONH-(CH2)n-phényl (n représentant un entier de 1 à 6), imidazolyle, pipérazinyle éventuellement substitué, notamment par un radical phényle.
Parmi les composés de formule (I) avec R5 étant un radical phényle au moins substitué en position 3, on peut également citer les composés de formule (I) dans laquelle R5 est un radical phényle substitué en positions 3 et 4, notamment par une chaîne hydrocarboné comportant éventuellement au moins un hétéroatome, tel que l'oxygène, comme la chaîne méthylènedioxy (-O-CH2-O-) formant un cycle avec le radical phényl auquel il est rattaché.

L'invention a également pour objet particulier des composés de formule générale (I) ci-avant dans laquelle R5 est le radical 3-pyridyle, 4-isoquinolyle, pipérazinyle éventuellement substitué, notamment en position 4 par un groupement aryle, tel que le phényl.

L'invention a également pour objet particulier des composés de formule générale (I) ci-avant dans laquelle Z représente un atome de soufre ou -NR2, avec de préférence R2 formant un cycle avec R1 de type imidazole.
Des composés particuliers au sens de l'invention sont ceux dans lesquels :
- Z est l'atome d'oxygène et/ou
- R₆ représente l'atome d'hydrogène, un atome d'halogène, un radical phényle, un groupe (C₁-C₆) alkyle ou un groupe OR₁₀ dans lequel R₁₀ est un groupe (C₁-C₆) alkyle, de préférence un groupe éthyle ou méthyle, et/ou
- R₃ et R₃', identiques ou différents, représentent un atome d'hydrogène, et/ou
- R₁ est un groupe (C1-C6)alkyle non substitué.

Une famille de composés particulière est représentée par les composés de formule générale (II) telle que définie ci-avant dans laquelle R₃ et R₃' représentent l'atome d'hydrogène.

Une autre famille comprend les composés de formule générale (I) dans laquelle Z est l'atome d'oxygène, R₇ et R₈ représentent, indépendamment l'un de l'autre, un groupe OR₂ dans lequel R₂ est un groupe (C₁-C₆) alkyl, R₁ représente l'atome d'hydrogène ou un groupe (C₁-C₆) alkyle, R₆ représente l'atome d'hydrogène et R₃ et R₃' représentent l'atome d'hydrogène.

Une autre famille comprend les composés de formule générale (I) dans laquelle Z est l'atome d'oxygène, R₇ et R₈ représentent, indépendamment l'un de l'autre, un groupe OR₂ dans lequel R₂ est un groupe (C₁-C₆) alkyle, R₆ représente l'atome d'hydrogène, un atome d'halogène ou un groupe (C₁-C₆) alkyle et R₁ représente un groupe (C₁-C₁₂) alkyle.

Selon un aspect particulier de l'invention, les composés de formule générale (I) ci-avant sont ceux dans laquelle au moins un des groupements R7 et R8, avantageusement les deux, représente un radical OR₁₀ avec R₁₀ représente un groupe (C₁-C₆) alkyle. De manière préférée, dans les composés de formule générale (I) selon l'invention et dans les familles particulières mentionnées ci-avant, au moins un des groupes R₇ et R₈ représentent, indépendamment l'un de l'autre, un groupe méthoxy ou éthoxy, avantageusement méthoxy, plus préférentiellement, ils représentent tous deux un groupe méthoxy ou éthoxy, avantageusement méthoxy.

De manière préférée, dans les composés de formule générale (I) selon l'invention et dans les familles particulières mentionnées ci-avant, les groupes R₃ et R₃', égaux ou différents, représentent un atome d'hydrogène ou un groupe méthyle, éthyle ou n-propyle. Selon une variante particulièrement avantageuse, dans les composés de formule générale (I) selon l'invention et dans les familles particulières mentionnées ci-avant, les groupes R₃ et R₃' représentent un atome d'hydrogène.

Comme indiqué, dans les composés de formule générale (I) selon l'invention et dans les familles particulières mentionnées ci-avant, R₅ est avantageusement un radical phényle au moins substitué en position 3.

Selon une première variante de l'invention, R₅ est un groupe phényle substitué par :
(a) un ou plusieurs groupes OR', en particulier méthoxy ou éthoxy, ou
(b) un groupe COR', en particulier acétyle ou aldéhyde, ou
(c) un groupe CONR'R", en particulier CONH2,ou
(d) un groupe CN, ou
(e) un groupe trifluorométhyle, ou
(f) un groupe alkyle, par exemple méthyle, ou alkynyle, par exemple hexynyle ou propynyle, ou
(g) un groupe aryle ou hétérocycle, notamment un groupe phényle, furyle, pyridyle, pipéridine, thiazole ou thiényle, ledit aryle ou hétérocycle étant lui-même éventuellement substitué par un ou plusieurs groupes choisis de préférence parmi les groupes (a)-(g).

Les composés tout particulièrement préférés sont choisis parmi les composés suivants :
3-(7,8-Diméthoxy-2-oxo-2,3-dihydro-1H-1,4-benzodiazépin-5-yl)benzonitrile , **3a**
3-(7,8-diméthoxy-1-méthyl-2-oxo-2,3-dihydro-1*H*-1,4-benzodiazépin-5-yl)-benzonitrile, **4a**
3-[1-(4-chlorobenzyl)-7,8-diméthoxy-2-oxo-2,3-dihydro-1*H*-1,4-benzodiazépin-5-yl]-benzonitrile, **4c**
3-(1-éthyl-7,8-dimethoxy-2-oxo-2,3-dihydro-1H-1,4-benzodiazépin-5-yl)benzonitrile, **4k**
3-(7,8-diméthoxy-1-propyl-2-oxo-2,3-dihydro-1H-1,4-benzodiazépin-5-yl)benzonitrile, **4l**
7,8-Diméthoxy-1-méthyl-[5-(3-trifluorométhyl)phényl]-1,3-dihydro-2*H*-1,4-benzodiazépin-2-one **4p**
7,8-diméthoxy-1-éthyl-5-[3-(trifluorométhyl)phényl]-1,3-dihydro-2H-1,4-benzodiazépin-2-one **4q**
5-[3-(trifluorométhyl)phényl]-7,8-diméthoxy-1-*n*-propyl-1,3-dihydro-1,4-benzodiazépin-2-one, **4r**
3-(7,8-Diméthoxy-1-méthyl-2-oxo-2,3-dihydro-1*H*-1,4-benzodiazépin-5-yl)-benzamide **5a**
3-(6-Bromo-7,8-diméthoxy-1-méthyl-2-oxo-2,3-dihydro-1*H*-1,4-benzodiazépin-5-yl)benzamide, **5b**
3-(7,8-Diméthoxy-1-methyl-2-oxo-6-phényl-2,3-dihydro-1*H*-1,4-benzodiazépin-5-yl)benzamide, **5c**
3-(7,8-diméthoxy-2-oxo-2,3-dihydro-1H-1,4-benzodiazépin-5-yl)benzamide, **5e**
3-(7,8-diméthoxy-1-propyl-2-oxo-2,3-dihydro-1H-1,4-benzodiazépin-5-yl)benzamide, **5f**
3-(1-éthyl-7,8-diméthoxy-2-oxo-2,3-dihydro-1H-1,4-benzodiazépin-5-yl)benzamide, **5g**
3-(7,8-diméthoxy-1,3-diméthyl-2-oxo-2,3-dihydro-1H-1,4-benzodiazépin-5-yl) benzamide, **5j**
3-[3-(3,4-dichlorobenzyl)-7,8-diméthoxy-1-méthyl-2-oxo-2,3-dihydro-1H-1,4-benzodiazépin-5-yl]benzamide, **5k**
3-(8-méthoxy-1-méthyl-2-oxo-2,3-dihydro-1H-1,4-benzodiazépin-5-yl)benzamide, **5l**
3-(6,8-Dimethoxy-2-oxo-2,3-dihydro-1*H*-1,4-benzodiazepin-5-yl)benzamide, **5n**
3-(6,8-Dimethoxy-1-méthyl-2-oxo-2,3-dihydro-1*H*-1,4-benzodiazépin-5-yl)benzamide, **5o**
*Tert*-butyl 3-[3-(7,8-diméthoxy-1-méthyl-2-oxo-2,3-dihydro-1*H*-1,4-benzodiazépin-5-yl)phényl]propynylcarbamate, **6a**
7,8-Diméthoxy-5-(3'-hex-1-ynylphényl)-1-*N*-méthyl-1,3-dihydro-2*H*-1,4-benzodiazépin-2-one, **6b**
7,8-Diméthoxy-1-méthyl-5-[3-(3-pipéridin-1-ylprop-1-ynyl)phényl]-1,3-dihydro-2*H-*1,4-benzodiazépin-2-one, **6c**
6-[3-(7,8-Diméthoxy-1-méthyl-2-oxo-2,3-dihydro-1*H*-1,4-benzodiazépin-5-yl)phényl]hex-5-ynenitrile, **6d**
7,8-Diméthoxy-5-(3'-hexylphényl)-1-*N*-méthyl-1,3-dihydro-2*H*-1,4-benzodiazépin-2-one, **6e**
5-[3-(3-aminopropyl)phényl-7,8-diméthoxy-1-méthyl-1,3-dihydro-2H-1,4-benzodiazépin-2-one trifluoroacétate, **6h**
6-[3-(7,8-diméthoxy-1-méthyl-2-oxo-2,3-dihydro-1*H*-1,4-benzodiazépin-5-yl)phényl]hexanamide, **6i**
5-(4'-chloro-1,1'-biphényl-3-yl)-7,8-diméthoxy-1-méthyl-1,3-dihydro-2H-1,4-benzodiazépin-2-one, **6j**
5- {3-[3-(benzyloxy)prop-1-ynyl]phényl}-1-éthyl-7,8-diméthoxy-1,3-dihydro-2H-1,4-benzodiazépin-2-one, **6k**
3'-(7,8-diméthoxy-1-méthyl-2-oxo-2,3-dihydro-1H-1,4-benzodiazépin-5-yl)-1,1'-biphényl-3-carbonitrile, **6l**
3'-(7,8-diméthoxy-1-méthyl-2-oxo-2,3-dihydro-1H-1,4-benzodiazépin-5-yl)-1,1'-biphényl-4-carbonitrile, **6m**
3'-(7,8-diméthoxy-1-méthyl-2-oxo-2,3-dihydro-1H-1,4-benzodiazépin-5-yl)-1,1'-biphényl-4-carboxamide, **6n**
3'-(7,8-diméthoxy-1-méthyl-2-oxo-2,3-dihydro-1H-1,4-benzodiazépin-5-yl)-1,1'-biphényl-3-carboxamide, **6o**
3-[3-(3,4-dichlorobenzyl)-7,8-diméthoxy-1-méthyl-2-oxo-2,3-dihydro-1H-1,4-benzodiazépin-5-yl]benzonitrile, **7b**
7,8-diméthoxy-1,3-diméthyl-5-(3-trifluorométhylphényl)-1,3-dihydro-2H-1,4-benzodiazépin-2-one, **7c**
3-(7,8-diméthoxy-1,3-diméthyl-2-oxo-2,3-dihydro-1H-1,4-benzodiazépin-5-yl)benzonitrile, **7d**
5-[3-(aminométhyl)phényl]-7,8-diméthoxy-1-méthyl-1,3-dihydro-2H-1,4-benzodiazépin-2-one, **8a**
N-[3-(7,8-diméthoxy-1-méthyl-2-oxo-2,3-dihydro-1H-1,4-benzodiazépin-5-yl)benzyl]acétamide, **8b**
3-(7,8-diméthoxy-1-méthyl-2-oxo-2,3-dihydro-1H-1,4-benzodiazépin-5-yl)thiobenzamide, **9a**
7,8-diméthoxy-1-méthyl-5-[3-(4-phényl-1, 3-thiazol-2-yl)phényl]-1,3-dihydro-2H-1,4-benzodiazépin-2-one, **9b**
5-(3-cyanophényl)-7,8-diméthoxy-1,3-dihydro-2H-1,4-benzodiazépin-2-thione, **10d**
3-(7,8-diméthoxy-2-méthylamino-1,3-dihydro-3H-1,4-benzodiazépin-5-yl)benzonitrile, **12a**
7,8-diméthoxy-1-méthyl-5-(3-pyridyl)-1,3-dihydro-1,4-benzodiazépin-2-one, **17b**
7,8-diméthoxy-1-méthyl-5-(3-nitrophényl)-1,3-dihydro-1,4-benzodiazépin-2-one, **17c**
5-(7,8-diméthoxy-1-méthyl-2-oxo-2,3-dihydro-1H-1,4-benzodiazépin-5-yl)-2-benzonitrile, **17d**
5-(3-acétylphényl)-7,8-diméthoxy-1-méthyl-1,3-dihydro-1,4-benzodiazépin-2-one, **17e**
5-(4-isoquinoléinyl)-7,8-diméthoxy-1-méthyl-1,3-dihydro-1,4-benzodiazépin-2-one, **17f**
7,8-diméthoxy-5-(3-hydroxyméthylphényl)-1-méthyl-3-propyl-1,3-dihydro-2H-1,4-benzodiazépin-2-one, **17h**
5-(3-aminophényl)-7,8-diméthoxy-1-méthyl-1,3-dihydro-2H-1,4-benzodiazépin-2-one, **17i**
5-(3,4-dichlorophényl)-7,8-diméthoxy-1-méthyl-1,3-dihydro-1,4-benzodiazépin-2-one, **17j**
7,8-diméthoxy-1-méthyl-5-(3-méthylphényl)-1,3-dihydro-1,4-benzodiazépin-2-one, **17k.**
5-(3-formylphényl)-7,8-diméthoxy-1-méthyl-1,3-dihydro-1,4-benzodiazépin-2-one, **17l**
Chlorhydrate de la 5-[3-(benzylaminométhyl)phényl]-7,8-diméthoxy-1-méthyl-1,3-dihydro-2H-1,4-benzodiazépin-2-one, **17m**
N-[3-(7,8-diméthoxy-1-méthyl-2-oxo-2,3-dihydro-1H-1,4-benzodiazépin-5-yl) phényl]acétamide, **17n**
7,8-diméthoxy-1-méthyl-5-(3,4-méthylènedioxyphényl)-1,3-dihydro-2H-1,4-benzodiazépin-2-one, **17o**
3-(6-Bromo-7,8-diméthoxy-1-méthyl-2-oxo-2,3-dihydro-1*H*-1,4-benzodiazépin-5-yl)benzonitrile, **24b**
3-(7,8-Diméthoxy-1-methyl-2-oxo-6-phényl-2,3-dihydro-1*H*-1,4-benzodiazépin-5-yl)benzonitrile, **25b**
Tert-butyl 3-[5-(cyanophényl)-(7,8-diméthoxy-1-méthyl-2-oxo-2,3-dihydro-1*H*-1,4-benzodiazépin-6-yl)phényl]prop-2-ynylcarbamate, **25e**
[6-(3-aminoethynyl)-7,8-diméthoxy-1-méthyl-2-oxo-2,3-dihydro-1*H-*1,4-benzodiazépin-5-yl]benzonitrile, **25g**
3-(8-méthoxy-2-oxo-2,3-dihydro-1H-1,4-benzodiazépin-5-yl)benzonitrile, **28a**
3-(7-méthoxy-2-oxo-2,3-dihydro-1H-1,4-benzodiazépin-5-yl)benzonitrile, **28c**
3-(6,8-Dimethoxy-2-oxo-2,3-dihydro-1*H*-1,4-benzodiazepin-5-yl)benzonitrile, **28g**
3-(8-méthoxy-1-méthyl-2-oxo-2,3-dihydro-1H-1,4-benzodiazépin-5-yl)benzonitrile, **29a**
3-(6,8-Dimethoxy-1-methyl-2-oxo-2,3-dihydro-1*H*-1,4-benzodiazepin-5-yl)benzonitrile, **29b**
Benzoate de 3-(7,8-diméthoxy-1-méthyl-2-oxo-2,3-dihydro-1H-1,4-benzodiazépin-5-yl)méthyl, **34a**
Acide 3-(7,8-diméthoxy-1-méthyl-2-oxo-2,3-dihydro-1H-1,4-benzodiazépin-5-yl) benzoïque, **35a**
3-(7,8-diméthoxy-1-méthyl-2-oxo-2,3-dihydro-1H-1,4-benzodiazépin-5-yl)N-isopropylbenzamide, **36a**
N-benzyl-3-(7,8-diméthoxy-1-méthyl-2-oxo-2,3-dihydro-1H-1,4-benzodiazépin-5-yl) benzamide, **36b**
N-(6-amino-6-oxohexyl)-3-(7,8-diméthoxy-1-méthyl-2-oxo-2,3-dihydro-1H-1,4-benzodiazépin-5-yl) benzamide, **36c**
3-(7,8-diméthoxy-1-méthyl-2-oxo-2,3-dihydro-1H-1,4-benzodiazépin-5-yl) N, N-diméthylbenzamide **36d**
5- {3-[(4-benzylpypérazin-1-yl)carbonyl]phényl}7,8-diméthoxy-1-méthyl-2-oxo-2,3-dihydro-1H-1,4-benzodiazépin-2-one, **36e**
3-(7,8-diméthoxy-1-méthyl-2-oxo-2,3-dihydro-1H-1,4-benzodiazépin-5-yl) N-(3-phénylpropyl)benzamide, **36f**

Les composés particulièrement préférés sont choisis parmi les composés suivants :
7,8-Diméthoxy-1-méthyl-[5-(3-trifluorométhyl)phényl]-1,3-dihydro-2*H*-1,4-benzodiazépin-2-one **4p**
3-(7,8-Diméthoxy-1-méthyl-2-oxo-2,3-dihydro-1*H*-1,4-benzodiazépin-5-yl)-benzamide **5a**
3-(6-Bromo-7,8-diméthoxy-1-méthyl-2-oxo-2,3-dihydro-1*H*-1,4-benzodiazépin-5-yl)benzamide, **5b**
Tert-butyl 3-[3-(7,8-diméthoxy-1-méthyl-2-oxo-2,3-dihydro-1*H*-1,4-benzodiazépin-5-yl)phényl]propynylcarbamate, **6a**
7,8-Diméthoxy-5-(3'-hex-1-ynylphényl)-1-*N*-méthyl-1,3-dihydro-2*H*-1,4-benzodiazépin-2-one, **6b**
6-[3-(7,8-Diméthoxy-1-méthyl-2-oxo-2,3-dihydro-1*H*-1,4-benzodiazépin-5-yl)phényl]hex-5-ynenitrile, **6d**
7,8-Diméthoxy-5-(3'-hexylphényl)-1-*N*-méthyl-1,3-dihydro-2*H*-1,4-benzodiazépin-2-one, **6e**
5-(4'-chloro-1,1'-biphényl-3-yl)-7,8-diméthoxy-1-méthyl-1,3-dihydro-2H-1,4-benzodiazépin-2-one, **6j**
3'-(7,8-diméthoxy-1-méthyl-2-oxo-2,3-dihydro-1H-1,4-benzodiazépin-5-yl)-1,1'-biphényl-4-carbonitrile, **6m**
3'-(7,8-diméthoxy-1-méthyl-2-oxo-2,3-dihydro-1H-1,4-benzodiazépin-5-yl)-1,1'-biphényl-4-carboxamide, **6n**
7,8-diméthoxy-1-méthyl-5-[3-(4-phényl-1, 3-thiazol-2-yl)phényl]-1,3-dihydro-2H-1,4-benzodiazépin-2-one, 9b
7,8-diméthoxy-1-méthyl-5-(3-pyridyl)-1,3-dihydro-1,4-benzodiazépin-2-one, **17b**

Les composés de l'invention peuvent être sous forme de sels, notamment de sels d'addition basiques ou acides, préférentiellement compatibles avec un usage pharmaceutique. Parmi les acides pharmaceutiquement acceptables, on peut citer, les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, méthane ou éthanesulfonique, camphorique, etc. Parmi les bases pharmaceutiquement acceptables, on peut citer l'hydroxyde de sodium, l'hydroxyde de potassium, la triéthylamine, la *tert-*butylamine, etc.

L'invention a également pour objet une composition, en particulier pharmaceutique comprenant un composé tel que défini ci-dessus, en particulier en association avec un véhicule ou un excipient acceptable sur le plan pharmaceutique.

Les inhibiteurs de PDE2 de formule (I) ou les compositions selon l'invention peuvent être administrés de différentes manières et sous différentes formes. Ainsi, ils peuvent être administrés de manière systémique, par voie orale, par inhalation ou par injection, comme par exemple par voie intraveineuse, intra-musculaire, sous-cutanée, trans-dermique, intra-artérielle, etc., les voies intraveineuse, intra-musculaire, sous-cutanée, orale et par inhalation étant préférées. Pour les injections, les composés sont généralement conditionnés sous forme de suspensions liquides, qui peuvent être injectées au moyen de seringues ou de perfusions, par exemple. A cet égard, les composés sont généralement dissous dans des solutions salines, physiologiques, isotoniques, tamponnées, etc., compatibles avec un usage pharmaceutique et connues de l'homme du métier. Ainsi, les compositions peuvent contenir un ou plusieurs agents ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc.

Les composés peuvent également être administrés sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Il est entendu que le débit et/ou la dose injectée peuvent être adaptés par l'homme du métier en fonction du patient, de la pathologie concernée, du mode d'administration, etc. Typiquement, les composés sont administrés à des doses pouvant varier entre 0.1 µg et 100 mg/kg de poids corporel, plus généralement de 0,01 à 10 mg/kg, typiquement entre 0,1 et 10 mg/kg. En outre, des injections répétées peuvent être réalisées, le cas échéant. D'autre part, pour des traitements chroniques, des systèmes retard ou prolongés peuvent être avantageux.

Les composés selon l'invention peuvent agir en particulier sur la phosphodiestérase de type PDE2. Ainsi, les composés de l'invention peuvent être des inhibiteurs (sélectifs) de PDE-2, c'est à dire qu'ils présentent une activité inhibirice moindre sur les autres phosphodiestérases et notamment les PDE1, PDE3, PDE4 et PDE5. Certains composés de l'invention présentent un profil d'inhibiteur spécifique de la PDE2, y compris vis-à-vis de l'adénosine déaminase, et possèdent également à ce titre des propriétés thérapeutiques avantageuses.

Les composés de formule (I) selon l'invention inhibiteurs de PDE2 sont particulièrement intéressants dans le traitement de pathologies concernant le système nerveux central, notamment dues à une dérégulation de la fonction d'un des neurotransmetteurs ou un déficit de libération d'un des neurotransmetteurs (e.g. dopamine, noradrenaline, acetylcholine, ...), telles que plus spécifiquement pour le traitement d'une pathologie choisie parmi la dépression, la schizophrénie, l'anxiété, le désordre bipolaire, les désordres de défaut d'attention, les troubles du sommeil, les troubles obsessionnelles compulsifs (TOC ou en anglais : OCD - obsessive compulsive disorder), la fibromyalgie, syndrome de Tourette, pharmacodépendance (drogue, médicament, alcool, etc.), l'épilepsie, la maladie d'Alzheimer, la maladie de Parkinson, la sclérose latérale amyotrophique, la sclérose multiple, l'obésité et la démence des corps de Lewy (« Lewy body dementia »).

Les composés selon l'invention inhibiteurs de PDE2 sont particulièrement intéressants dans le traitement d'autres désordres impliquant le système nerveux périphérique et les organes périphériques en général, en particulier les pathologies de type natriurie réduite, insuffisance rénale aigüe, dysfonctionnement du foie, insuffisance hépatique aigüe, notamment dûs à l'âge, et les pathologies dues ou impliquant des dysfonctionnements de la libération de la prolactine, telles que le syndrome de la jambe sans repos, les désordres rhumatismaux, allergiques ou autoinflammatoires, tels que arthrite rhumatoïde, rhinite et asthme.

Un objet particulier de l'invention réside donc dans l'utilisation des composés tels que décrits ci-avant pour la préparation d'un médicament destiné au traitement de désordres du système nerveux, central ou périphérique, de nature chronique ou aiguë ou l'utilisation périphérique de ces composés en tant que vasoconstricteurs.

L'invention concerne aussi l'utilisation des composés à titre d'agents anxiolytiques, anti-convulsivants, sédatifs ou pour le traitement des troubles de la mémoire ou de troubles cognitifs, en particulier troubles cognitifs modérés (mild cognitive impairment).

L'invention concerne aussi l'utilisation des composés ci-dessus pour le traitement de pathologies neuro-dégénératives.

Au sens de l'invention, le terme traitement désigne aussi bien un traitement préventif que curatif, qui peut être utilisé seul ou en combinaison avec d'autres agents ou traitements. En outre, il peut s'agir d'un traitement de troubles chroniques ou aiguës.

La présente invention a également pour objet l'utilisation des composés décrits pour le traitement de l'obésité.

Les composés préférés de l'invention possèdent avantageusement une puissante activité inhibitrice sur la PDE2. Les composés préférés de l'invention présentent en outre un profil de sélectivité avantageux, notamment une activité faible vis-à-vis de la PDE3.

Les composés de l'invention peuvent être préparés à partir de produits du commerce, en mettant en oeuvre une combinaison de réactions chimiques connues de l'homme du métier.

### Légende des figures

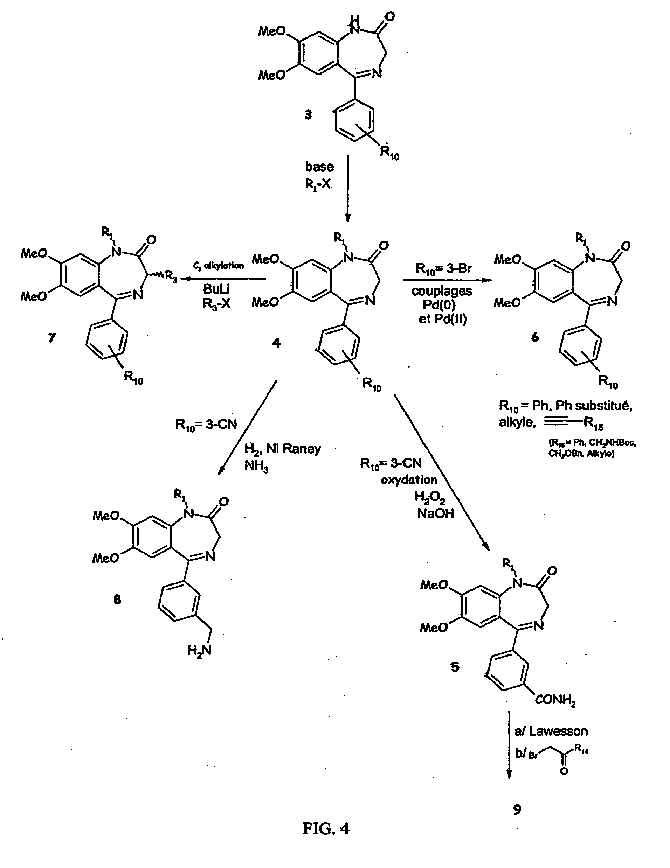

Les figures 1-7 représentent des voies de synthèse des composés de formule (I) selon l'invention.
Figure 1 : Synthèse de 1,4 benzodiazépinones et imidazobenzodiazépines correspondantes, via la **Voie A** (réaction de Friedel Crafts utilisant un nitrile et AlCl₃/BCl₃ comme acide de Lewis), avec R₁₀ = H, CN, Br, CF₃.
Figure 2 : Synthèse de 1,4 benzodiazépinones via la **Voie B** (réaction de Friedel Crafts utilisant un chlorure d'acide et SnCl₄ comme acide de Lewis), avec R₁₀ ≠ CN.
Figure 3 : **Voie C,** passage par l'iminochlorure **16** de la benzodiazépinone.
Figure 4 : Synthèse et autres substitutions des benzodiazépinones **4.**
Figure 5 : Halogénation régiosélective du catéchol des benzodiazépinones. Il est possible de faire également une halogénation directe (en présence de AcOH, NXS) en position R₉ sur une benzophénone de type **2** (schéma 1) qui conduit après cyclisation à une benzodiazépinone de type **(23a)**
Figure 6 : Dérivés substitués sur le cycle benzo des benzodiazépinones.
Figure 7 : Accés aux composés phényl méta carboxamides substitués
Figures 8 et 9 : Résultats du test du labyrinthe en croix surélevé réalisé avec un composé selon l'invention
Figures 10 et 11 : Résulats du test de Porsolt réalisé avec un composé selon l'invention
Figures 12 et 13 : Résulats du test clair/obscure réalisé avec un composé selon l'invention

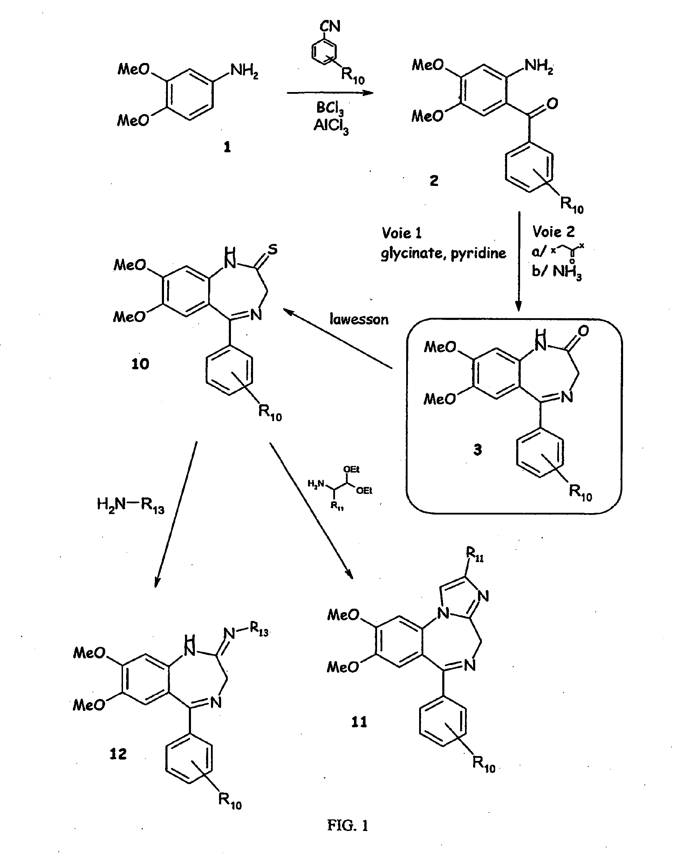

Concernant les procédés de préparation des composés de formule (I), et selon un premier procédé représenté dans la figure 1, les composés de formule générale (I) selon l'invention peuvent être obtenus en mettant en oeuvre les étapes suivantes à partir d'un composé de formule générale **1.**

### - Accès aux ortho-aminobenzophénones 2 :

La réaction de Friedel Crafts à partir d'un composé de formule générale **1** 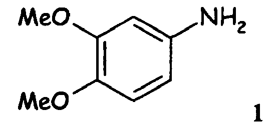 en présence d'un composé de type benzonitrile substitué, de préférence dans un solvant halogéné de type C₂H₄Cl₂, en présence d'un mélange d'acide de Lewis tel que AlCl₃/BCl₃ (réaction de Friedel Crafts), suivie d'une hydrolyse de l'imine formée en présence d'acide chlorhydrique, ce qui permet de former un composé de formule **2** dans laquelle R₁₀ représente les groupes substituants de R5, tels que définis ci-avant, ou est tel que défini dans la figure 1,

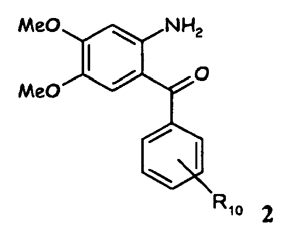

### - Construction du cycle benzodiazépinone. 3 et dérivés 10-11-12

La réalisation de la voie **1** par chauffage au reflux du composé de formule générale **2** en présence de chlorhydrate d'ester d'α-aminoacide et de pyridine à une température comprise entre 100-150°C permet de former un composé de formule générale **3.** La réalisation de la **voie 2,** par addition d'un halogénure d'acétyle de type bromure de bromoacétyle, puis la cyclisation en présence d'ammoniac gaz dans un solvant hydroxylé de type méthanol conduit au composé de formule générale **3** dans laquelle R₁₀ est tel que défini ci-avant.

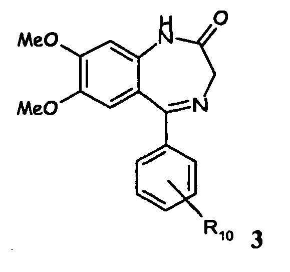

La réaction avec le réactif de Lawesson dans le toluène à reflux d'un composé de type **3,** peut transformer un composé de formule (I) dans laquelle Z est atome d'oxygène en un composé de formule (I) dans laquelle Z est un atome de soufre et ainsi former un composé de type **10.**

La transformation d'un composé de formule (I) dans laquelle Z est un atome de soufre en un composé de formule (I) dans laquelle Z représente NR₁₃ peut notamment être réalisée en faisant réagir le composé soufré **10** obtenu à l'étape précédente, en présence d'une amine de formule NH₂R₁₃ ou par un composé de formule (NH₂)(R₁₁)(CH₂)₂(OEt)₂, R₁₁ et R₁₃ représentant un groupe substituant tel que défini ci-dessus.

### - Autres substitutions et transformations des benzodiazépinones 3

La réaction en présence d'un halogénure d'alkyle, de préférence dans un solvant de type DMF en présence de NaH, permet d'accéder à un composé N-alkylé de formule générale **4** dans laquelle R₁ et R₁₀ sont tels que définis ci-avant

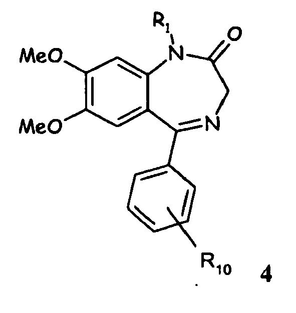

Eventuellement, la transformation d'un composé de formule **4** (R₁₀ = 3-CN) en composé **5** est obtenu par une oxydation de la fonction nitrile de l'aromatique, par réaction avec H₂O₂ et NaOH à 50°C dans l'éthanol.

Eventuellement, la transformation d'un composé de formule **4** (R₁₀ = 3-Br) en composé **6** est obtenu par un couplage palladium en présence d'un acide aryl boronique, ou d'un alkyne monosubstitué ou mono fonctionnalisé et d'une base K₃PO₄, K₂CO₃, triéthylamine selon les partenaires réactionnels. Le complexe au Pd(0) ou Pd(II) est de type Pd(PPh₃)₄ ou PdCl₂, dans un solvant de type DMF, EtOH.

Eventuellement, la transformation d'un composé de formule **4** (R₁₀ = 3-CN) en composé **8** est obtenu par une réduction de la fonction nitrile par hydrogénation dans le méthanol en présence de Nickel de Raney.

Eventuellement, la transformation d'un composé de formule **4** en composé **7** est obtenu par une alkylation sur le carbone 3 par réaction d'une base, de préférence le BuLi, dans un solvant de type THF, et addition d'un électrophile de type bromure ou chlorure d'alkyle, cycloalkyle, benzyle substitué ou non.

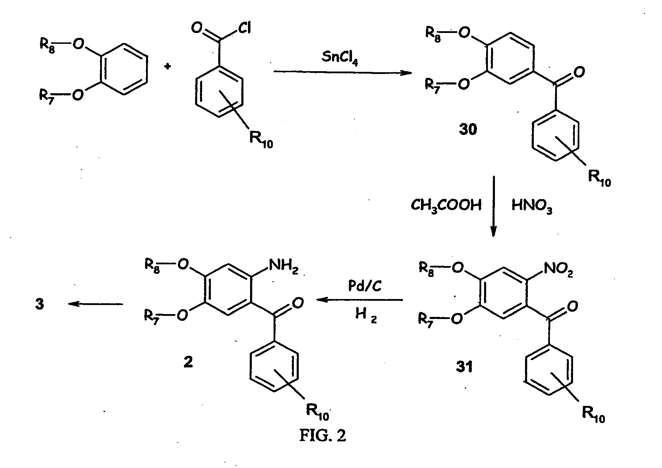

Selon un deuxième procédé représenté dans la figure 2, les composés de formule générale (I) peuvent être préparés par un procédé comprenant les étapes suivantes :
La réaction d'un composé de formule générale (II) 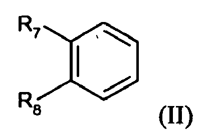 dans laquelle R₇ et R₈ sont tels que définis précédemment, avec un agent d'acylation, tel qu'un composé de type chlorure de benzoyle au moins substitué en position 3, en présence d'un acide de Lewis, en particulier en présence de SnCl₄, dans un solvant halogéné de type CH₂Cl₂ conduit à une benzophénone de formule **30** 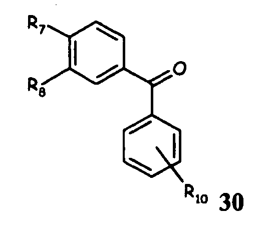 dans laquelle R₇ et R₈ sont tels que définis précédemment et R₁₀ est un groupe substituant sur le phényle;
La réaction du composé de formule **30** en présence de CH₃COOH et HNO₃ à température ambiante permet d'obtenir un composé nitré de formule **31** 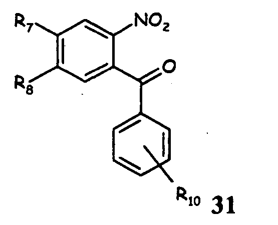
La réaction d'hydrogénation en présence d'un catalyseur de type Pd/C dans le méthanol fournit un composé de type **2**

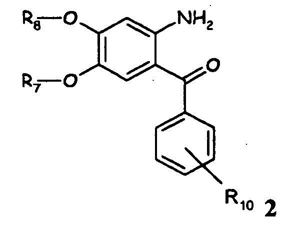

La réalisation de **la voie 1** ou de **la voie 2** à partir d'un composé de type **2** conduit à un composé de type **3.**

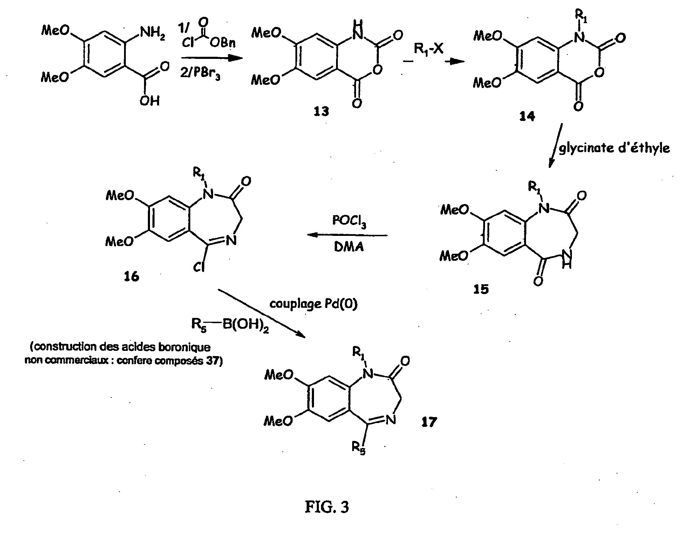

Selon un autre mode de mise en oeuvre (figure 3), les composés de formule générale (I) selon l'invention dans laquelle Z est un atome d'oxygène peuvent être préparés à partir d'un composé de formule générale **13.**

La réaction d'un composé de formule générale **13 :** 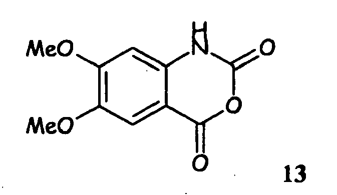 en présence d'un halogénure d'alkyle, de préférence dans un solvant de type DMF en présence de NaH, permet d'accéder à un composé N-alkylé de formule générale **14** dans laquelle R₁ tels que définis ci-avant

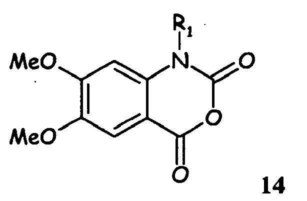

Le chauffage au reflux du composé de formule générale **14** en présence de chlorhydrate d'ester d'α-aminoacide et de pyridine, suivi d'une cyclisation en milieu acide, par exemple en présence d'acide acétique, à une température comprise de préférence entre 100 et 150°C, fournit un composé de formule générale **15** dans laquelle R₁ est tel que défini ci-avant

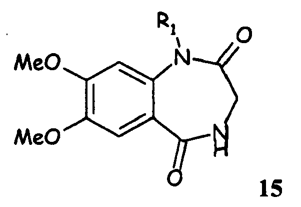

La réaction du composé de formule générale **15** en présence de diméthylaniline (ou de diméthylaminopyridine) et de d'oxyhalogénure de phosphore (de préférence POCl₃), de préférence à une température comprise entre 80 et 150°C en milieu CHCl₃ anhydre et en tube scellé, permet de former un composé iminochlorure de formule générale **16.**

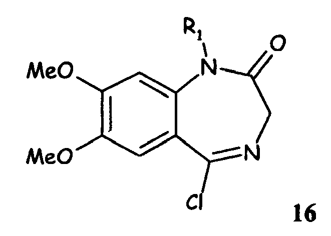

Les couplages avec un acide boronique de formule générale R₅-B(OH)₂ dans laquelle R₅ est tel que défini ci-avant, en présence d'une base de type K₃PO₄, K₂CO₃ et d'un complexe au Pd(0) de type Pd(PPh₃)₄, dans un solvant de type DMF, EtOH, conduit à un composé de formule générale **17.**

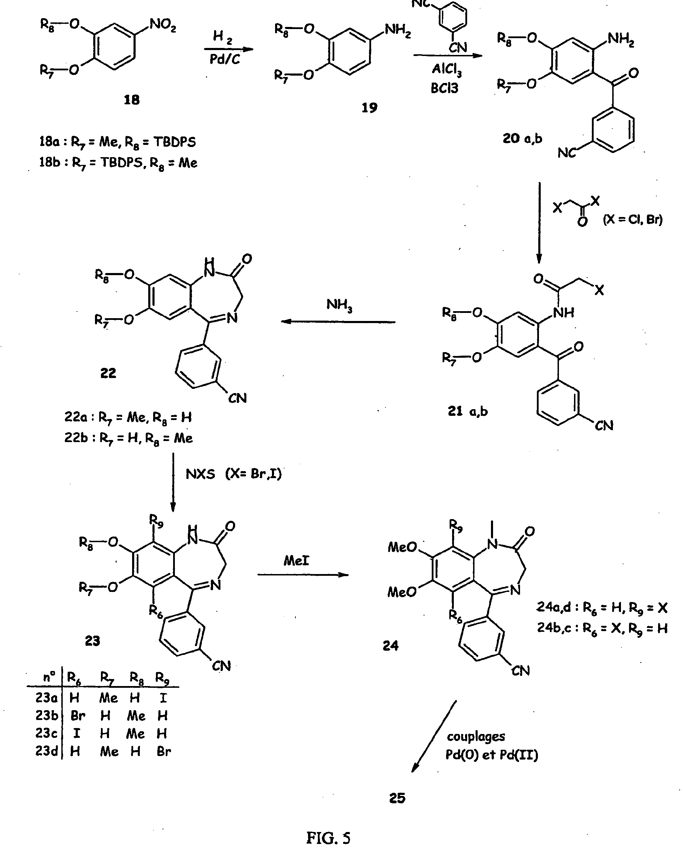

Après hydrogénation catalysée des nitrocatéchols **18** convenablement substitués ou protégés, les composés **22,** qui répondent à la formule générale (I), sont préparés selon des voies précédemment décrites, avec R₈ et R₇ qui sont définis tel que dans la figure 5.

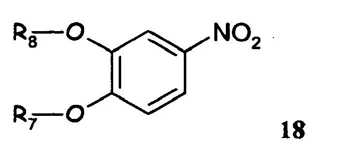

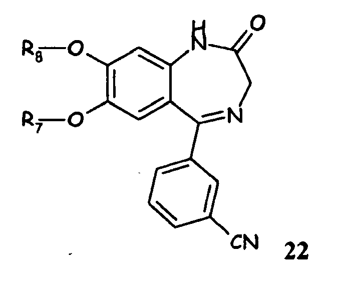

La réaction d'un composé **22** en présence de N-bromo ou N-chloro ou N-iodo succinimide, dans un solvant de type CH₂Cl₂, et un acide de type acide acétique conduit à un composé de formule générale **23,** avec R₈ et R₇ qui sont définis tel que dans la figure 5 et dans cet exemple R₆ ou R₉ représentent un atome d'halogène.

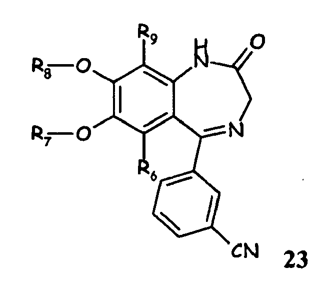

La réaction du composé **23** en présence de iodométhane, de préférence dans un solvant de type DMF en présence de NaH, permet d'accéder à un composé de formule générale **24** dans laquelle R₆ ou R₉ R₉ représentent un atome d'halogène.

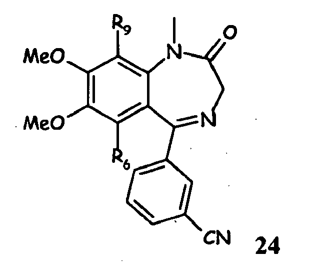

Les couplages palladium en présence d'un acide aryl boronique, ou d'un alkyne monosubstitué ou mono fonctionnalisé et d'une base K₃PO₄, K₂CO₃, triéthylamine selon les partenaires réactionnels. Le complexe au Pd(0) ou Pd(II) est de type Pd(PPh₃)₄ ou PdCl₂, dans un solvant de type DMF, EtOH conduit à des composés de formule générale **25.**

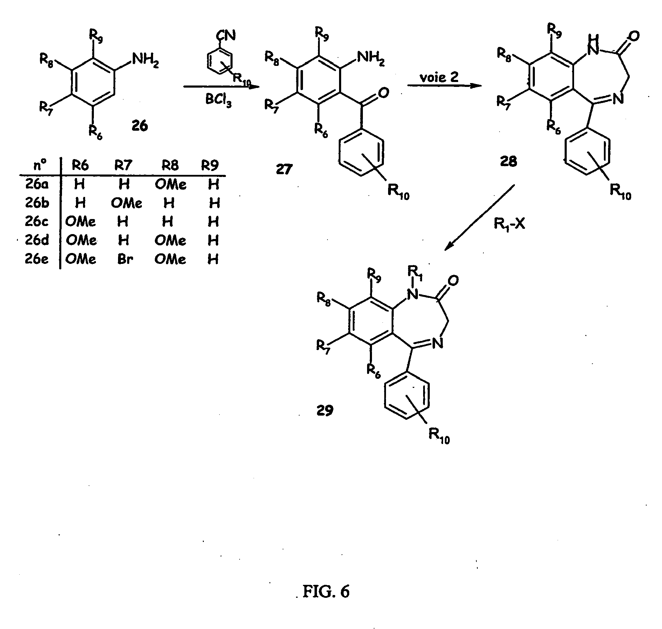

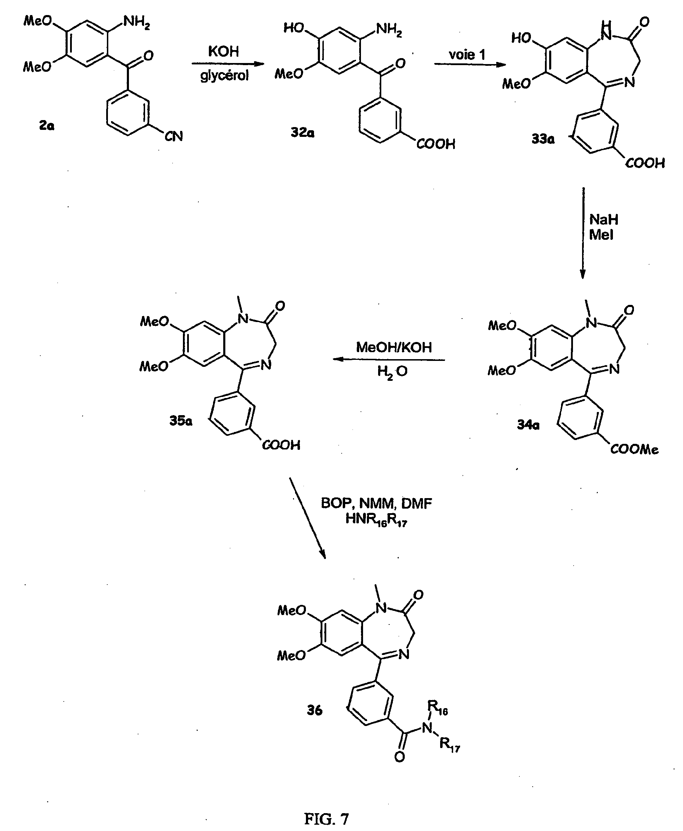

Les composés **29** répondant à la formule générale **I** mais différemment substitués ou trisubstitués sur le cycle benzodiazépine, sont préparés selon une méthode décrite dans la figure 1 et telles que illustrées dans la figure 6
Selon un autre mode de mise en oeuvre (figure 7), les composés de formule générale (I) selon l'invention dans laquelle Z est un atome d'oxygène peuvent être préparés à partir d'un composé **2a**

La réaction du composé **2a** : 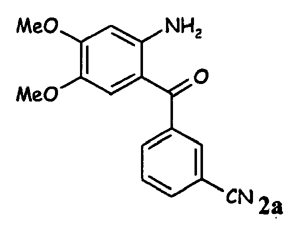 par chauffage en présence d'une base de type NaOH, KOH, de préférence dans un solvant de type alcoolique tel que méthanol, éthanol, glycérol forme le composé **32a**

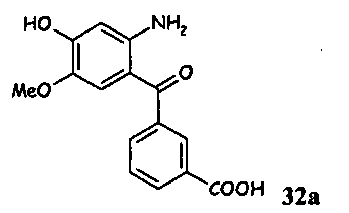

Le chauffage au reflux du composé de formule générale **32a** en présence de chlorhydrate d'ester d'α-aminoacide et de pyridine, à une température comprise de préférence entre 100 et 150°C, forme un composé **33a**

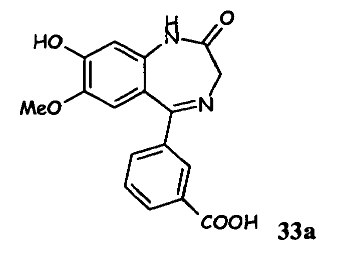

La réaction d'un composé **33a** en présence du iodure de méthyle, de préférence dans un solvant de type DMF en présence de NaH, conduit à un composé de formule générale **34a**

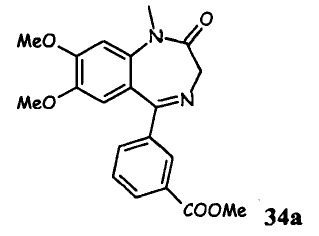

La réaction du composé **34a** par chauffage en présence d'une base de type NaOH, KOH, de préférence dans un solvant de type alcoolique tel que méthanol, éthanol, glycérol forme le composé **35a**

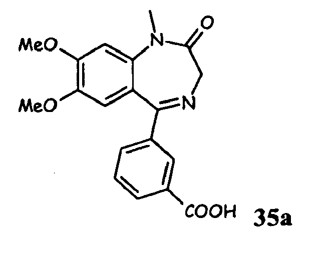

La réaction du composé **35a** avec une amine primaire ou secondaire , en présence d'une base de type N-méthyl morpholine, de BOP dans un solvant de type DMF conduit aux amides de formule générale **36,** avec R₁₆ et R₁₇ qui sont définis tel que ci-avant.

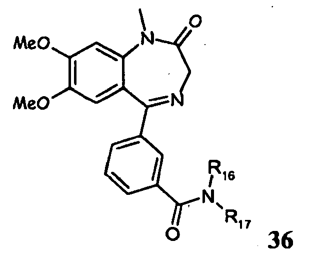

Un autre objet de l'invention réside dans une méthode de traitement d'une pathologie liée à un désordre du système nerveux central ou périphérique, en particulier central, comprenant l'administration à un animal, de préférence l'homme, d'un composé inhibiteur de la PDE2, de préférence un composé inhibiteur sélectif de PDE2, tel que décrit ci-dessus. En particulier, les pathologies sont celles identifiées ci-dessus. Les inhibiteurs de PDE2 sont de préférence des dérivés de 1,4-benzodiazépine et en particulier les composés de formule (I).

L'invention est illustrée par les exemples qui suivent.

### EXEMPLES

### EXEMPLE 1 : SYNTHESE DE COMPOSES DE FORMUULE (I)

### - Synthèses des benzophénones de type 2.

### 3-(2-Amino-4,5-diméthoxybenzoyl)benzonitrile, 2a

Ajouter, à 0°C et sous atmosphère inerte, à une solution de 14.4 mL de tribromure de bore (1M/ CH₂Cl₂, 14.4 mmo les), 2.0 g de 3,4-diméthoxyaniline (13.06 mmoles) dissout dans 17 mL de 1,2-dichloroéthane, 2.5 g d'isophtalonitrile (19.51 mmoles), et 1.92 g d'AlCl₃ (14.40 mmoles). Agiter 30 minutes à température ambiante. Evaporer le dichlorométhane. Chauffer à reflux 16 heures. Laisser refroidir. Additionner 14 mL d'HCl (1M) à 0°C, agiter à 80°C pendant 2 heures. Additionner 50 mL d'eau, et extraire avec 3 fois 100 mL de CH₂Cl₂. Sécher les fractions organiques sur Na₂SO₄, filtrer, évaporer à sec et purifier par chromatographie sur silice (AcOEt/hexane : 1/3 ). Rdt : 61%. RMN ¹H (CDCl₃, 300 MHz) : δ 3.66 (s, 3H, OC**H₃**), 3.92 (s, 3H, OC**H₃**), 6.17-6.48 (m, 3H, N**H₂** + **1H Ar**), 6.74 (s, **1H Ar**), 7.56-7.91 (m, **4H Ar**).

### (2-Amino-4,5-diméthoxyphényl)(3-bromophényl)méthanone, 2b

En remplaçant dans l'exemple **2a** l'isophtalonitrile par le 3-bromobenzonitrile, on obtient de la même manière le produit titre. Rdt : 50%. RMN ¹H (CDCl₃, 200 MHz) : δ 3.67 (s, 3H, OC**H₃**), 3.92 (s, 3H, OC**H₃**), 6.20-6.24 (m, 3H, N**H₂** + **1H Ar**), 6.86 (s, **1H Ar**), 7.29-7.37 (m, **1H Ar**), 7.51-7.55 (m, **1H Ar**), 7.61-7.65 (m, **1H Ar**), 7.75-7.78 (m, **1H Ar**).

### (2-Amino-4,5-diméthoxyphényl)(phényl)méthanone, 2c

En remplaçant dans l'exemple **2a** l'isophtalonitrile par le benzonitrile, on obtient de la même manière le produit titre. Rdt : 57%. RMN ¹H (CDCl₃, 200 MHz) : δ 3.66 (s, 3H, OC**H₃**), 3.91 (s, 3H, OC**H₃**), 6.21 (m, 3H, N**H₂** + **1H Ar**), 6.94 (s, **1H Ar**), 7.45-7.64 (m, **5H Ar**).

### (2-Amino-4,5-diméthoxyphényl)([3-(trifluorométhyl)phényl]méthanone, 2d

En remplaçant dans l'exemple **2a** l'isophtalonitrile par le 3-(trifluorométhyl)benzonitrile, on obtient de la même manière le produit titre. Rdt : 60%. RMN ¹H (CDCl₃, 200 MHz) : δ 3.66 (s, 3H, OC**H₃**), 3.93 (s, 3H, OC**H₃**), 6.21-6.31 (m, 3H, N**H₂** + **1H Ar),** 6.74 (s, **1H Ar**), 7.57-7.83 (m, **4H Ar**).

### (2-amino-4,5-diéthoxyphényl)(phényl)méthanone, 2e

En remplaçant dans l'exemple **2a** l'isophtalonitrile par le benzonitrile, et le 3,4-diméthoxyaniline par le 3,4-diéthoxyaniline on obtient de la même manière le produit titre. Rdt : 35%. ¹H-RMN (CDCl₃, 300MHz) : δ 1,32 (t, 3H, -C**H₃**), 1,48 (t, 3H, -C**H₃**), 3,85 (q, 2H, OC**H₂**), 4,10 (q, 2H, OC**H₂**), 6,19 (s, **1H Ar**), 6,23 (s, 2H échangeable, - N**H₂**), 6,99 (s, 1**H Ar**), 7,42-7,62 (m, 5**H Ar**).

### - Accès aux benzodiazépinones de type 3.

### 3-(7,8-Diméthoxy-2-oxo-2,3-dihydro-1H-1,4-benzodiazépin-5-yl)benzonitrile 3a

Additionner à une solution de 3-(2-amino-4,5-diméthoxybenzoyl)benzonitrile **2a** (2.0 g, 7.09 moles) dans le dichlorométhane (15 mL) à 0-5°C, le bromure de bromoacétate (0.76 mL, 8.72 mmoles) puis goutte à goutte le Na₂CO₃ 10% (8.5 mL). Agiter 1 heure à cette température. Séparer les deux phases et laver la phase organique avec 10 mL d'eau, sécher Na₂SO₄, filtrer, évaporer à sec (2.8 g). Agiter, à 0°C avec un tube à CaCl₂, le solide précédemment obtenu (2.8 g, 6.94 mmoles) en solution dans NH3 (7N)/MeOH (90 mL) pendant 3 heures puis à température ambiante 1 heure. Chauffer à reflux pendant 3 heures, filtrer le précipité (1.78 g). Rdt : 80%. RMN ¹H (CDCl₃, 300 MHz) : δ 3.75 (s, 3H, OC**H₃**), 3.98 (s, 3H, OC**H₃**), 4.50 (large s, 2H; C**H₂**), 6.60 (s, **1H Ar**), 6.65 (s, **1H Ar**), 7.50-7.95 (m, **4H Ar**), 9.04 (large s, 1H, N**H**).

### 5-(3-bromophényl)-7,8-diméthoxy-1,3-dihydro-2H-1,4-benzodiazépin-2-one 3b

En remplaçant dans l'exemple **3a** le 3-(2-amino-4,5-diméthoxybenzoyl)benzonitrile **2a** par le (2-amino-4,5-diméthoxyphényl)(3-bromophényl)méthanone **2b,** on obtient de la même manière le produit titre. Rdt : 70%. RMN ¹H (CDCl₃, 200 MHz) : δ 3.75 (s, 3H, OC**H**₃), 3.97 (s, 3H, OC**H₃**), 4.32 (large s, 2H, C**H₂**), 6.61 (s, 1H, **1H Ar**), 6.68 (s, **1H Ar**), 7.22-7.30 (m, **1H Ar),** 7.46-7.50 (m, **1H Ar**), 7.57-7.61 (m, **1H Ar**), 7.79-7.80 (m, **1H Ar),** 8.83 (s, 1H, N**H**).

### 7,8-Diméthoxy-5-phényl-1,3-dihydro-2H-1,4-benzodiazépin-2-one 3c

En remplaçant dans l'exemple **3a** le 3-(2-amino-4,5-diméthoxybenzoyl)benzonitrile **2a** par le (2-amino-4,5-diméthoxyphényl)(phényl)méthanone **2c,** on obtient de la même manière le produit titre. Rdt : 85%. RMN ¹H (CDCl₃, 200 MHz) : δ 3.72 (s, 3H, OC**H₃**), 3.95 (s, 3H, OC**H₃**), 4.31 (large s, 2H, C**H₂**), 6.64 (s, 1H, **1H Ar**), 6.70 (s, **1H Ar**), 7.37-7.59 (m, **5H Ar**), 9.40 (s, 1H, N**H**).

### 7,8-Diméthoxy-[5-(3-trifluorométhyl)phényl]-1,3-dihydro-2H-1,4-benzodiazépin-2-one, 3d

En remplaçant dans l'exemple **3a** le 3-(2-amino-4,5-diméthoxybenzoyl)benzonitrile **2a** par le (2-amino-4,5-diméthoxyphényl)([3-(trifluorométhyl)phényl]méthanone **2d,** on obtient de la même manière le produit titre. Rdt : 80%. RMN ¹H (CDCl₃, 300 MHz) : δ 3.73 (s, 3H, OC**H₃**), 3.98 (s, 3H, OC**H₃**), 4.35 (large s, 2H, C**H₂**), 6.62 (s, 1H, **1H Ar**), 6.67 (s, **1H Ar**), 7.50-7.55 (m, **1H Ar**), 7.71-7.73 (m, **1H Ar**), 7.78-7.81 (m, **1H Ar**), 7.91 (m, **1H Ar**), 8.67 (s, 1H, N**H**).

### 7,8-diéthoxy-5-phényl-1,3-dihydro-2H-1,4-benzodiazépin-2-one, 3e

En remplaçant dans l'exemple **3a,** le 3-(2-amino-4,5-diméthoxybenzoyl)benzonitrile **2a** par (2-amino-4,5-diéthoxyphényl)(phényl)méthanone **2e,** on obtient de la même manière le produit titre. Rdt : 60%. F : 233-236°C. RMN ¹H (CDCl₃, 200MHz): δ 1,39 (t, 3H, C**H₃**), 1,54 (t, 3H, C**H₃**), 3,94 (q, 2H, OC**H₂**), 4,18 (q, 2H, OC**H₂**), 4,35 (s, 2H, C**H₂**), 6,66 (s, **1H Ar**), 6,74 (s, 1**H** Ar), 7,36-7,63 (m, **5H Ar**), 9,51 (s, 1H échangeable, -N**H**).

### - Alkylation de l'azote de type 4.

### 3-(7,8-diméthoxy-1-méthyl-2-oxo-2,3-dihydro-1H-1,4-benzodiazépin-5-yl)-benzonitrile, 4a

A un mélange, à froid, de toluène (10 mL) et d'Aliquat 336 (34 mL), est ajouté l'iodométhane (0.42 mL, 6.72 mmoles). Sous agitation, on additionne le 3-(7,8-diméthoxy -2-oxo-2,3-dihydro-1*H*-1,4-benzodiazèpin-5-yl)-benzonitile **3a** (1.13 g, 3.36 mmoles) et une solution aqueuse de soude à 50% (4 mL). On laisse remonter le mélange réactionnel à température ambiante et on agite 4 heures. La réaction est diluée avec un mélange dichlorométhane/eau : 50/50 (200 mL). Les phases sont séparées & la phase aqueuse est extraite une fois au dichlorométhane. Les phases organiques rassemblées sont séchées sur sulfate de sodium puis filtrées, on évapore à sec & on chromatographie : éluant CH₂Cl₂/Et₂O : 1/1. On obtient un solide blanc (1.08 g). Rdt : 96%. RMN ¹H (DMSO-d₆, 200 MHz) : δ 3.41 (s, 3H, NC**H₃**), 3.76 (s, 3H, OC**H₃**), 3.99 (s, 3H, OC**H₃**), 4.30 (system AB, Δδ = 1.0, J_{AB} = 10 Hz, 2H, C**H₂**), 6.60 (s, **1H Ar**), 6.80 (s, **1H Ar**), 7.49-7.96 (m, **4H Ar**).

### 5-(3-bromophényl)-7,8-diméthoxy-1-méthyl-1,3-dihydro-2H-1,4-benzodiazépin-2-one, 4b

En remplaçant dans l'exemple **4a** le 3-(7,8-diméthoxy-2-oxo-2,3-dihydro-1*H*-1,4-benzodiazépin-5-yl)-benzonitrile **3a** par la 5-(3-bromophényl)-7,8-diméthoxy-1,3-dihydro-2*H-*1,4-benzodiazépin-2-one **3b,** on obtient de la même manière le produit titre. Rdt : 70%. RMN ¹H (CDCl₃, 200 MHz) : δ 3.40 (s, 3H, NC**H₃**), 3.77 (s, 3H, OC**H₃**), 3.99 (s, 3H, OC**H₃**), 4.30 (system AB, Δδ = 1.1, J_{AB} = 10 Hz, 2H, C**H₂**), 6.66 (s, **1H Ar),** 6.78 (s, **1H Ar),** 7.26 (m, **1H Ar**), 7.55 (m, **2H Ar**), 7.84 (m, **1H Ar**).

### 3-[1-(4-chlorobenzyl)-7,8-diméthoxy-2-oxo-2,3-dihydro-1H-1,4-benzodiazépin-5-yl]-benzonitrile, 4c

En remplaçant dans l'exemple **4a** l'iodométhane par le bromure de 4-chlorobenzyle, on obtient de la même manière le produit titre. Rdt : 75%. RMN ¹H (CDCl₃, 300 MHz) : δ 3.71 (s, 3H, OC**H₃**), 3.88 (s, 3H, OC**H₃**), 4.40 (system AB, Δδ = 1.0, J_{AB} = 10 Hz, 2H, C**H₂**), 5.10 (system AB, Δδ = 0.8, J_{AB} = 15 Hz, 2H, NC**H₂**), 6.46 (s, **1H Ar**), 6.82 (s, **1H Ar**), 6.97-7.00 (m, **2H Ar**), 7.10-7.13 (m, **2H Ar**), 7.46-7.55 (m, **2H Ar**), 7.73-7.79 (m, **2H Ar**).

### 3-[1-(3,4-chlorobenzyl)-7,8-diméthoxy-2-oxo-2,3-dihydro-1H-1,4-benzodiazépin-5-yl]-benzonitrile, 4d

En remplaçant dans l'exemple **4a** l'iodométhane par le chlorure de 3,4-dichlorobenzyle, on obtient de la même manière le produit titre. Rdt : 65%. RMN ¹H (CDCl₃, 300 MHz) : δ 3.73 (s, 3H, OC**H₃**), 3.90 (s, 3H, OC**H₃**), 4.40 (system AB, Δδ = 1.0, J_{AB} = 10 Hz, 2H, C**H**₂), 5.10 (system AB, Δδ = 0.8, J_{AB} = 15 Hz, 2H, NC**H₂**), 6.52 (s, **1H Ar**), 6.80 (s, **1H Ar**), 6.93-6.95 (d, **1H Ar**), 7.09 (s, **1H Ar**), 7.23-7.26 (d, **1H Ar**), 7.51-7.53 (t, **1H Ar**), 7.64-7.67 (d, **1H Ar**), 7.75-7.77 (d, **1H Ar**), 7.86 (s, **1H Ar).**

### 3-[7,8-diméthoxy-1-(4-méthoxybenzyl)-2-oxo-2,3-dihydro-1H-1,4-benzodiazépin-5-yl]-benzonitrile, 4e

En remplaçant dans l'exemple **4a** l'iodométhane par le chlorure de 4-méthoxybenzyle, on obtient de la même manière le produit titre. Rdt : 60%. RMN ¹H (CDCl₃, 300 MHz) : δ 3.70 (s, 3H, OC**H₃**), 3.74 (s, 3H, OC**H₃**), 4.40 (system AB, Δδ = 1.0, J_{AB} = 10 Hz, 2H, C**H₂**), 3.90 (s, 3H, OC**H₃**), 5.10 (system AB, Δδ = 0.9, J_{AB} = 15 Hz, 2H, NC**H₂**), 6.42 (s, **1H Ar**), 6.65-6.68 (d, **2H Ar**), 6.87 (s, **1H Ar**), 6.95-6.97 (d, **2H Ar**), 7.45-7.50 (t, **1H Ar**), 7.62 (s, **1H Ar**), 7.61-7.65 (d, **1H Ar**), 7.68-7.70 (d, **1H Ar**).

### 3-[1-(3-chlorobenzyl)-7,8-diméthoxy-2-oxo-2,3-dihydro-1H-1,4-benzodiazépin-5-yl]-benzonitrile, 4f

En remplaçant dans l'exemple **4a** l'iodométhane par le bromure de 3-chlorobenzyle, on obtient de la même manière le produit titre. Rdt : 70%. RMN ¹H (CDCl₃, 300 MHz) : δ 3.72 (s, 3H, OC**H₃**), 3.89 (s, 3H, OC**H₃**), 4.40 (system AB, Δδ = 1.0, J_{AB} = 10 Hz, 2H, C**H₂**), 5.10 (system AB, Δδ = 0.9, J_{AB} = 15 Hz, 2H, NC**H₂**), 6.49 (s, **1H Ar**), 6.81 (s, **1H Ar**), 6.99-7.09 (m, **2H Ar**), 7.11-7.18 (t, **1H Ar**), 7.20 (m, **1H Ar**), 7.48-7.54 (t, **1H Ar**), 7.69-7.74 (m, **3H Ar**).

### 3-{7,8-diméthoxy-2-oxo-1-[3-(trifluorométhyl)benzyl]-2,3-dihydro-1H-1,4-benzodiazépin-5-yl]-benzonitrile, 4g

En remplaçant dans l'exemple **4a** l'iodométhane par le chlorure de (3-trifluorométhyl) benzyle, on obtient de la même manière le produit titre. Rdt : 70%. RMN ¹H (CDCl₃, 300 MHz) : δ 3.72 (s, 3H, OC**H₃**), 3.88 (s, 3H, OC**H₃**), 4.40 (system AB, Δδ = 1.0, J_{AB} = 10 Hz, 2H, C**H₂**), 5.20 (system AB, Δδ = 0.8, J_{AB} = 15 Hz, 2H, NC**H₂**), 6.50 (s, **1H Ar),** 6.81 (s, **1H Ar**), 7.29-7.31 (m, **3H Ar**), 7.46-7.52 (m, **2H Ar**), 7.69-7.75 (m, **3H Ar**).

### 3-[1-(2-chlorobenzyl)-7,8-diméthoxy-2-oxo-2,3,-dihydro-1H-1,4-benzodiazépin-5-yl]-benzonitrile, 4h

En remplaçant dans l'exemple **4a** l'iodométhane par le bromure de 2-chlorobenzyle, on obtient de la même manière le produit titre. Rdt : 60%. RMN ¹H (CDCl₃, 300 MHz) : δ 3.70 (s, 3H, OC**H**₃), 3.92 (s, 3H, OC**H₃**), 4.40 (system AB, Δδ = 1.0, J_{AB} = 10 Hz, 2H, C**H₂**), 5.30 (system AB, Δδ = 0.6, J_{AB} = 15 Hz, 2H, NC**H₂**), 6.45 (s, **1H** Ar), 6.88 (s, **1H Ar**), 6.95-6.96 (m, **2H Ar**), 7.13-7.18 (m, **1H Ar**), 7.28-7.30 (m, **1H Ar**), 7.48-7.51 (m, **1H** Ar), 7.54 (s, **1H Ar**), 7.66-7.74 (m, **2H Ar**).

### 3-{7,8-diméthoxy-2-oxo-1-[4-(trifluorométhyl)benzyl]-2,3-dihydro-1H-1,4-benzodiazépin-5-yl]-benzonitrile, 4i

En remplaçant dans l'exemple 4a l'iodométhane par le bromure de (4-trifluorométhyl) benzyle, on obtient de la même manière le produit titre. Rdt : 70%. RMN ¹H (CDCl₃, 300 MHz) : δ 3.47 (s, 3H, OC**H₃**), 3.71 (s, 3H, OC**H₃**), 4.40 (system AB, Δδ = 1.0, J_{AB} = 10 Hz, 2H, C**H₂**), 5.20 (system AB, Δδ = 0.8, J_{AB} = 15 Hz, 2H, NC**H₂**), 6.48 (s, **1H Ar**), 6.82 (s, **1H Ar**), 7.17-7.20 (m, **2H Ar**), 7.40-7.47 (m, **4H Ar**), 7.73 (m, **1H Ar**), 7.87 (m, **1H Ar**).

### 3-[7,8-diméthoxy-2-oxo-1-(2-phényléthyl)-2,3-dihydro-1H-1,4-benzodiazépin-5-yl]-benzonitrile, 4j

En remplaçant dans l'exemple **4a** l'iodométhane par le 2-bromoéthyl benzène de benzyle, on obtient de la même manière le produit titre. Rdt : 65%. RMN ¹H (CDCl₃, 300 MHz) : δ 2.88-2.94 (m, 2H, Ph-C**H₂**), 3.74 (s, 3H, OC**H₃**), 3.92 (s, 3H, OC**H₃**), 4.15 (system AB, Δδ = 0.8, J_{AB} = 15 Hz, 2H, NC**H₂**), 4.40 (system AB, Δδ = 0.8, J_{AB} = 14 Hz, 2H, C**H₂**), 6.50 (s, **1H Ar**), 6.75 (s, **1H Ar**), 7.08-7.12 (m, **5H Ar**), 7.47-7.50 (t, **1H Ar**), 7.53 (s, **1H Ar**), 7.67 (d, **1H Ar),** 7.72 (d, **1H Ar).**

### 3-(1-éthyl-7,8-dimethoxy-2-oxo-2,3-dihydro-1H-1,4-benzodiazépin-5-yl)benzonitrile, 4k

A un mélange, à froid, de DMF (4 mL) et de NaH à 60% (29 mg, 0.71 mmols), est ajouté le 3-(7,8-diméthoxy-2-oxo-2,3-dihydro-1H-1,4-benzodiazépin-5-yl)-benzonitrile **3a** (200 mg, 065 mmols). La solution est agitée une demi heure à r.t., puis, à 0°C, est additionné le iodoéthane (67 µL, 0.84 mmols). La solution reste sous agitation 1 heure à r.t. Un mélange de 30mL H₂O/glace est versé, puis la phase aqueuse est extraite par 3* 30mL d'Et₂O. Les phases organiques rassemblées sont séchées sur sulfate de sodium puis filtrées, on évapore à sec & on chromatographie sur silice : éluant : AcOEt. On obtient un solide blanc (110 mg). Rdt : 48%. RMN ¹H (CDCl₃, 200 MHz) : δ 1.13-1.20 (t, 3H, -C**H₃**), 3.66-3.78 (m, 1H, NC**H₂**), 3.80 (s, 3H, OC**H₃**), 4.02 (s, 3H, OC**H₃**), 4.29-4.40 (m, 1H, NC**H₂**), 4.34 (system AB, Δδ = 1.0, J_{AB} = 10 Hz, 2H, C**H2**), 6.61 (s, **1H Ar**), 6.90 (s, **1H Ar**), 7.57-7.61 (t, **1H Ar**), 7.77-7.80 (d, **1H Ar**), 7.93-7.99 (m, **2H Ar**).

### 3-(7,8-diméthoxy-1-propyl-2-oxo-2,3-dihydro-1H-1,4-benzodiazépin-5-yl)benzonitrile, 41

En remplaçant dans l'exemple **4k** l'iodoéthane par le iodopropane, on obtient de la même manière le produit titre. Rdt : 49%. RMN ¹H (CDCl₃, 200 MHz) : δ 0.75-0.82 (t, 3H, -C**H₃**), 1.42-1.58 (m, 2H, C**H₂**-CH₃), 3.48-3.58 (m, 1H, NC**H₂**), 3.80 (s, 3H, OC**H₃**), 3.83 (system AB, Δδ = 1.0, J_{AB} = 10 Hz, 2H, C**H2**), 4.02 (s, 3H, OC**H₃**), 4.32-4.46 (m, 1H, NC**H₂**), 4.83 (system AB, Δδ = 1.0, J_{AB} = 10 Hz, 2H, C**H2**), 6.61 (s, **1H Ar**), 6.89 (s, **1H Ar**), 7.57-7.61 (t, **1H Ar**), 7.77-7.80 (d, **1H** Ar), 7.94-8.00 (m, **2H Ar**).

### 3-(1-benzyl-7,8-diméthoxy-2-oxo-2,3-dihydro-1H-1,4-benzodiazépin-5-yl)benzonitrile, 4m

En remplaçant dans l'exemple **4k** l'iodoéthane par le bromure de benzyle, on obtient de la même manière le produit titre. Rdt : 49%. RMN ¹H (CDCl₃, 200 MHz) : δ 3.69 (s, 3H, OC**H₃**), 3.88 (s, 3H, OC**H₃**), 4.40 (system AB, Δδ = 1.0, J_{AB} = 10 Hz, 2H, C**H2**), 5.13 (system AB, Δδ = 0.85, J_{AB} = 15 Hz, 2H, C**H2**), 6.43 (s, **1H Ar**), 6.86 (s, **1H Ar**), 7.01-7.26 (m, **4H Ar**), 7.44-7.55 (m, **2H Ar**), 7.66-7.71 (m, **2H Ar**).

### éthyl [5-(3-cyanophényl)-7,8-diméthoxy-2-oxo-2,3-dihydro-1H-1,4-benzodiazépin-1-yl]acétate, 4n

En remplaçant dans l'exemple **4k** l'iodoéthane par le bromoacétate d'éthyle, on obtient de la même manière le produit titre. Rdt : 55%. RMN ¹H (CDCl₃, 200 MHz) : δ 1.22 (t, 3H, C**H₃**), 3.75 (s, 3H, OC**H₃**), 3.94 (s, 3H, OC**H₃**), 4.18 (m , 2H, OC**H₂**), 4.36 (system AB, Δδ = 0.93, J_{AB} = 10 Hz, 2H, CH2), 4.51 (s, 2H, NC**H₂**), 6.58 (s, **1H Ar**), 6.83 (s, **1H Ar**), 7.49-7.57 (t, **1H Ar**), 7.72-7.76 (d, **1H Ar**), 7.92-7.97 (m, **2H Ar**).

### 7,8-Diméthoxy-1-éthyl-5-phényl-1,3-dihydro-2H-1,4-benzodiazépin-2-one 4o

En remplaçant dans l'exemple **4a** le 3-(7,8-diméthoxy-2-oxo-2,3-dihydro-1*H*-1,4-benzodiazépin-5-yl)-benzonitrile **3a** par la 7,8-diméthoxy-5-phényl-1,3-dihydro-2*H-*1,4-benzodiazépin-2-one **3c** et le iodométhane par le iodoéthane, on obtient de la même manière le produit titre. Rdt : 95%. RMN ¹H (CDCl₃, 200 MHz) : δ 1.11 (t, 3H C**H₃**), 3.58-3.70 (m, 1H NC**H₂**), 3.75 (s, 3H, OC**H₃**), 3.97 (s, 3H, OC**H₃**), 4.27 (system AB, Δδ = 0.98, J_{AB} = 10 Hz, 2H, C**H₂**), 4.29-4.39 (m, 1H NC**H₂**), 6.68 (s, **1H Ar**), 6.84 (s, **1H Ar**), 7.39-7.45 (m, **3H Ar**), 7.62-7.65 (m, **2H Ar**).

### 7,8-Diméthoxy-1-méthyl-[5-(3-trifluorométhyl)phényl]-1,3-dihydro-2H-1,4-benzodiazépin-2-one 4p

En remplaçant dans l'exemple **4a** le 3-(7,8-diméthoxy-2-oxo-2,3-dihydro-1*H*-1,4-benzodiazépin-5-yl)-benzonitrile **3a** par la 7,8-diméthoxy-[5-(3-trifluorométhyl)phényl]-1,3-dihydro-2*H-*1,4-benzodiazépin-2-one **3d,** on obtient de la même manière le produit titre. Rdt : 95%. RMN ¹H (CDCl₃, 200 MHz) : δ 3.42 (s, 3H, NC**H₃**), 3.81 (s, 3H, OC**H₃**), 4.00 (s, 3H, OC**H₃**), 4.30 (system AB, Δδ = 1.0, J_{AB} = 10 Hz, 2H, C**H₂**), 6.65 (s, **1H** Ar), 6.81 (s, **1H Ar**), 7.52-7.57 (m, **1H Ar**), 7.72-7.74 (m, **1H Ar**), 7.88-7.91 (m, **1H Ar**), 7.95 (s, **1H Ar**).

### 7,8-diméthoxy-1-éthyl-5-[3-(trifluorométhyl)phényl]-1,3-dihydro-2H-1,4-benzodiazépin-2-one 4q

En remplaçant dans l'exemple **4a** le 3-(7,8-diméthoxy-2-oxo-2,3-dihydro-1*H*-1,4-benzodiazépin-5-yl)-benzonitrile **3a** par le 7,8-Diméthoxy-[5-(3-trifluorométhyl) phényl]-1,3-dihydro-2*H*-1,4-benzodiazépin-2-one **3d,** et le iodométhane par le iodoéthane, on obtient de la même manière le produit titre. Rdt : 71%. RMN ¹H (CDCl₃, 300MHz) : δ 1.14 (t, 3H, -C**H₃**), 3.76 (s, 3H, OC**H₃**), 3.99 (s, 3H, OC**H₃**), 4.00 (système ABX, Δδ = 0.61, J_{AX} = J_{BX} = 13.9, 2H, -NC**H₂**), 4.31 (système AB, Δδ = 1.01, J_{AB} = 10, 2H, C**H₂**), 6.62 (s, 1**H Ar**), 6.87 (s, 1**H Ar**), 7.51-7.57 (t, **1H Ar**), 7.71-7.74 (d, 1**H Ar**), 7.80-7.85 (d, 1**H** Ar), 7.96 (s, 1**H Ar**).

### 5-[3-(trifiuorométhyl)phényl]-7,8-diméthoxy-1-n-propyl-1,3-dihydro-1,4-benzodiazépin-2-one, 4r

En remplaçant dans l'exemple **4a** la 3-(7,8-diméthoxy-2-oxo-2,3-dihydro-1H-1,4-benzodiazépin-5-yl)benzonitrile **3a** par la 7,8-diméthoxy-5-(3-trifluomméthylphényl)-1,3-dihydro-1,4-benzodiazépin-2-one 3d et l'iodométhane par le bromopropane, on obtient de la même manière le produit titre. Rdt : 75%. F : 135-137°C. ¹H-RMN (CDCl₃, 200MHz) : δ 0,74-0,82 (m, 3H, CH₂C**H₃**), 1,49-1,63 (m, 2H, C**H₂**CH₃), 3,49-3,62 (m, 1H, C**H**), 3,78 (s, 3H, OC**H₃**), 4,01 (s, 3H, OC**H₃**), 4,34 (système AB, Δδ = 1,00, J_{AB} = 10,0, 2H, C**H₂**),4,31-4,42 (m, 1H, C**H**), 6,65 (s, 1**H Ar**), 7,89 (s, 1**H Ar**), 7,53-7,99 (m, **4H Ar**).

### 1-benzyl-5-[3-(trifluorométhyl)phényl]-7,8-diméthoxy-1,3-dihydro-1,4-benzodiazépin-2-one, 4s

En remplaçant dans l'exemple **4a** la 3-(7,8-diméthoxy-2-oxo-2,3-dihydro-1H-1,4-benzodiazépin-5-yl)benzonitrile **3a** par la 7,8-diméthoxy-5-(3-trifluorométhylphényl)-1,3-dihydro-1,4-benzodiazépin-2-one **3d** et l'iodométhane par le bromure de benzyl, on obtient de la même manière le produit titre. Rdt : 80%. F : 175-178°C. ¹H-RMN (CDCl₃, 200MHz) : δ 3,71 (s, 3H, OC**H₃**), 3,90-3,98 (m, 4H, OC**H₃**+C**H**), , 4,92-4,97 (m, 1H, C**H**), 5,19 (système AB, Δδ = 0,80, J_{AB} = 15, 2H, C**H₂**),6,51 (s, 1**H Ar**), 6,88 (s, 1**H Ar**), 7,08-7,76 (m, **9H Ar**).

### - Oxydation de la fonction nitrile de type 5.

### 3-(7,8-Diméthoxy-1-méthyl-2-oxo-2,3-dihydro-1H-1,4-benzodiazépin-5-yl)-benzamide 5a

A une solution de 3-(7,8-diméthoxy-1-méthyl-2-oxo-2,3-dihydro-1*H-*1,4-benzodiazépin-5-yl)-benzonitrile **4a** (7.5 g, 22.4 mmoles) dans l'éthanol (100 mL), additionner goutte à goutte H₂O₂ aqueous (30% w/w dans l'eau, 7.6 mL) et NaOH (0.5 M, 10 mL). Agiter le mélange 2 heures à 60°C. Refroidir à température ambiante puis ajouter une solution saturée de Na₂S₂O₃ (10 mL) et agiter 15 minutes. Evaporer l'éthanol; diluer le milieu réactionnel avec de l'eau (100mL) et extraire à l'AcOEt (3X100 mL). Sécher les phases organiques rassemblées sur Na₂SO₄, filtrer & évaporer à sec. Recrystalliser dans l'éthanol pour obtenir un solide blanc. Rdt : 75%. RMN ¹H (CDCL₃, 300MHz) : 3.42 (s, 3H, NC**H₃**), 3.74 (s, 3H, OC**H₃**), 3.99 (s, 3H, OC**H₃**), 4.33 (systéme AB, = 1.0, J_{AB} = 10 Hz, 2H, C**H₂**), 5.89 (s, 1H, N**H**H), 6.39 (s, 1H, NH**H**), 6.65 (s, **1H Ar**), 6.80 (s, **1H Ar**), 7.47-7.50 (t, **1H Ar**), 7.77 (d, **1H Ar**), 7.95 (d, **1H Ar**), 8.15 (s, **1H Ar**).

### 3-(6-Bromo-7,8-diméthoxy-1-méthyl-2-oxo-2,3-dihydro-1H-1,4-benzodiazépin-5-yl)benzamide, 5b

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(6-bromo-7,8-diméthoxy-1-méthyl-2-oxo-2,3-dihydro-1*H*-1,4-benzodiazèpin-5-yl)benzonitrile **24b,** on obtient de la même manière le produit titre. Rdt : 65%. RMN ¹H (CDCl₃, 200 MHz) : δ 3.39 (s, 3H, NC**H₃**), 3.86 (s, 3H, OC**H₃**), 4.00 (s, 3H, OC**H₃**),4.30 (system AB, Δδ = 0.9, J_{AB} = 10 Hz, 2H, NC**H₂**), 6.83 (s, **1H Ar**), 7.44 (t, **1H Ar**), 7.63 (d, **1H Ar**), 7.86 (d, **1H Ar**), 8.00 (s, **1H Ar**).

### 3-(7,8-Diméthoxy-1-methyl-2-oxo-6-phényl-2,3-dihydro-1H-1,4-benzodiazépin-5-yl)benzamide, 5c

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(7,8-diméthoxy-1-methyl-2-oxo-6-phényl-2,3-dihydro-1*H*-1,4-benzodiazèpin-5-yl)benzonitrile **25b,** on obtient de la même manière le produit titre. Rdt : 95%. RMN ¹H (DMSO-d₆, 300 MHz) : δ 3.39 (s, 3H, NC**H₃**), 3.46 (s, 3H, OC**H₃**), 4.02 (s, 3H, OC**H₃**),4.40 (system AB, Δδ = 0.5, J_{AB} = 10 Hz, 2H, NC**H₂**), 6.82-6.85 (m, **2H Ar**), 7.00-7.15 (m, **4H Ar**), 7.24-7.26 (m, **2H Ar**), 7.45 (s, **1H Ar**), 7.62-7.64 (d, **1H Ar**), 10.21 (s, 2H, N**H₂**).

### 3-(9-Bromo-7,8-diméthoxy-1-méthyl-2-oxo-2,3-dihydro-1H-1,4-benzodiazépin-5-yl)benzamide, 5d

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(9-bromo-7,8-diméthoxy-1-méthyl-2-oxo-2,3-dihydro-1H-1,4-benzodiazèpin-5-yl)benzonitrile **24d,** on obtient de la même manière le produit titre. Rdt : 60%. RMN ¹H (DMSO-d₆, 200 MHz) : δ 3.18 (s, 3H, NC**H₃**), 3.74 (s, 3H, OC**H₃**), 3.89 (s, 3H, OC**H₃**),4.20 (system AB, Δδ = 0.8, J_{AB} = 11 Hz, 2H, NC**H₂**), 6.87 (s, **1H Ar**), 7.46 (large s, 1H, N**H**), 7.54-7.61 (t, **1H Ar**), 7.85-7.89 (d, **1H Ar**), 8.03-8.20 (m, 3H, **2H Ar** et N**H**).

### 3-(7,8-diméthoxy-2-oxo-2,3-dihydro-1H-1,4-benzodiazépin-5-yl)benzamide, 5e

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(7,8-diméthoxy-2-oxo-2,3-dihydro-1H-1,4-benzodiazépin-5-yl)-benzonitrile **3a,** on obtient de la même manière le produit titre. Rdt : 67%. RMN ¹H (CDCl₃, 200 MHz) : δ 3.19 (s, 3H, OC**H₃**), 3.44 (s, 3H, OC**H₃**), 3.73 (s, 2H, C**H₂**), 6.14 (s, **1H Ar**), 6.22 (large s, 2H, N**H₂**), 6.34 (s, **1H Ar**), 6.95-7.02 (t, **1H Ar**), 7.20-7.24 (d, **1H Ar**), 7.50-7.54 (d, **1H Ar**), 7.62 (s, **1H Ar**), 9.70 (s, 1H échangeable, N**H**).

### 3-(7,8-diméthoxy-1-propyl-2-oxo-2,3-dihydro-1H-1,4-benzodiazépin-5-yl)benzamide, 5f

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(7,8-dimethoxy-1-propyl-2-oxo-2,3-dihydro-1H-1,4-benzodiazepin-5-yl)benzonitrile **4l**, on obtient de la même manière le produit titre. Rdt : 50%. RMN ¹H (DMSO-d₆, 200 MHz) : δ 0.61 (t, 3H, C**H₃**), 1.28-1.42 (m, 2H, C**H₂**CH3), 3.38-3.45 (m, 1H, NC**H₂**), 3.64 (s, 3H, OC**H₃**), 3.92 (s, 3H, OC**H₃**), 4.16 (système AB, Δδ = 0.79, J_{AB} = 10 Hz, 2H, C**H₂**), 4.21-4.27 (m, 1H, NC**H₂**), 6.68 (s, **1H Ar**), 7.19 (s, **1H Ar**), 7.43-7.58 (m, 2H, 1H N**H₂**, **1H Ar**), 7.69-7.74 (d, **1H Ar**), 8.00-8.17 (m, 3H, 1H N**H₂**, **2H Ar**).

### 3-(1-éthyl-7,8-diméthoxy-2-oxo-2,3-dihydro-1H-1,4-benzodiazépin-5-yl)benzamide, 5g

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(7,8-dimethoxy-1-éthyl-2-oxo-2,3-dihydro-1H-1,4-benzodiazepin-5-yl)benzonitrile **4k,** on obtient de la même manière le produit titre. Rdt : 53%. RMN ¹H (DMSO-d₆, 200 MHz) : δ 0.96 (t, 3H, C**H₃**), 3.33-3.42 (m, 1H, NC**H₂**), 3.64 (s, 3H, OC**H₃**), 3.92 (s, 3H, OC**H₃**), 4.14 (système AB, Δδ = 0.79, J_{AB} = 10 Hz, 2H, C**H₂**), 4.21-4.28 (m, 1H, NC**H₂**), 6.67 (s, **1H Ar**), 7.16 (s, **1H Ar**), 7.43 (s, 1H N**H₂** échangeable), 7.50-7.58 (t, **1H Ar**), 7.71-7.75 (d, **1H Ar**), 8.00-8.14 (m, 2H, 1H N**H₂**, **1H Ar**).

### 3-(1-benzyl-7,8-diméthoxy-2-oxo-2,3-dihydro-1H-1,4-benzodiazépin-5-yl)benzamide, 5h

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(1-benzyl-7,8-dimethoxy-2-oxo-2,3-dihydro-1H-1,4-benzodiazepin-5-yl)benzonitrile **4m** on obtient de la même manière le produit titre. Rdt : 38%. RMN ¹H (CDCl₃, 300 MHz) : δ 3.68 (s, 3H, OC**H₃**), 3.87 (s, 3H, OC**H₃**), 4.42 (system AB, Δδ = 0.96, J_{AB} = 10 Hz, 2H, C**H₂**), 5.16 (system AB, Δδ = 0.73 J_{AB} = 15 Hz, 2H, NC**H₂**), 5.66 (s, 1H N**H₂**), 6.17 (s, 1H N**H₂**), 6.51 (s, **1H Ar**), 7.09-7.19 (m, **6H Ar**), 7.46-7.49 (d, **2H Ar**), 7.89 (s, **1H Ar**), 7.99-8.01 (d, 1H, **1H Ar**).

### éthyl {5-[3-(aminocarbonyl)phényl]-7,8-diméthoxy-2-oxo-2,3-dihydro-1H-1,4-benzodiazépin-1-yl}acétate, 5i

En remplaçant dans l'exemple **5a** le 3-(7,8-diméthoxy-1-méthyl-2-oxo-2,3-dihydro-1H-1,4-benzodiazépin-5-yl)benzonitrile **4a** par le éthyl [5-(3-cyanophényl)-7,8-diméthoxy-2-oxo-2,3-dihydro-1H-1,4-benzodiazépin-1-yl]acétate **4n,** on obtient de la même manière le produit titre. Rdt : 44%. RMN ¹H (CDCl₃, 300 MHz) : δ 1.22-1.27 (t, 3H C**H₃**), 3.75 (s, 3H, OC**H₃**), 3.96 (s, 3H, OC**H₃**), 4.15-4.24 (m, 2H C**H₂**-CH₃), 4.38 (system AB, Δδ = 0.90, J_{AB} = 10 Hz, 2H, C**H₂**), 4.50-4.57 (m, 2H NC**H₂**), 5.70 (s, 1H N**H₂**), 6.65 (s, 1H N**H₂**), 6.85 (s, **1H Ar**), 7.50-7.55 (t, **1H Ar**), 7.80-7.82 (d, **1H Ar**), 7.97-7.99 (d, **1H Ar**), 8.17 (s, **1H Ar**).

### 3-(7,8-diméthoxy-1,3-diméthyl-2-oxo-2,3-dihydro-1H-1,4-benzodiazépin-5-yl) benzamide, 5j

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(7,8-diméthoxy-1,3-diméthyl-2-oxo-2,3-dihydro-1H-1,4-benzodiazépin-5-yl) benzonitrile **7d,** on obtient de la même manière le produit titre. Rdt : 44%. RMN ¹H (CDCl₃, 200 MHz) : δ 1.75-1.78 (d, 3H C**H₃**), 3.46 (s, 3H, NC**H₃**), 3.76-3.80 (m, 4H, C**H** et OC**H₃**), 4.01 (s, 3H OC**H₃**), 5.68 (s, 1H échangeable N**H**), 7.18 (s, 1H échangeable N**H**), 6.72 (s, **1H Ar**), 6.82 (s, **1H Ar**), 7.48-7.56 (t, **1H Ar**), 7.79-7.81 (d, **1H Ar**), 7.93-7.95 (d, **1H Ar**), 8.15 (s, **1H Ar**).

### 3-[3-(3,4-dichlorobenzyl)-7,8-diméthoxy-1-méthyl-2-oxo-2,3-dihydro-1H-1,4-benzodiazépin-5-yl]benzamide, 5k

En remplaçant dans l'exemple **5a** le 3-(7,8-diméthoxy-1-méthyl-2-oxo-2,3-dihydro-1H-1,4-benzodiazépin-5-yl)benzonitrile **4a** par le 7,8-diméthoxy-1,3-diméthyl-5-(3-trifluorométhylphenyl)-1,3-dihydro-2H-1,4-benzodiazépin-2-one **7c,** on obtient de la même manière le produit titre. Rdt : 58%. RMN ¹H (CDCl₃, 200 MHz) : δ 3.42 (s, 3H, NC**H₃**), 3.50-3.54 (m, 2H C**H₂**), 3.71 (s, 4H, C**H** et OC**H₃**), 3.97 (s, 3H OC**H₃**), 6.01 (s, 1H échangeable N**H**), 6.15 (s, 1H échangeable N**H**), 6.60 (s, **1H Ar**), 6.77 (s, **1H Ar**), 7.21-7.50 (m, **4H Ar**), 7.76-7.80 (d, **1H Ar**), 7.89-7.92 (d, **1H Ar**), 8.03 (s, **1H Ar**).

### 3-(8-méthoxy-1-méthyl-2-oxo-2,3-dihydro-1H-1,4-benzodiazépin-5-yl)benzamide, 51

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(8-méthoxy-1-méthyl-2-oxo-2,3-dihydro-1H-1,4-benzodiazépin-5-yl)benzonitrile **29a"** on obtient de la même manière le produit titre. Rdt : 60%. RMN ¹H (CDCl₃, 300 MHz) : δ 3.43 (s, 3H, NC**H₃**), 3.92 (s, 3H, OC**H₃**), 4.32 (system AB, Δδ = 0.99 J_{AB} = 10 Hz, 2H, C**H₂**), 5.70 (s, 1H échangeable N**H**), 6.28 (s, 1H échangeable N**H**), 6.75-6.78 (d, **1H Ar**), 6.84 (s, **1H Ar**), 7.19-7.22 (d, 1**H Ar**), 7.47-7.52 (t, **1H Ar**), 7.76-7.78 (d, **1H Ar**), 7.94-7.96 (d, **1H Ar**), 8.09 (s, **1H Ar**).

### 3-(7,8-Diméthoxy-1-methyl-2-oxo-9-phényl-2,3-dihydro-1H-1,4-benzodiazépin-5-yl)benzamide, 5m

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(7,8-diméthoxy-1-methyl-2-oxo-9-phényl-2,3-dihydro-1*H*-1,4-benzodiazèpin-5-yl)benzonitrile **25a,** on obtient de la même manière le produit titre. Rdt : 75%. RMN ¹H (CDCl₃, 200 MHz) : δ 2.44 (s, 3H, NC**H₃**), 3.62 (s, 3H, OC**H₃**), 3.77 (s, 3H, OC**H₃**),4.41 (system AB, Δδ = 0.5, J_{AB} = 10 Hz, 2H, NC**H₂**), 6.74 (s, **1H Ar**), 7.44-7.57 (m, **6H Ar**), 7.88-7.98 (m, **2H Ar**), 8.25 (s, **1H Ar**), 10.19 (s, 2H, N**H₂**).

### 3-(6,8-Dimethoxy-2-oxo-2,3-dihydro-1H-1,4-benzodiazepin-5-yl)benzamide, 5n

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(6,8-dimethoxy-2-oxo-2,3-dihydro-1*H-*1,4-benzodiazepin-5-yl)benzonitrile, **28g,** on obtient de la même manière le produit titre. Rdt : 80%. RMN ¹H (DMSO-d₆, 300 MHz) : δ 3.42 (s, 3H, OC**H₃**), 3.85 (s, 3H, OC**H₃**),4.17 (system AB, Δδ = 0.7, J_{AB} = 11 Hz, 2H, NC**H₂**), 6.44-6.45 (m, **2H Ar**), 7.35-7.88 (m, **4H Ar**), 8.03 (s, 2H, N**H₂**), 10.39 (s, 1H, N**H**).

### 3-(6,8-Dimethoxy-1-methyl-2-oxo-2,3-dihydro-1H-1,4-benzodiazepin-5-yl)benzamide, 5o

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3-(6,8-dimethoxy-1-methyl-2-oxo-2,3-dihydro-1*H-*1,4-benzodiazepin-5-yl)benzonitrile, **29b,** on obtient de la même manière le produit titre. Rdt : 73%. RMN ¹H (CDCl₃, 300 MHz) : δ 3.37 (s, 3H, NC**H₃**), 3.48 (s, 3H, OC**H₃**), 3.90 (s, 3H, OC**H₃**),4.28 (system AB, Δδ = 0.8, J_{AB} = 9 Hz, 2H, NC**H₂**), 6.33 (s, **2H Ar**), 6.44 (s, **2H Ar**), 7.39-8.01 (m, **4H Ar**).

### - Couplages Palladium de type 6.

### Tert-butyl 3-[3-(7,8-diméthoxy-1-méthyl-2-oxo-2,3-dihydro-1H-1,4-benzodiazépin-5-yl)phényl]propynylcarbamate, 6a

Agiter 20 heures, sous atmosphère inerte à 50°C, un mélange de 100 mg (0.257 mmole) de 5-(3-bromophényl)-7,8-diméthoxy-1-méthyl-1,3-dihydro-2*H*-1,4-benzodiazépin-2-one 4b, 200 mg de *tert*-butyl prop-2-ynylcarbamate (1.3 mmoles), 9.0 mg de CuI, 5.0 mg de PdCl₂ 18.0 mg de PPh₃, 0.5 mL de TEA, 2 mL de CH₃CN. Evaporer à sec et purifier par chromatographie sur silice (Et2O/CH₂Cl₂ : 1/1). Recristralliser dans Et₂O/Pentane. Rdt : 50%. RMN ¹H (CDCl₃, 200 MHz) : δ 1.47 (s, 9H, C(C**H₃**)₃), 3.41 (s, 3H, NC**H₃**), 3.76 (s, 3H, OC**H₃**), 3.99 (s, 3H, OC**H₃**), 4.20 (system AB, Δδ = 1.0, J_{AB} = 10 Hz, 2H, NC**H₂**), 4.12-4.15 (d, 2H, C**H₂**), 6.65 (s, **1H Ar**), 6.79 (s, **1H Ar**), 7.47-7.73 (m, **4H Ar**).

### 7,8-Diméthoxy-5-(3'-hex-1-ynylphényl)-1-N-méthyl-1,3-dihydro-2H-1,4-benzodiazépin-2-one, 6b

En remplaçant dans l'exemple **6a** le *tert*-butyl prop-2-ynylcarbamate par le 1-hexyne, on obtient de la même manière le produit titre. Rdt : 89%. RMN ¹H (CDCl₃, 200 MHz) : δ 0.91-0.98 (t, 3H, C**H₃**), 1.40-1.60 (m, 4H, 2 C**H₂**), 2.36-2.43 (t, 2H, C**H₂**), 3.40 (s, 3H, NC**H₃**), 3.76 (s, 3H, OC**H₃**), 3.99 (s, 3H, OC**H₃**), 4.20 (system AB, Δδ = 1.0, J_{AB} = 11 Hz, 2H, NC**H₂**), 6.67 (s, **1H Ar**), 6.78 (s, **1H Ar**), 7.29-7.36 (t, **1H Ar**), 7.46-7.50 (d, **1H** Ar), 7.54-7.58 (d, **1H Ar**), 7.65 (d, **1H Ar**).

### 7,8-Diméthoxy-1-méthyl-5-[3-(3-pipéridin-1-ylprop-1-ynyl)phényl]-1,3-dihydro-2H-1,4-benzodiazépin-2-one, 6c

En remplaçant dans l'exemple **6a** le *tert*-butyl prop-2-ynylcarbamate par le bromure de propargyl, le PdCl₂ par le Pd(OAc)₂,la TEA par la pipéridine et le CH₃CN par le THF, on obtient de la même manière le produit titre. Rdt : 20%. RMN ¹H (CDCl₃, 200 MHz) : δ 1.48 (m, 2H, C**H₂**), 1.62-1.65 (m, 4H, 2 C**H₂**), 2.56 (m, 4H, 2 C**H₂**), 3.41 (s, 3H, NC**H₃**), 3.46 (s, 2H, C**H₂**), 3.76 (s, 3H, OC**H₃**), 3.99 (s, 3H, OC**H₃**), 4.20 (system AB, Δδ = 1.0, J_{AB} = 11 Hz, 2H, NC**H₂**), 6.67 (s, **1H Ar**), 6.78 (s, **1H Ar**), 7.34-7.71 (m, **4H Ar**).

### 6-[3-(7,8-Diméthoxy-1-méthyl-2-oxo-2,3-dihydro-1H-1,4-benzodiazépin-5-yl)phényl]hex-5-ynenitrile, 6d

En remplaçant dans l'exemple **6a** le *tert*-butyl prop-2-ynylcarbamate par le 5-cyano-1-pentyne, on obtient de la même manière le produit titre. Rdt : 81%. RMN ¹H (CDCl₃, 300 MHz) : δ 1.94-2.01 (m, 2H, C**H₂**), 2.54-2.63 (m, 4H, 2 C**H₂**), 3.41 (s, 3H, NC**H₃**), 3.76 (s, 3H, OC**H₃**), 3.99 (s, 3H, OC**H₃**), 4.20 (system AB, Δδ = 1.0, J_{AB} = 11 Hz, 2H, NC**H₂**), 6.66 (s, **1H Ar**), 6.79 (s, **1H Ar**), 7.32-7.37 (t, **1H Ar**), 7.47-7.50 (d, **1H Ar**), 7.56-7.59 (d, **1H Ar**), 7.69 (d, **1H Ar**).

### 7,8-Diméthoxy-5-(3'-hexylphényl)-1-N-méthyl-1,3-dihydro-2H-1,4-benzodiazépin-2-one, 6e

Laisser sous agitation un mélange de 68 mg (0.172 mmole) de 7,8-diméthoxy-5-(3'-hex-1-ynylphényl)-1-*N-*méthyl-1,3-dihydro-2*H-*1,4-benzodiazépin-2-one **6b,** 20 mg de Pd/C à 10% en poids dans 10 mL de MeOH et 2 mL de CH₂Cl₂ à pression atmosphérique d'H₂ pendant 3 heures. Filtrer la suspension sur célite, rincer 3X10mL de CH₂CL₂/MeOH : 8/2. Evaporer à sec et purifier par chromatographie sur silice (CH₂Cl₂/Et₂O: 1/1). Rdt : 65%. RMN ¹H (CDCl₃, 300 MHz) : δ 0.88 (m, 3H, C**H₃**), 1.31 (m, 8H, 4 C**H₂**), 2.60-2.65 (t, 2H, C**H₂**), 3.40 (s, 3H, NC**H₃**), 3.74 (s, 3H, OC**H₃**), 3.98 (s, 3H, OC**H₃**), 4.20 (system AB, Δδ = 1.0, J_{AB} = 11 Hz, 2H, NC**H₂**), 6.71 (s, **1H Ar**), 6.78 (s, **1H Ar**), 7.28-7.49 (m, **4H Ar**).

### Tert-butyl 3-[3-(7,8-diméthoxy-1-méthyl-2-oxo-2,3-dihydro-1H-1,4-benzodiazépin-5-yl)phényl]propylcarbamate, 6f

En remplaçant dans l'exemple **6e** le 7,8-diméthoxy-5-(3'-hex-1-ynylphényl)-1-*N-*méthyl-1,3-dihydro-2*H*-1,4-benzodiazépin-2-one 6b par le tert-butyl 3-[3-(7,8-diméthoxy-1-méthyl-2-oxo-2,3-dihydro-1*H*-1,4-benzodiazépin-5-yl)phényl]propynylcarbamate **6a,** on obtient de la même manière le produit titre. Rdt : 58%. RMN ¹H (CDCl₃, 200 MHz) : δ 1.44 (s, 9H, C(C**H₃**)₃), 1.81 (m, 2H, C**H₂**), 2.63-2.70 (m, 2H, C**H₂**), 3.14-3.22 (m, 2H, C**H₂**), 3.41 (s, 3H, NC**H₃**), 3.75 (s, 3H, OC**H₃**), 3.99 (s, 3H, OC**H₃**), 4.30 (system AB, Δδ = 1.0, J_{AB} = 10 Hz, 2H, NC**H₂**), 4.56 (large s, 1H, N**H**), 6.70 (s, **1H Ar**), 6.78 (s, **1H Ar**), 7.26-7.69 (m, **4H Ar**).

### 6-[3-(7,8-Diméthoxy-1-méthyl-2-oxo-2,3-dihydro-1H-1,4-benzodiazépin-5-yl)phényl]hexanenitrile, 6g

En remplaçant dans l'exemple **6e** le 7,8-diméthoxy-5-(3'-hex-1-ynylphényl)-1-*N-*méthyl-1,3-dihydro-2*H*-1,4-benzodiazépin-2-one **6b** par le 6-[3-(7,8-diméthoxy-1-méthyl-2-oxo-2,3-dihydro-1*H*-1,4-benzodiazépin-5-yl)phényl]hex-5-ynenitrile **6d,** on obtient de la même manière le produit titre. Rdt : 60%. RMN ¹H (CDCl₃, 200 MHz) : δ 1.44-1.70 (m, 6H, 3 C**H₂**), 2.34 (m, 2H, C**H₂**), 2.67 (m, 2H, C**H₂**), 3.41 (s, 3H, NC**H₃**), 3.75 (s, 3H, OC**H₃**), 3.99 (s, 3H, OC**H₃**), 4.30 (system AB, Δδ = 1.0, J_{AB} = 10 Hz, 2H, NC**H₂**), 6.71 (s, **1H Ar**), 6.79 (s, **1H Ar**), 7.28-7.53 (m, **4H Ar**).

### 5-[3-(3-aminopropyl)phényl-7,8-diméthoxy-1-méthyl-1,3-dihydro-2H-1,4-benzodiazépin-2-one trifluoroacétate, 6h

Agiter 3 heures, sous atmosphère inerte à température ambiante, un mélange de 25 mg (0.05 mmole) de *tert*-butyl 3-[3-(7,8-diméthoxy-1-méthyl-2-oxo-2,3-dihydro-1*H*-1,4-benzodiazépin-5-yl)phényl]propylcarbamate **6f,** d'acide trifluoroacétique (40 L, 0.52 mmole) et de CH₂Cl₂. Evaporer à sec puis cristalliser le produit dans l'éther (13 mg). Rdt : 50%. RMN ¹H (DMSO-d₆, 300 MHz) : δ 1.84 (m, 2H, C**H₂**), 2.67-2.69 (m, 2H, C**H₂**), 2.72-2.81 (m, 2H, C**H₂**), 3.34 (s, 3H, NC**H₃**), 3.68 (s, 3H, OC**H₃**), 3.93 (s, **3H,** OC**H₃**), 4.15 (system AB, Δδ = 0.7, J_{AB} = 11 Hz, 2H, NC**H₂**), 6.69 (s, **1H Ar**), 7.13 (s, **1H Ar**), 7.40-7.60 (m, **4H Ar**), 7.83 (large s, 3H, NH₃⁺).

### 6-[3-(7,8-diméthoxy-1-méthyl-2-oxo-2,3-dihydro-1H-1,4-benzodiazépin-5-yl)phényl]hexanamide, 6i

En remplaçant dans l'exemple **5a** le 3-(7,8-diméthoxy-1-méthyl-2-oxo-2,3-dihydro-1H-1,4-benzodiazépin-5-yl)benzonitrile **4a** par le 6-[3-(7,8-diméthoxy-1-méthyl-2-oxo-2,3-dihydro-1*H*-1,4-benzodiazépin-5-yl)phényl]hexanenitrile **6g,** on obtient de la même manière le produit titre. Rdt : 50%. RMN ¹H (CDCl₃, 200 MHz) : δ 1.37 (m, 2H, C**H₂**), 1.62 (m, 4H, 2 C**H₂**), 2.17-2.21 (m, 2H, C**H₂**), 2.60-2.64 (m, 2H, C**H₂**), 3.40 (s, 3H, NC**H₃**); 3.74 (s, 3H, OC**H₃**), 3.98 (s, 3H, OC**H₃**), 4.30 (system AB, Δδ = 1.0, J_{AB} = 11 Hz, 2H, NC**H₂**), 5.46 (large s, 2H, N**H₂**), 6.70 (s, **1H Ar**), 6.78 (s, **1H Ar**), 7.28-7.50 (m, **4H Ar**).

### 5-(4'-chloro-1,1'-biphényl-3-yl)-7,8-diméthoxy-1-méthyl-1,3-dihydro-2H-1,4-benzodiazépin-2-one, 6j

En remplaçant dans l'exemple **17a** le 5-chloro-7,8-diméthoxy-1-méthyl-1,3-dihydro-1,4-benzodiazépin-2-one **16a** par le 5-(3-bromophényl)-7,8-diméthoxy-1-méthyl-1,3-dihydro-2*H-*1,4-benzodiazépin-2-one **4b** et l'acide 3-chlorobenzène boronique par l'acide 4-chlorobenzène boronique on obtient de la même manière le produit titre. Rdt : 27%. F : 131 °C. RMN ¹H (DMSO, 300MHz) : δ 3.32 (s, 3H, NC**H₃**), 3.63 (s, 3H, OC**H₃**), 3.91 (s, 3H, OC**H₃**), 4.16 (système AB, Δδ = 1.0, J_{AB} = 10, 2H, C**H₂**), 6.74 (s, 1**H Ar**), 7.10 (s, 1**H Ar**), 7.51-7.80 (m, **8H Ar**).

### 5-{3-[3-(benzyloxy)prop-1-ynyl]phényl}-1-éthyl-7,8-diméthoxy-1,3-dihydro-2H-1,4-benzodiazépin-2-one, 6k

En remplaçant dans l'exemple **6a** le *tert*-butyl prop-2-ynylcarbamate par le [(prop-2-ynyloxy)méthyl]benzène on obtient de la même manière le produit titre. Rdt : 24%. F : °C. RMN ¹H (CDCl₃, 200MHz) : δ 1.12 (s, 3H, C**H₃**), 3.50-3.72 (m, 7H, 1H NC**H₂** + 2H OC**H₂**Ph+ 1H C**H₂** + OC**H₃**), 3.97 (s, 3H, OC**H₃**)**,** 4.24-4,38 (m, 2H, 1H ≡C-C**H₂** + 1H NC**H₂**), 4,66 (s, 1H, ≡C-C**H₂**), 4.73-4.78 (m, 1H C**H₂**), 6.63 (s, 1**H Ar**), 6,84 (s. 1**H** Ar), 7.26-7.83 (m, **9H Ar**).

### 3'-(7,8-diméthoxy-1-méthyl-2-oxo-2,3-dihydro-1H-1,4-benzodiazépin-5-yl)-1,1'-biphényl-3-carbonitrile, 6l

En remplaçant dans l'exemple **17a** le 5-chloro-7,8-diméthoxy-1-méthyl-1,3-dihydro-1,4-benzodiazépin-2-one **16a** par le 5-(3-bromophényl)-7,8-diméthoxy-1-méthyl-1,3-dihydro-2*H*-1,4-benzodiazépin-2-one **4b** et l'acide 3-chlorobenzène boronique par l'acide 3-cyanobenzène boronique on obtient de la même manière le produit titre. Rdt : 54%. RMN ¹H (CDCl3, 200MHz) : δ 3,43 (s, 3H, NC**H₃**), 3.76 (s, 3H, OC**H₃**), 4.00 (s, 3H, OC**H₃**), 4.33 (système AB, Δδ = 1.00, J_{AB} = 10, 2H, C**H₂**), 6.72 (s, 1**H Ar**), 6.81 (s, 1**H Ar**), 7.47-8.00 (m, **8H Ar**).

### 3'-(7,8-diméthoxy-1-méthyl-2-oxo-2,3-dihydro-1H-1,4-benzodiazépin-5-yl)-1,1'-biphényl-4-carbonitrile, 6m

En remplaçant dans l'exemple **17a** le 5-chloro-7,8-diméthoxy-1-méthyl-1,3-dihydro-1,4-benzodiazépin-2-one **16a** par le 5-(3-bromophényl)-7,8-diméthoxy-1-méthyl-1,3-dihydro-2*H-*1,4-benzodiazépin-2-one **4b** et l'acide 3-chlorobenzène boronique par l'acide 4-cyanobenzène boronique on obtient de la même manière le produit titre. Rdt : 42%. RMN ¹H (CDCl3, 200MHz) : δ 3.42 (s, 3H, NC**H₃**), 3.75 (s, 3H, OC**H₃**), 4.00 (s, 3H, OC**H₃**), 4.33 (système AB, Δδ = 1.00, J_{AB} = 10.29, 2H, C**H₂**), 6.73 (s, 1**H** Ar), 6.80 (s, 1**H** Ar), 7.40-7.70 (m, **7H Ar**), 7.97 (s, 1**H Ar**).

### 3'-(7,8-diméthoxy-1-méthyl-2-oxo-2,3-dihydro-1H-1,4-benzodiazépin-5-yl)-1,1'-biphényl-4-carboxamide, 6n

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3'-(7,8-diméthoxy-1-méthyl-2-oxo-2,3-dihydro-1H-1,4-benzodiazépin-5-yl)-1,1'-biphényl-3-carbonitrile **61,** on obtient de la même manière le produit titre. Rdt : 44%. RMN ¹H (CDCl₃, 300 MHz) : δ 3,44 (s, 3H, NC**H₃**), 3.77 (s, 3H, OC**H₃**), 4.01 (s, 3H, OC**H₃**), 4.35 (système AB, Δδ = 1.00, J_{AB} = 10, 2H, C**H₂**), 5.66 (s, 1H échangeable N**H₂**), 6.08 (s, 1H échangeable N**H₂**), 6.76 (s, 1**H Ar**), 6.82 (s, 1**H Ar**), 7.48-7.74 (m, **5H Ar**), 7.89-7.92 (d, **2H Ar**), 7.99 (s, 1**H Ar**).

### 3'-(7,8-diméthoxy-1-méthyl-2-oxo-2,3-dihydro-1H-1,4-benzodiazépin-5-yl)-1,1'-biphényl-3-carboxamide, 6o

En remplaçant dans l'exemple **5a** le 3-(7,8-diméthoxy-1-méthyl-2-oxo-2,3-dihydro-1H-1,4-benzodiazépin-5-yl)benzonitrile **4a** par le 3'-(7,8-diméthoxy-1-méthyl-2-oxo-2,3-dihydro-1H-1,4-benzodiazépin-5-yl)-1,1'-biphényl-4-carbonitrile **6m**, on obtient de la même manière le produit titre. Rdt : 44%. RMN ¹H (CDCl₃, 300 MHz) : δ 3.44 (s, 3H, NC**H₃**), 3.77 (s, 3H, OC**H**₃), 4.01 (s, 3H, OC**H**₃), 4.34 (système AB, Δδ = 1.00, J_{AB} = 10, 2H, C**H₂**), 5.65 (s, 1H échangeable N**H₂**), 6.19 (s, 1H échangeable N**H₂**), 6.76 (s, 1**H Ar**), 6.82 (s, 1**H Ar**), 7.47-7.62 (m, 4**H Ar**), 7.71-7.80 (m, 3**H Ar**), 7.99 (s, 1**H Ar**).

### - Alkylation du carbone 3 de type 7.

### 3-(3,4-dichlorobenzyl)-1-ethyl-7,8-diméthoxy-5-phényl-1,3-dihydro-2H-1,4-benzodiazépin-2-one, 7a

Placer le LDA (2M, 0.31 mL, 0.62 mmols) à -78°C sous argon et agitation, additionner gouttes à gouttes le 7,8-Diméthoxy-1-éthyl-5-phényl-1,3-dihydro-2H-1,4-benzodiazépin-2-one **4o** (100 mg, 0.31 mmols) dissout dans le THF (4mL). Laisser revenir la température à 0°C pendant 30 minutes, puis replacer à -78°C, pour additionner le 3.4 dichlorobenzyle (81.5mg, 0.35 mmols). Laisser revenir à r.t. et agiter une nuit. Quencher à NaCl sat. (15mL) et extraire par 3 * 10 mL de dichlorométhane. Réunir les phases organiques et les laver avec 30 mL d'eau, sécher sur Na₂SO₄, filtrer, évaporer à sec et purifier par chromatographie sur silice (AcOEt/Hex : 1/1). On obtient ainsi le produit titre. Rdt : 53%. RMN ¹H (CDCl₃, 200 MHz) : δ 1.03-1.11 (t, 3**H**, C**H₃**), 3.50-3.68 (m, 4**H**, C**H**, C**H₂** et 1H NC**H₂**), 3.73 (s, 3H, OC**H₃**), 3.96 (s, 3H, OC**H₃**), 6.63 (s, **1H Ar**), 6.82 (s, **1H Ar**), 7.19-7.55 (m, 8**H Ar**).

### 3-[3-(3,4-dichlorobenzyl)-7,8-diméthoxy-1-méthyl-2-oxo-2,3-dihydro-1H-1,4-benzodiazépin-5-yl]benzonitrile, 7b

En remplaçant dans l'exemple **7a** le 7,8-Diméthoxy-1-éthyl-5-phényl-1,3-dihydro-2H-1,4-benzodiazépin-2-one **4o** par le 3-(7,8-diméthoxy-2-oxo-2,3-dihydro-1*H*-1,4-benzodiazépin-5-yl)-benzonitrile **3a**, on obtient de la même manière le produit titre. Rdt : 53%. RMN ¹H (CDCl₃, 200 MHz) : δ 3.46 (s, 3H, NC**H₃**), 3.50-3.57 (m, 2H, C**H** et 1H C**H₂**), 3.72-3.78 (m, 4H, 1H C**H₂** et OC**H₃**), 4.01 (s, 3H OC**H₃**), 6.60 (s, **1H Ar**), 6.82 (s, **1H Ar**), 7.21-7.26 (m, **1H Ar**), 7.38-7.42 (m, **1H Ar**), 7.50-7.60 (m, **2H Ar**), 7.75-7.80 (d, **1H Ar**), 7.88-7.91 (m, **2H Ar**).

### 7,8-diméthoxy-1,3-diméthyl-5-(3-trifluorométhylphényl)-1,3-dihydro-2H-1,4-benzodiazépin-2-one, 7c

En remplaçant dans l'exemple **7a** le 3.4 dichlorobenzyle par le iodométhane, on obtient de la même manière le produit titre. Rdt : 45 %. RMN ¹H (CDCl₃, 200 MHz) : δ 1.62-1.79 (d, 3H C**H₃**), 3.40 (s, 3H, NC**H₃**), 3.78-3.80 (m, 4H, CH et OC**H₃**), 4.03 (s, 3H OC**H₃**), 6.68 (s, **1H Ar**), 6.84 (s, **1H Ar**), 7.52-7.60 (t, **1H Ar**), 7.72-7.77 (d, **1H Ar**), 7.90-7.95 (m, **2H Ar**).

### 3-(7,8-diméthoxy-1,3-diméthyl-2-oxo-2,3-dihydro-1H-1,4-benzodiazépin-5-yl)benzonitrile, 7d

En remplaçant dans l'exemple **7a** le 7,8-Diméthoxy-1-éthyl-5-phényl-1,3-dihydro-2H-1,4-benzodiazépin-2-one **4o** par le 3-(7,8-diméthoxy-2-oxo-2,3-dihydro-1*H*-1,4-benzodiazépin-5-yl)-benzonitrile **3a**, et le 3.4 dichlorobenzyle par le iodométhane, on obtient de la même manière le produit titre. Rdt : 53%. RMN ¹H (CDCl₃, 200 MHz) : δ 1.72-1.75 (d, 3H C**H₃**), 3.42 (s, 3H, NC**H₃**), 3.75-3.77 (m, 4H, C**H** et OC**H₃**), 3.99 (s, 3H OC**H₃**), 6.61 (s, **1H Ar**), 6.80 (s, **1H Ar**), 7.48-7.56 (t, **1H** Ar), 7.71-7.75 (d, **1H** Ar), 7.90-7.97 (m, **2H Ar**).

### - Réduction du nitrile de type 8.

### 5-[3-(aminométhyl)phényl]-7,8-diméthoxy-1-méthyl-1,3-dihydro-2H-1,4-benzodiazépin-2-one, 8a

Placer 1 nuit sous pression d'hydrogène le 3-(7,8-Diméthoxy-1-méthyl-2-oxo-2,3-dihydro-1*H*-1,4-benzodiazépin-5-yl)-benzamide **5a** (100 mg, 0.30 mmole), le Nickel de Raney (1 pointe de spatule), ammoniaque (30%, 1mL) et le méthanol (10 mL). Filtrer sur cellite, et laver par 3 fois 25 mL de méthanol. Evaporer et reprendre le résidu dans 20 mL de CH₂Cl₂. Laver la phase organique par 3 fois 20 mL ammoniaque 30% et 1 fois 20 mL d'eau. Sécher la phase organique sur Na₂SO₄, filtrer, évaporer à sec. On obtient ainsi le produit titre. Rdt : 77%. RMN ¹H (CDCl₃, 200 MHz) : δ 1.28 (s, 2H échangeable N**H₂**), 1.79 (s, 2H C**H₂**), 3.42 (s, 3H, NC**H₃**), 3.76 (s, 3H, OC**H₃**), 3.94 (s, 2H C**H₂**), 4.00 (s, 3H OC**H₃**), 4.31 (système AB, Δδ = 0.98, J_{AB} = 10, 2H, C**H₂**), 6.72 (s, **1H Ar**), 6.81 (s, **1H Ar**), 7.36-7.56 (m, **3H Ar**), 7.71 (s, **1H Ar**).

### N-[3-(7,8-diméthoxy-1-méthyl-2-oxo-2,3-dihydro-1H-1,4-benzodiazépin-5-yl)benzyl]acétamide, 8b

Placer sous agitation 1 nuit le 5-[3-(aminométhyl)phényl]-7,8-diméthoxy-1-méthyl-1,3-dihydro-2H-1,4-benzodiazépin-2-one **8a** (50mg, 0.15 mmole), l'anhydride acétique (16 µL, 0.18 mmole), la pyridine (29.6 µL, 0.37 mmole), 3mL de CH₂Cl₂. Evaporer à sec, purifier par chromatographie sur silice (CH₂Cl₂/MeOH : 9/1 ). Ainsi on obtient le produit titré. Rdt : 77%. RMN ¹H (CDCl₃, 200 MHz) : δ 2.00 (s, 3H C**H₃**), 3.38 (s, 3H, NC**H₃**), 3.76 (s, 3H, OC**H₃**), 3.94 (s, 3H OC**H₃**), 4.25 (système AB, Δδ = 0.98, J_{AB} = 10, 2H, C**H₂**), 4.43 (s, 2H C**H₂**), 5.99 (s, 1H échangeable N**H**), 6.65 (s, **1H Ar**), 6.77 (s, **1H Ar**), 7.26-7.40 (m, **3H Ar**), 7.63 (s, **1H Ar**).

### - Synthèses des thiobenzamides de type 9

### 3-(7,8-diméthoxy-1-méthyl-2-oxo-2,3-dihydro-1H-1,4-benzodiazépin-5-yl)thiobenzamide, 9a

Chauffer à 90°C pendant une nuit un mélange de 500mg de 3-(7,8-diméthoxy-1-méthyl-2,3-dihydro-1H-1,4-benzodiazépin-5-yl)benzamide **5a** (1,41 mmole), 285mg de réactif de Lawesson (0,71 mmole) dans 30 ml de toluène. Ajouter 150 ml d'H₂O et extraire avec 4 X 100 ml d'AcOEt ; sécher sur MgSO₄, évaporer l'AcOEt et purifier par chromatographie sur silice (AcOEt 5 / CH₂Cl₂ 4 / EtOH 1). Rdt : 70%. ¹H-RMN (CDCl₃, 300MHz) : δ 3,41 (s, 3H, NC**H₃**), 3,77-3,84 (m, 4H, 1**H** C**H₂** + OC**H₃**), 3,99 (s, 3H, OC**H₃**), 4,81 (m, 1H, C**H₂**), 6,68 (s, 1**H Ar**), 6,79 (s, 1**H Ar**), 7,40-8,20 (m, 6H, N**H₂** + 4**H Ar**)**.** Masse : (M+H)⁺ = 370,09.

### 7,8-diméthoxy-1-méthyl-5-[3-(4-phényl-1, 3-thiazol-2-yl)phényl]-1,3-dihydro-2H-1,4-benzodiazépin-2-one, 9b

Chauffer à 100°C pendant une nuit un mélange de 20mg de 3-(7,8-diméthoxy-1,3-dihydro-2H-1,4-benzodiazépin-5-yl)thiobenzamide **9a** (0,05 mmole), 12mg de Bromoacétophénone (0,06 mmole) dans 3 ml d'EtOH. Ajouter 50 ml d'H₂O et extraire avec 4 X 50 ml d'AcOEt ; sécher sur MgSO₄, évaporer l'AcOEt et purifier par chromatographie sur silice (AcOEt 1/ Hex 1). Rdt : 90%. ¹H-RMN (CDCl₃, 300MHz) : δ 3,45 (s, 3H, NC**H₃**), 3,77 (s, 3H, OC**H₃**), 4,01 (s, 3H, OC**H₃**), 4,36 (système AB, Δδ = 1,02, J_{AB} = 10,5, 2H, C**H₂**), 6,76 (s, 1**H Ar**), 6,82 (s, 1**H Ar**), 7,44-8,00 (m, 9H Ar),8,34 (s, **1H thiazol**). Masse : (M+H)⁺ = 470,14.

### - Synthèse des benzodiazepin-thiones de type 10

### 7,8-dimethoxy-5-phenyl-1,3-dihydro-2H-1,4-benzodiazepine-2-thione, 10a

Placer à reflux pendant une nuit le 7,8-Diméthoxy-5-phényl-1,3-dihydro-2H-1,4-benzodiazépin-2-one **3c** (400mg, 1.35 mmols), 600 mg du réactif de Lawesson, et 70 mL de toluène anhydre. Evaporer à sec, purifier par chromatographie sur silice (AcOEt). Ainsi on obtient le produit titré. Rdt : 85%. RMN ¹H (CDCl₃, 200 MHz) : δ 3.68 (s, 3H, OC**H₃**), 3.98 (s, 3H OC**H₃**), 4.77 (s, 2H, C**H₂**), 6.46 (s, 1H Ar), 7.20 (s, **1H Ar**), 7.36-7.40 (m, **2H Ar**), 7.54-7.60 (m, **3H Ar**), 13.02 (s, 1H échangeable N**H**).

### 7,8-diethoxy-5-phenyl-1,3-dihydro-2H-1,4-benzodiazepine-2-thione, 10b

En remplaçant dans l'exemple **11a** le 7,8-Diméthoxy-5-phényl-1,3-dihydro-2H-1,4-benzodiazépin-2-one **3c** par le 7,8-diéthoxy-5-phényl-1,3-dihydro-2H-1,4-benzodiazépin-2-one 3e. Ainsi on obtient le produit titré. Rdt : 53%. RMN ¹H (CDCl₃, 300 MHz) : δ 1.33-1.38 (t, 3H, C**H₃**), 1.50-1.54 (t, 3H, C**H₃**), 3.83-3.90 (q, 2H, C**H₂**), 4.15-4.22 (q, 2H, C**H₂**), 4.75 (s, 1H échangeable NH), 6.47 (s, **1H Ar**), 7.12 (s, **1H Ar**), 7.36-7.39 (m, **2H Ar**), 7.48-7.62 (m, **3H Ar**).

### 1-ethyl-7,8-dimethoxy-5-phenyl-1,3-dihydro-2H-1,4-benzodiazepine-2-thione, 10c

En remplaçant dans l'exemple **4a** le 3-(7,8-diméthoxy-2-oxo-2,3-dihydro-1*H*-1,4-benzodiazépin-5-yl)-benzonitrile **3a** par la 7,8-diethoxy-5-phenyl-1,3-dihydro-2H-1,4-benzodiazepine-2-thione **11b** et le iodométhane par le iodoéthane, on obtient de la même manière le produit titre. Rdt : 75%. RMN ¹H (CDCl₃, 300 MHz) : δ 1.16-1.21 (t, 3H C**H₃**), 3.76 (s, 3H, OC**H₃**), 3.95-4.02 (m, 4H, OC**H₃** et 1H NC**H₂**), 4.76 (system AB, Δδ = 1.22, J_{AB} = 10 Hz, 2H, C**H₂**), 5.05-5.12 (m, 1H NC**H₂**), 6.68 (s, **1H** Ar), 6.91 (s, 1H Ar), 7.42-7.48 (m, 3H Ar), 7.65-7.68 (m, 2H Ar).

### 5-(3-cyanophényl)-7,8-diméthoxy-1,3-dihydro-2H-1,4-benzodiazépin-2-thione, 10d

Chauffer à reflux pendant 45 min un mélange de 200mg de 5-(3-cyanophényl)-7,8-diméthoxy-1,3-dihydro-2H-1,4-benzodiazépin-2-one **3a** (0,62 mmole), 150mg de P₂S₅ (0,34 mmole) dans 4 ml de pyridine. Refroidir à 0°C et ajouter la suspension à 100ml d'une solution saturée de NaCl, filtrer, rincer à l'eau froide, sécher sous vide, triturer dans l'Et₂O, filtrer puis sécher. Rdt : 76%. ¹H-RMN (CDCl₃, 300MHz) : δ 3,76 (s, 3H, OC**H₃**), 3,99 (s, 3H, OC**H₃**), 4,78 (m, 2H, C**H₂**), 6,63 (s, 1**H Ar**), 6,68 (s, 1**H Ar**), 7,50-7,92 (m, 3H, 4**H Ar**), 9,84 (s, 1H échangeable, N**H** ). Masse : (M+H)⁺ = 338,10.

### - Réaction de substitution nucléophile (de type 11 et 12) d'amines sur une thione.

### 3-(8,9-diméthoxy-4Himidazo[1,2-a][1,4]benzodiazépin-6-yl)benzonitrile, 11a

Chauffer à reflux pendant la nuit un mélange de 100mg de 5-(3-cyanophényl)-7,8-diméthoxy-1,3-dihydro-2H-1,4-benzodiazépin-2-thione 10d (0,30 mmole), 40mg d'aminoacétaldiéthylacétal (0,30 mmole) et de 3mg d'acide paratoluènesulfonique monohydrate (0,015mmole) dans 5 ml de butanol. Ajouter 55mg d'acide paratoluènesulfonique monohydrate (0,29mmole) et chauffer à reflux 6h. Evaporer le butanol au 2/3, ajouter 100 ml d'H₂O glace et basifier la solution jusqu'à PH = 8, 9 avec NaOH 1N. Extraire avec 3 X 100 ml d'AcOEt ; sécher sur MgSO₄, évaporer l'AcOEt et purifier par chromatographie sur silice (AcOEt). Rdt: 55%. ¹H-RMN (CDCl₃, 200MHz) : δ 3,77 (s, 3H, OC**H₃**), 4,02 (m, 4H, 1NC**H₂** + OC**H₃**), 5,33 (m, 1HC**H₂**), 6,68 (s, 1**H Ar**), 6,99 (s, 1**H Ar**), 7,13 (d, J₁₂ = 1,22, 1**Himidazol),** 7,36 (d, J₂₁ = 1,22, 1**Himidazol**),7,48-7,84 (m, 3H, **4H Ar**).

### 3-(8,9-diméthoxy-4Himidazo[1,2-a][1,4]benzodiazépin-6-yl)benzamide, 11b

En remplaçant dans l'exemple **5a** le 3-(7,8-diméthoxy-1-méthyl-2-oxo-2,3-dihydro-1H-1,4-benzodiazépin-5-yl) benzonitrile **4a** par le 3-(8,9-diméthoxy-4Himidazo[1,2-a][1,4]benzodiazépin-6-yl)benzonitrile **11a,**on obtient de la même manière le produit titre. Rdt : 70%. ¹H-RMN (CDCl₃, 300MHz) : δ 3,76 (s, 3H, OC**H₃**), 4,03 (m, 4H, 1HC**H₂** + OC**H₃**), 5,33 (m, 1HC**H₂**), 6,53 (s, 1**H Ar**), 6,75 (s, **1H Ar**), 6,99 (s, 1**Himidazol),** 7,27 (s, 1**Himidazol**),7,42-8,09 (m, 3H, 4**H Ar).**

### 3-(7,8-diméthoxy-2-méthylamino-1,3-dihydro-3H-1,4-benzodiazépin-5-yl)benzonitrile, 12a

Ajouter à une solution de 100mg de 5-(3-cyanophényl)-7,8-diméthoxy-1,3-dihydro-2H-1,4-benzodiazépin-2-thione **10d** (0,30 mmole) dans 3 ml d'EtOH et 0,5 ml de DMSO, 1,5 ml de méthylamine 2N dans le THF. Chauffer une nuit à 110°C. Ajouter 100 ml d'H₂O et extraire avec 3 X 100 ml de CH₂Cl₂; sécher sur MgSO₄, évaporer le CH₂Cl₂ et purifier par chromatographie sur silice (AcOEt ; AcOEt 5 / CH₂Cl₂ 4 / EtOH 1). Rdt : 30%. ¹H-RMN (DMSO, 300MHz) : δ 2,96 (s, 3H, NC**H₃**), 3,59-3,71 (m, 4H, 1HC**H₂** + OC**H₃**), 3,98 (s, 3H, OC**H₃**), 4,54 (m, 1HC**H₂**), 4,89 (s large, 1Héchangeable, N**H**), 6,54 (s, **1H Ar**), 6,81 (s, 1**H** Ar), 7,70-7,80 (m, 4**H Ar**). Masse : (M+H)⁺ 335,1.

### - Synthèse de l'iminochlorure de type 16.

### 6,7-dimethoxy-2H-3,1-benzoxazine-2,4(1H)-dione, 13a

Verser le 2-amino-4,5-dimethoxybenzoic acid (25g, 0.13 mols) dans le THF (400 mL), puis additionner le chloroformiate de benzyle (54mL, 0.38 mols) sous très forte agitation. Placer la mixture à reflux pendant une nuit. Evaporer à sec, puis sécher le résidu sous vide. Verser de l'éther (425 mL) sur le résidu, additionner PBr3 (11.88 mL, 0.13 mols), et placer à reflux pendant 48 heures. Filtrer le mélange réactionnel, laver 3* avec 150 mL d'éther. Reprendre le résidu dans l'éther et agiter 1 heure, puis filtrer, laver, sécher. On obtient 27 g de produit titre sous forme d'une poudre blanche. Rdt : 96 %. RMN ¹H (DMSO-d₆, 200 MHz) : δ 3.82 (s, 3H, OC**H₃**), 3.88 (s, 3H, OC**H₃**), 6.66 (s, **1H Ar**), 7.27 (s, **1H Ar**), 11.58 (s, 1H échangeable N**H**).

### 6,7-diméthoxy-1-méthyl-1,2-dihydro-4H-3,1-benzoxazine-2,4-dione, 14a

Ajouter sous atmosphère inerte à une solution de 500 mg (3.06 mmoles) de 6,7-diméthoxy-1,2-dihydro-4H-3,1-benzoxazine-2,4-dione **13a,** dans 6 mL de DMF anhydre 134 mg (3.37 mmoles) de NaH à 60% dans l'huile. Après 10 min à température ambiante, ajouter au goutte à goutte 219 µL (3.52 mmoles) de MeI. Laisser 3 heures à température ambiante. Ajouter 40 mL d'un mélange eau-glace. Filtrer le précipité et laver 2 fois avec 1 mL d'EtOH et 3 mL d'Et2O. On obtient 320 mg de produit titre sous forme d'une poudre blanche. Rdt : 59%. RMN ¹H (CDC13, 300MHz) : δ 3.31 (s, 3H, -C**H₃**), 3.82 (s, 3H, OC**H₃)**, 3.96 (s, 3H, OC**H₃)**, 6.85 (s, **1H Ar**), 7.32 (s, **1H Ar**).

### 7,8-diméthoxy-1-méthyl-3,4-dihydro-1H-1,4-benzodiazépine-2,5-dione, 15a

Chauffer à reflux pendant 6 heures un mélange de 320 mg (1.35 mmole) de 6,7-diméthoxy-1-méthyl-1,2-dihydro-4H-3,1-benzoxazine-2,4-dione **14a,** 452 mg (3.24 mmoles) de chlorhydrate de glycinate de méthyle dans 4 mL de pyridine. Ajouter 3 mL d'AcOH et chauffer à 130°C pendant 12 heures. Evaporer à sec. Ajouter 10 mL d'un mélange H2O/glace. Laisser cristalliser pendant 30 minutes à 0°C. Filtrer et laver 2 fois avec 2 mL d'H2O, 2 fois avec 1 mL d'EtOH et 2 fois avec 5 mL d'Et2O. Recristalliser dans l'EtOH. On obtient 240 mg de produit titre sous forme de cristaux incolores. Rdt : 71%. F : 260-263°C. RMN ¹H (CDCl3, 300MHz) : δ 3.42 (s, 3H, NC**H₃**), 3.75-3.92 (m, 2H, C**H₂**), 3.98 (s, 6H, 2 x OC**H₃**), 6.39 (s, 1H échangeable, N**H**), 6.69 (s, **1H Ar**), 7.37 (s, **1H Ar**).

### 5-chloro-7,8-diméthoxy-1-méthyl-1,3-dihydro-1,4-benzodiazépin-2-one, 16a

Chauffer en tube scellé pendant ¾ d'heure à 125°C une solution de 100 mg (0.40 mmole) de 7,8-diméthoxy-1-méthyl-3,4-dihydro-1H-1,4-benzodiazépine-2,5-dione **15a,** 280 µL de diméthylaniline, 800 µL de POCl3, dans 10 mL de CHCl3 anhydre. Laisser revenir à température ambiante. Ajouter 3 g de silice et 5 mL de CH2Cl2. Ajouter à 0°C, 1 mL de triéthylamine. Evaporer à sec. Purifier par chromatographie (AcOEt 1/ Hexane 1 puis, AcOEt). Triturer dans 1 mL d'Et2O. Filtrer et laver 2 fois avec 2 mL de pentane. On obtient 93 mg de produit titre sous forme d'une poudre blanche. Rdt : 87%. RMN ¹H (CDCl3, 200MHz) : δ 3.42 (s, 3H, NC**H₃**), 3.77 (s large, 1H de C**H₂**), 3.99 (s, 3H, OC**H₃**), 4.00 (s, 3H, OC**H₃**), 4.65 (s large, 1H de C**H₂**), 6,71 (s. **1H Ar**), 7.22 (s, **1H Ar**).

### - Couplages Palladium, sur l'iminochlorure, de type 17.

### 5-(3-chlorophényl)-7,8-diméthoxy-1-méthyl-1,3-dihydro-1,4-benzodiazépin-2-one, 17a

Chauffer à 115°C pendant 12 h et sous atmosphère inerte un mélange de 5-chloro-7,8-diméthoxy-1-méthyl-1,3-dihydro-1,4-benzodiazépin-2-one **16a** (130 mg, 0.48 mmoles), 3-chlorobenzène boronique (90.8 mg, 0.58 mmoles), K₃PO₄ (118 mg, 0.56 mmole), tétrakis(triphénylphosphine) Pd (0) (15 mg, 0.01 mmols) dans 3 mL de DMF. Laisser revenir à température ambiante. Ajouter 30 mL d'H₂O et extraire 3 fois avec 30 mL d'Et₂O. Sécher les fractions organiques sur Na₂SO₄. Evaporer à sec, purifier par chromatographie (AcOEt). Recristalliser dans EtOH. On obtient 103 mg de produit titre sous forme de cristaux blanc. Rdt : 62%. F : 109-111°C. RMN ¹H (CDCl₃, 200MHz) : δ 3.41 (s, 3H, NC**H₃**), 3.77 (s, 3H, OC**H₃**), 3.99 (s, 3H, OC**H₃**), 4.34 (système AB, Δδ = 1.02, J_{AB} = 10, 2H, C**H₂**), 6.67 (s, 1**H Ar**), 6.79 (s, 1**H Ar**), 7.19-7.69 (m, **4H Ar**).

### 7,8-diméthoxy-1-méthyl-5-(3-pyridyl)-1,3-dihydro-1,4-benzodiazépin-2-one, 17b

En remplaçant dans l'exemple **17a** l'acide 3-chlorobenzène boronique par l'acide pyridine-3-boronique-1,3-propanediol ester cyclique on obtient de la même manière le produit titre. Rdt : 58%. F : 177-179°C. RMN ¹H (CDCl₃, 200MHz) : δ 3.42 (s, 3H, NC**H₃**), 3.76 (s, 3H, OC**H₃**), 3.99 (s, 3H, OC**H₃**), 4.32 (système AB, Δδ = 1.03, J_{AB} = 10, 2H, C**H₂**), 6.67 (s, 1**H Ar**), 6.80 (s, 1**H Ar**), 7.34-7.40 (m, 1**H Ar**), 8.03-8.09 (m, 1**H Ar**), 8.68-8.71 (m, 2**H Ar**).

### 7,8-diméthoxy-1-méthyl-5-(3-nitrophényl)-1,3-dihydro-1,4-benzodiazépin-2-one, 17c

En remplaçant dans l'exemple **17a** l'acide 3-chlorobenzène boronique par l'acide 3-nitrobenzène boronique on obtient de la même manière le produit titre. Rdt : 43%. F : 152-155°C. RMN ¹H (CDCl₃, 300MHz) : δ 3.43 (s, 3H, NC**H₃**), 3.76 (s, 3H, OC**H₃**), 4.01 (s, 3H, OC**H₃**), 4.35 (système AB, Δδ = 1.03, J_{AB} = 10, 2H, C**H₂**), 6.64 (s, 1**H Ar**), 6.82 (s, 1**H Ar**), 7.59-7.64 (m, 1**H Ar**), 8.08-8.12 (m, 1**H Ar**), 8.31-8.35 (m, 1**H Ar**), 8.49-8.51 (m, 1**H Ar**).

### 5-(7,8-diméthoxy-1-méthyl-2-oxo-2,3-dihydro-1H-1,4-benzodiazépin-5-yl)-2benzonitrile, 17d

En remplaçant dans l'exemple **17a** l'acide 3-chlorobenzène boronique par l'Acide-3-cyano-4-[(4-méthoxybenzyl)oxy]phénylboronique **37c** on obtient de la même manière le produit titre. Rdt : 20%. ¹H-RMN (DMSO, 300MHz) : δ 3,32 (s, 3H, NC**H₃**), 3,65 (s, 3H, OC**H₃**), 3,70-3,77 (m, 4H, 1HC**H₂** + BnOC**H₃**) 3,90 (s, 3H, OC**H₃**), 4,51 (m, 1HC**H₂**), 5,26 (s, 2H, PhC**H₂**), 6,70 (s, 1**H** Ar), 6,98 (m, **2HBn**), 7,09 (s, 1**H Ar**), 7,43 (m, **2HBn**), 7,55-7,87 (m, 3**H Ar**).

### 5-(3-acétylphényl)-7,8-diméthoxy-1-méthyl-1,3-dihydro-1,4-benzodiazépin-2-one, 17e

En remplaçant dans l'exemple **17a** l'acide 3-chlorobenzène boronique par l'acide 3-acétylbenzène boronique on obtient de la même manière le produit titre. Rdt : 43%. F : 148-150°C. RMN ¹H (CDCl₃, 200MHz) : δ 2.64 (s, 3H, C**H₃**CO), 3.42 (s, 3H, NC**H₃**), 3.74 (s, 3H, OC**H₃**), 4.00 (s, 3H, OC**H₃**), 4.30 (système AB, Δδ = 1.03, J_{AB} = 10, 2H, C**H₂**), 6.65 (s, 1**H Ar**), 6.81 (s, 1**H Ar**), 7.48-8.23 (m, 4**H Ar**).

### 5-(4-isoquinoléinyl)-7,8-diméthoxy-1-méthyl-1,3-dihydro-1,4-benzodiazépin-2-one, 17f

En remplaçant dans l'exemple **17a** l'acide 3-chlorobenzène boronique par le 2-(isoquinoléin-4-yl)-4,4,5,5-tétraméthyl-1,3-dioxaborolane on obtient de la même manière le produit titre. Rdt : 34%. F : 131-135°C. RMN ¹H (CDCl₃, 300MHz) : δ 3.53 (s, 3H, NC**H₃**), 3.55 (s, 3H, OC**H₃**), 4.00 (s, 3H, OC**H₃**), 4.50 (système AB, Δδ = 1,00, J_{AB} = 10.5, 2H, C**H₂**), 6.45 (s, 1**H Ar**), 6.84 (s, 1**H Ar**), 7.60-7.69 (m, 2**H Ar**), 7.89-8.08 (m, 2**H Ar**), 8.55 (s, 1**H Ar**), 9.32 (s, 1**H Ar**).

### 7,8-diméthoxy-5-(3-hydroxyméthylphényl)-1-méthyl-1,3-dihydro₋2H-1,4-benzodiazépin-2-one, 17g

En remplaçant dans l'exemple **17a** l'acide 3-chlorobenzène boronique par l'acide 3-hydroxyméthylbenzène boronique on obtient de la même manière le produit titre. Rdt : 25%. F : 143-146°C. ¹H-RMN (DMSO, 300MHz) : δ 3,44 (s, 3H, NC**H₃**), 3,63 (s, 3H, OC**H₃**), 4,01 (s, 3H, OC**H₃**), 4,35 (système AB, Δδ = 0,25, J_{AB} = 12,8, 2H, C**H₂**), 4,60 (s, 2H, C**H₂**OH) 6,69 (s, 1**H Ar**), 6,91 (s, **1H Ar**), 7,61-7,74 (m, 4**H Ar**).

### 7,8-diméthoxy-5-(3-hydroxyméthylphényl)-1-méthyl-3-propyl-1,3-dihydro-2H-1,4-benzodiazépin-2-one, 17h

Ajouter, à 0°C et sous atmosphère inerte, à une solution de 140 mg (0,47 mmole) de 7,8-diméthoxy-5-(3-hydroxyméthylphényl)-1-méthyl-1,3-dihydro-1,4-benzodiazépin-2-one 17g dans 5 ml de DMF, 21mg (0,52 mmole) de NaH à 60% dans l'huile. Laisser sous agitation à température ambiante pendant 1h. Ajouter goutte à goutte à 0°C, 50 µl de Bromopropane. Laisser sous agitation à température ambiante pendant la nuit. Ajouter 50 ml d'H₂O et extraire avec 3 X 50 ml d'AcOEt ; sécher sur MgSO₄, évaporer l'AcOEt et purifier par chromatographie sur silice (AcOEt 1 / Hex 1 puis AcOEt). Recristalliser dans CHCl₃ / cHex. Rdt : 11%. F : 134-136°C. ¹H-RMN (DMSO, 200MHz) : δ 0,85-0,93 (m, 3H, C**H₃**), 1,35-1,55 (m, 2H, C**H₂**CH₃), 1,97-2,01 (m, 2H, CHC**H₂**), 3,31 (s, 3H, NC**H₃**), 3,42-3,47 (m, 1H, C**H**CH₂), 3;62 (s, 3H, OC**H₃**), 3,88 (s, 3H, OC**H₃**), 4,50 (d, J = 5,6, 2H, C**H₂**OH), 5,21 (t, J = 5,6, 1H, O**H**), 6,66 (s, 1**H Ar**), 7,08 (s, **1H Ar**), 7,38-7,53 (m, 4**H Ar**). Masse : (M + H)⁺ = 383,27.

### 5-(3-aminophényl)-7,8-diméthoxy-1-méthyl-1,3-dihydro-2H-1,4-benzodiazépin-2-one, 17i

Placer sous agitation le 7,8-diméthoxy-1-méthyl-5-(3-nitrophényl)-1,3-dihydro-1,4-benzodiazépin-2-one **17c** (100 mg, 0.28 mmols) , 10 mg de Pd/C (10 %), dans 2 mL de méthanol, sous pression d'hydrogène pendant 8 heures. Filtrer le tout sur cellite, laver par 3 fois 25 mL de méthanol. Evaporer à sec, purifier par chromatographie (AcOEt). Ainsi on obtient le produit titre sous forme de cristaux blanc. Rdt : 77%. RMN ¹H (CDCl₃, 300MHz) : δ 1.60 (s, 2H échangeable N**H₂**), 3.40 (s, 3H, NC**H₃**), 3.78 (s, 3H, OC**H₃**), 3.99 (s, 3H, OC**H₃**), 4.38 (système AB, Δδ = 1.03, J_{AB} = 10, 2H, C**H₂**), 6.76 (m, 3**H Ar**), 6.89-6.91 (d, 1**H Ar**), 7.10 (s, 1**H Ar**), 7.14-7.22 (t, 1**H Ar**).

### 5-(3,4-dichlorophényl)-7,8-diméthoxy-1-méthyl-1,3-dihydro-1,4-benzodiazépin-2-one, 17j

En remplaçant dans l'exemple **17a** l'acide 3-chlorobenzène boronique par l'acide 3,4-dichlorophényl boronique on obtient de la même manière le produit titre. Rdt : 25%. F : 174-177°C. ¹H-RMN (CDCl₃, 200MHz) : δ 3,44 (s, 3H, NC**H₃**), 3,81 (s, 3H, OC**H₃**), 4,02 (s, 3H, OC**H₃**), 4,50 (système AB, Δδ = 1,00, J_{AB} = 10,5, 2H, C**H₂**), 6,67 (s, 1**H Ar**), 6,82 (s, 1**H Ar**), 7,52 (s, 2**H Ar**), 7,83 (s, 1**H Ar**).

### 7,8-diméthoxy-1-méthyl-5-(3-méthylphényl)-1,3-dihydro-1,4-benzodiazépin-2-one, 17k.

En remplaçant dans l'exemple **17a** l'acide 3-chlorobenzène boronique par l'acide 3-méthylbenzène boronique on obtient de la même manière le produit titre. Rdt : 20%. F : 119-121°C. ¹H-RMN (CDCl₃, 300MHz) : δ 2,39 (s, 3H, C**H₃**), 3,40 (s, 3H, NC**H₃**), 3,75 (s, 3H, OC**H₃**), 3,98 (s, 3H, OC**H₃**), 4,31 (système AB, Δδ = 0,99, J_{AB} = 10,3, 2H, C**H₂**), 6,71 (s, 1**H Ar**), 6,78 (s, 1**H Ar**), 7,27-7,28 (m, 2**H Ar**), 7,35-7,38 (m, 1**H Ar**), 7,53 (s, 1**H Ar**).

### 5-(3-formylphényl)-7,8-diméthoxy-1-méthyl-1,3-dihydro-1,4-benzodiazépin-2-one, 171

En remplaçant dans l'exemple **17a** l'acide 3-chlorobenzène boronique par l'acide 3-formylbenzène boronique on obtient de la même manière le produit titre. Rdt : 25%. F : 175-178°C. ¹H-RMN (CDCl₃, 200MHz) : δ 3,45 (s, 3H, NC**H₃**), 3,77 (s, 3H, OC**H₃**), 4,03 (s, 3H, OC**H₃**), 4,39 (système AB, Δδ = 1,01, J_{AB} = 10,5, 2H, C**H₂**), 6,68 (s, 1**H** Ar), 6,84 (s, 1**H** Ar), 7,60-7,67 (m, 1**H Ar**), 8,01-8,05 (m, 2H **Ar**), 8,18 (s, 1**H Ar**), 10,09 (s, 1**H**, C**H**O).

### Chlorhydrate de 1a 5-[3-(benzylaminométhyl)phényl]-7,8-diméthoxy-1-méthyl-1,3-dihydro-2H-1,4-benzodiazépin-2-one, 17m

Chauffer à reflux pendant une nuit un mélange de.200 mg (0,53 mmole) 5-(3-formylphényl)-7,8-diméthoxy-1-méthyl-1,3-dihydro-1,4-benzodiazépin-2-one 17**Hy**, 60µl (0,56mmole) de benzylamine dans 5 ml de MeOH. Rajouter par petite portion et à 0°C 53 mg (1,4 mmole) de NaBH₄. Laisser sous agitation 1h à température ambiante. Evaporer le MeOH. Ajouter 30 ml d'H₂O, extraire avec 3x 50 ml d'AcOEt. Ajouter 10 ml de CH₂Cl₂ et faire buller à 0°C HCl jusqu'à saturation de la solution. Evaporer le solvant puis triturer à 1' Et₂O en éliminant plusieurs fois le surnageant. Recristalliser dans un mélange EtOH/Et₂O ; filtrer puis sécher. Rdt : 75%. F : 88-90°C. ¹H-RMN (DMSO, 200MHz) : δ 3,41 (s, 3H, NC**H₃**), 3,50-3,69 (m, 4H, 1HC**H₂** + OC**H₃**), 3,98 (s, 3H, OC**H₃**), 4,13-4,23 (m, 4H, 2C**H₂P**h), 4,47-4,54 (m, 1H, 1H**CH₂**), 6,70 (s, 1**H Ar**), 7,23 (s, 1**H Ar**), 7,38-7,98 (m, 9**H Ar**). Masse : (M+H)^{┐+}= 430,18.

### N-[3-(7,8-diméthoxy-1-méthyl-2-oxo-2,3-dihydro-1H-1,4-benzodiazépin-5-yl) phényl]acétamide, 17n

En remplaçant dans l'exemple **17a** l'acide 3-chlorobenzène boronique par l'acide 3-acétamidobenzène boronique on obtient de la même manière le produit titre. Rdt : 15%. ¹H-RMN (DMSO, 300MHz) : δ 2,01 (s, 3H, C**H₃**CO), 3,31 (s, 3H, NC**H₃**), 3,66 (s, 3H, OC**H₃**), 3,90 (s, 3H, OC**H₃**), 4,12 (système AB, Δδ = 0,78, J_{AB} = 10,17, 2H, C**H₂**), 6,71 (s, 1**H Ar**), 7,08 (s, 1**H Ar**), 7,29-7,78 (m, 4**H Ar**), 10,01 (s, 1H, N**H**Ac).

### 7,8-diméthoxy-1-méthyl-5-(3,5-méthylènedioxyphényl)-1,3-dihydro-2H-1,4-benzodiazépin-2-one, 17o

En remplaçant dans l'exemple **17a** l'acide 3-chlorobenzène boronique par l'acide 3,5-méthylènedioxybenzène boronique on obtient de la même manière le produit titre. Rdt : 55 %. F : 161-162 °C. ¹H-RMN (CDCl₃, 300MHz) : δ 3,39 (s, 3H, NC**H₃**), 3,74-3,79 (m, 4H, 1HC**H₂** + OC**H₃**), 3,98 (s, 3H, OC**H₃**), 4,72-4,76 (m, 1HC**H₂**), 6,03 (s, 2**H**, C**H₂**O₂), 6,75-7,28 (m, 5**H Ar**).

### - Halogénation régioséléctive et synthèse des benzodiazépines de type 24

### Tert-butyl(2-méthoxy-5-nitrophénoxy)diphénylsilane, 18a

Ajouter sous atmosphère inerte, à 0°C, une solution de 7 g (41 mmoles) de 3-hydroxy-4-méthoxynitrobenzene dissout dans 50 mL de DMF, sur une solution de 2g (50.mmoles) d'hydrure de sodium dissout dans 50 mL de DMF. Après 30 minutes à température ambiante, ajouter au goutte à goutte 12.9 mL (50 mmoles) de *tert*-butylchlorodiphényl silane à 0°C. Laisser agiter 12 heures à température ambiante. Diluer avec 10 volumes d'eau puis extraire 3X50 mL d'Et₂O, laver la phase organique avec 100 mL d'HCl (1M) et NaCl (sat).
Sécher sur Na₂SO₄, filtrer & évaporer à sec. Ainsi on obtient le produit titre. Rdt : 82%. RMN ¹H (CDCl₃, 200 MHz) : δ 1.18 (s, 9H, C(C**H₃**)₃), 3.61 (s, 3H, OC**H₃**), 6.74-6.78 (d, **1H Ar**), 7.34-7.82 (m, **12H Ar**).

### Tert-butyl(2-méthoxy-4-nitrophénoxy)diphénylsilane, 18b

Ajouter sous atmosphère inerte, à 0°C, une solution de 1 g (6.45 mmoles) de 4-nitrocatéchol dissout dans 5 mL de DMF, sur une solution de 480 mg (7.10 mmoles) d'imidazole dissout dans 5 mL de DMF. Après 30 minutes à température ambiante, ajouter au goutte à goutte 1.77 mL (6.77 mmoles) de *tert*-butylchlorodiphényl silane à 0°C. Laisser agiter 12 heures à température ambiante. Diluer avec 10 volumes d'eau puis extraire 3X50 mL d'Et₂O, laver la phase organique avec 100 mL d'HCl (1M) et NaCl (sat).

Sécher sur Na₂SO₄, filtrer & évaporer à sec une huile brune. Solubiliser cette huile dans 30 mL de DMF puis ajouter 2.86 g de K₂CO₃ (20.69 mmoles) et agiter 30 minutes. Ajouter l'iodométhane (1.37 mL, 22.04 mmoles) et laisser réagir 2 heures. Diluer avec 10 volumes d'eau puis extraire 3X100 mL d'Et₂O, laver la phase organique avec 100 mL NaOH 10%. Sécher sur Na₂SO₄, filtrer, évaporer à sec et purifier par chromatographie sur silice (hexane/CH₂Cl₂ : 2/1 ). Rdt : 57%. RMN ¹H (CDCl₃, 200 MHz) : δ 1.15 (s, 9H, C(C**H₃**)₃), 3.58 (s, 3H, OC**H₃**), 6.71-6.76 (d, **1H Ar**), 7.36-7.82 (m, **12H Ar**).

### 3-{[Tert-butyl(diphényl)silyl]oxy}-4-méthoxyaniline, 19a

En remplaçant dans l'exemple **6e** le 7,8-diméthoxy-5-(3'-hex-1-ynylphényl)-1-*N-*méthyl-1,3-dihydro-2*H*-1,4-benzodiazépin-2-one **6b** par le *tert*-butyl(2-méthoxy-5-nitrophénoxy)diphénylsilane **18a,** on obtient de la même manière le produit titre. Rdt : 98%. RMN ¹H (CDCl₃, 300 MHz) : δ 1.12 (s, 9H, C(C**H₃**)₃), 3.59 (s, 3H, OC**H₃**), 6.23 (s, **1H Ar**), 6.24-6.30 (d, **1H Ar**), 6.62-6.65 (d, **1H Ar**), 7.28-7.75 (m, **10H Ar**).

### 4-{[Tert-butyl(diphényl)silyl]oxy}-3-méthoxyaniline, 19b

En remplaçant dans l'exemple **6e** le 7,8-diméthoxy-5-(3'-hex-1-ynylphényl)-1-*N-*méthyl-1,3-dihydro-2*H*-1,4-benzodiazépin-2-one **6b** par le *tert*-butyl(2-méthoxy-4-nitrophénoxy)diphénylsilane **18b**, on obtient de la même manière le produit titre. Rdt : 95%. RMN ¹H (CDCl₃, 200 MHz) : δ 1.08 (s, 9H, C(C**H₃**)₃), 3.53 (s, 3H, OC**H₃**), 5.98-6.02 (m, **1H Ar**), 6.18 (s, **1H Ar**), 6.50-6.54 (m, **1H Ar**), 7.37-7.72 (m, **10H Ar**).

### 3-(2-Amino-5-{[tert-butyl(diphényl)silyl]oxy}-4-méthoxybenzoyl)benzonitrile, 20b

Ajouter, à 0°C et sous atmosphère inerte, à une solution de 10 mL de tribromure de bore (1M/ CH₂Cl₂, 10 mmoles), 3.5 g de 4-{[*tert*-butyl(diphényl)silyl]oxy}-3-méthoxyaniline **19b** (9.22 mmoles) dissout dans 10 mL de 1,2-dichloroéthane, 2.35 g d'isophtalonitrile (18.36 mmoles), et 1.34 g d'AlCl₃ (10.00 mmoles). Agiter 30 minutes à température ambiante. Evaporer le dichlorométhane. Chauffer à reflux 12 heures. Laisser refroidir. Additionner 10 mL d'HCl (1M) à 0°C, agiter à 75°C pendant 1 heure. Additionner 50 mL d'eau, et extraire avec 3 fois 100 mL de CH₂Cl₂. Sécher les fractions organiques sur Na₂SO₄, filtrer, évaporer à sec et purifier par chromatographie sur silice (AcOEt/hexane : 1/3 ). Rdt : 30%. RMN ¹H (CDCl₃, 300 MHz) : δ 1.06 (s, 9H, C(C**H₃**)₃), 3.77 (s, 3H, OC**H₃**), 6.09 (s, **1H Ar**), 6.17 (large s, 2H, N**H₂**), 6.47 (s, **1H Ar**), 7.37-7.86 (m, **14H Ar**).

### 3-(2-Amino-4-{[tert-butyl(diphényl)silyl]oxy}-5-méthoxybenzoyl)benzonitrile, 20a

En remplaçant dans l'exemple **20b** le 4-{[*tert*-butyl(diphényl)silyl]oxy}-3-méthoxyaniline **19b** par le 3-{[*tert*-butyl(diphényl)silyl]oxy}-4-méthoxyaniline, **19**a, on obtient de la même manière le produit titre. Rdt : 38%. RMN ¹H (CDCl₃, 300 MHz) : δ 1.11 (s, 9H, C(C**H₃**)₃), 3.41 (s, 3H, OC**H₃**), 5.90 (large s, 2H, N**H₂**), 6.01 (s, **1H Ar**), 6.67 (s, **1H Ar**), 7.39-7.96 (m, **14H Ar**).

### 2-Bromo-N-[5-{[tert-butyl(diphényl)silyl]oxy}-2-(3-cyanobenzoyl)-4-méthoxyphényl]acétamide, 21b

Additionner, à une solution de 3-(2-Amino-5-{[*tert*-butyl(diphényl)silyl]oxy}-4-méthoxybenzoyl)benzonitrile **20b** (0.4 g, 0.79 mmole) dans le dichlorométhane (5 mL) à 0-5°C, le bromure de bromoacétate (82 L, 0.94 mmole) puis goutte à goutte le Na₂CO₃ 10% (2.4 mL). Agiter 1 heure à cette température. Séparer les deux phases et laver la phase organique avec 10 mL d'eau, sécher Na₂SO₄, filtrer, évaporer à sec (455 mg). Rdt : 92%. RMN ¹H (CDCl₃, 200 MHz) : δ 1.15 (s, 9H, C(C**H₃**)₃), 3.29 (s, 3H, OC**H₃**), 3.95 (s, 2H; C**H₂**), 6.75 (s, **1H Ar**), 7.37-7.74 (m, **14H Ar**), 8.23 (s, **1H Ar**), 11.55 (large s, 1H, N**H**).

### 2-Bromo-N-[4-{[tert-butyl(diphényl)silyl]oxy}-2-(3-cyanobenzoyl)-5-méthoxyphényl]acétamide, 21a

En remplaçant dans l'exemple **21b** le 3-(2-amino-5-{[*tert*-butyl(diphényl)silyl]oxy}-4-méthoxybenzoyl)benzonitrile **20b** par le 3-(2-amino-4-{[*tert*-butyl(diphényl)silyl]oxy}-5-méthoxybenzoyl)benzonitrile, **20a**, on obtient de la même manière le produit titre. Rdt : 90%. RMN ¹H (CDCl₃, 300 MHz) : δ 1.14 (s, 9H, C(C**H₃**)₃), 3.28 (s, 3**H**, OC**H₃**), 3.94 (s, 2H; C**H₂**), 6.74 (s, **1H Ar**), 7.37-7.87 (m, **14H Ar**), 8.23 (s, **1H Ar**), 11.52 (large s, 1H, N**H**).

### 3-(7-Hydroxy-8-méthoxy-2-oxo-2,3-dihydro-1H-1,4-benzodiazépin-5-yl)benzonitrile, 22b

Agiter, à 0°C avec un tube à CaCl₂, le 2-bromo-*N*-[5-{[*tert*-butyl(diphényl)silyl]oxy}-2-(3-cyanobenzoyl)-4-méthoxyphényl]acétamide **21b** (0.5 g, 0.79 mmole) en solution dans NH3 (7N)/MeOH (10 mL) pendant 30 minutes puis à température ambiante 30 minutes. Chauffer à reflux pendant 2 heures, évaporer à sec et purifier par chromatographie sur silice (MeOH/CH₂CL₂ : 1/9). Rdt : 95%. RMN ¹H (DMSO-d₆, 300 MHz) : δ 3.63 (s, 3H, OC**H₃**), 4.12 (large s, 2H; C**H₂**), 6.65 (s, **1H Ar**), 6.72 (s, **1H Ar**), 7.65-7.99 (m, **4H Ar**), 10.14 (large s, 1H, NH), 10.33 (large s, 1H, O**H)**.

### 3-(8-Hydroxy-7-méthoxy-2-oxo-2,3-dihydro-1H-1,4-benzodiazépin-5-yl)benzonitrile, 22a

En remplaçant dans l'exemple **22b** le 2-bromo-*N*-[5-{[*tert*-butyl(diphényl)silyl]oxy}-2-(3-cyanobenzoyl)-4-méthoxyphényl]acétamide **21b** par le 2-bromo-*N*-[4-{[*tert-*butyl(diphényl)silyl]oxyl}-2-(3-cyanobenzoyl)-5-méthoxyphényl]acétamide **21a** on obtient de la même manière le produit titre. Rdt : 30%. RMN ¹H (DMSO-d₆, 200 MHz) : δ 3.64 (s, 3H, OC**H₃**), 4.13 (large s, 2H; C**H₂**), 6.66 (s, **1H Ar**), 6.72 (s, **1H Ar**), 7.61-2.25 (m, **4H Ar**), 10.13 (large s, 1H, N**H**), 10.33 (large s, 1H, O**H**).

### 3-(6-Bromo-7-hydroxy-8-méthoxy-2-oxo-2,3-dihydro-1H-1,4-benzodiazépin-5-yl)benzonitrile, 23b

Chauffer 2 heures à 60°C un mélange de 3-(7-hydroxy-8-méthoxy-2-oxo-2,3-dihydro-1*H*-1,4-benzodiazépin-5-yl)benzonitrile **22b** (150 mg, 0.49 mmole) et de N-bromosuccinimide (90 mg, 051 mmole) dans l'acide acétique. Evaporer à sec et cristalliser dans l'Et₂O. Rdt: 70%. RMN ¹H (DMSO-d₆, 300 MHz) : δ 3.92 (s, 3H, OC**H₃**), 4.20 (system AB, Δδ = 0.6, J_{AB} = 10 Hz, 2H, NC**H₂**), 6.91 (s, **1H Ar**), 7.61-7.93 (m, 4H **Ar** + O**H**), 10.36 (large s, 1H, N**H**).

### 3-(9-Iodo-8-hydroxy-7-méthoxy-2-oxo-2,3-dihydro-1H-1,4-benzodiazépin-5-yl)benzonitrile, 23a

En remplaçant dans l'exemple **23b** le 3-(7-hydroxy-8-méthoxy-2-oxo-2,3-dihydro-1*H-*1,4-benzodiazépin-5-yl)benzonitrile **22b** par le 3-(8-hydroxy-7-méthoxy-2-oxo-2,3-dihydro-1*H*-1,4-benzodiazépin-5-yl)benzonitrile **22a** et le N-bromosuccinimide par le N-iodosuccinimide on obtient de la même manière le produit titre. Rdt: 83%. RMN ¹H (DMSO-d₆, 200 MHz) : δ 3.72 (s, 3H, OC**H₃**), 4.08 (m 2H, NC**H₂**), 6.78 (s, **1H Ar**), 7.61-7.93 (m, **4H Ar**), 9.22 (large s, 1H, O**H**), 10.61 (large s, 1H, N**H**).

### 3-(6-Iodo-7-hydroxy-8-méthoxy-2-oxo-2,3-dihydro-1H-1,4-benzodiazépin-5-yl)benzonitrile, 23c

En remplaçant dans l'exemple **23b** le N-bromosuccinimide par le N-iodosuccinimide on obtient de la même manière le produit titre Rdt: 65%. RMN ¹H (DMSO-d₆, 300 MHz) : δ 3.72 (s, 3H, OC**H₃**), 4.11 (m 2H, NC**H₂**), 6.77 (s, **1H Ar**), 7.65-8.03(m, **4H Ar**), 9.27 (large s, 1H, OH), 11.13 (large s, 1H, N**H**).

### 3-(9-Bromo-8-hydroxy-7-méthoxy-2-oxo-2,3-dihydro-1H-1,4-benzodiazépin-5-yl)benzonitrile, 23d

En remplaçant dans l'exemple **23b** le 3-(7-hydroxy-8-méthoxy-2-oxo-2,3-dihydro-1*H-*1,4-benzodiazépin-5-yl)benzonitrile **22b** par le 3-(8-hydroxy-7-méthoxy-2-oxo-2,3-dihydro-1*H*-1,4-benzodiazépin-5-yl)benzonitrile **22a** on obtient de la même manière le produit titre. Rdt: 86%. RMN ¹H (DMSO-d₆, 200 MHz) : δ 3.92 (s, 3H, OC**H₃**), 4.18 (m 2H, NC**H₂**), 6.84 (s, **1H Ar**), 7.61-7.93 (m, **4H Ar**), 9.22 (large s, 1H, OH), 11.05 (large s, 1H, N**H**).

### 3-(6-Bromo-7,8-diméthoxy-1-méthyl-2-oxo-2,3-dihydro-1H-1,4-benzodiazépin-5-yl)benzonitrile, 24b

Solubiliser le 3-(6-bromo-7-hydroxy-8-méthoxy-2-oxo-2,3-dihydro-1*H*-1,4-benzodiazépin-5-yl)benzonitrile **23b** (120 mg, 0.31 mmole) dans 2 mL de DMF puis ajouter 131 mg de K₂CO₃ (0.95 mmole) et agiter 30 minutes. Ajouter l'iodométhane (44 L, 0.71 mmole) et laisser réagir 6 heures. Ajouter un mélange glace/eau puis filtrer le précipité. Rdt : 70%. RMN ¹H (CDCl₃, 300 MHz) : δ 3.39 (s, 3H, NC**H₃**), 3.88 (s, 3H, OC**H₃**), 4.00 (s, 3H, OC**H₃**),4.30 (system AB, Δδ = 0.9, J_{AB} = 10 Hz, 2H, NC**H₂**), 6.84 (s, **1H Ar**), 7.49-7.51 (t, **1H Ar**), 7.67-7.70 (d, **1H Ar**), 7.74 (s, **1H Ar**), 7.81-7.83 (d, **1H Ar**)**.**

### 3-(9-Iodo-7,8-diméthoxy-1-méthyl-2-oxo-2,3-dihydro-1H-1,4-benzodiazépin-5-yl)benzonitrile, 24a

En remplaçant dans l'exemple **24b** le 3-(6-bromo-7-hydroxy-8-méthoxy-2-oxo-2,3-dihydro-1*H*-1,4-benzodiazépin-5-yl)benzonitrile **23b** par 3-(9-iodoo-8-hydroxy-7-méthoxy-2-oxo-2,3-dihydro-1*H*-1,4-benzodiazépin-5-yl)benzonitrile **23a** on obtient de la même manière le produit titre. Rdt : 91%. RMN ¹H (CDCl₃, 200 MHz) : δ 3.31 (s, 3H, NC**H₃**), 3.77 (s, 3H, OC**H₃**), 3.97 (s, 3H, OC**H₃**),4.31 (system AB, Δδ = 1.0, J_{AB} = 10 Hz, 2H, NC**H₂**), 6.67 (s, **1H Ar**), 7.53-8.03 (m, **4H Ar**).

### 3-(6-Iodo-7,8-diméthoxy-1-méthyl-2-oxo-2,3-dihydro-1H-1,4-benzodiazépin-5-yl)benzonitrile, 24c

En remplaçant dans l'exemple **24b** le 3-(6-bromo-7-hydroxy-8-méthoxy-2-oxo-2,3-dihydro-1*H*-1,4-benzodiazépin-5-yl)benzonitrile **23b** par le 3-(6-iodoo-7-hydroxy-8-méthoxy-2-oxo-2,3-dihydro-1*H*-1,4-benzodiazèpin-5-yl)benzonitrile **23c,** on obtient de la même manière le produit titre. Rdt : 58%. RMN ¹H (CDCl₃, 200 MHz) : δ 3.31 (s, 3H, NC**H₃**), 3.78 (s, 3H, OC**H₃**), 3.96 (s, 3H, OC**H₃**),4.28 (system AB, Δδ = 1.0, J_{AB} = 10 Hz, 2H, NC**H₂**), 6.67 (s, **1H Ar**), 7.53-8.03 (m, **4H Ar**).

### 3-(9-Bromo-7,8-diméthoxy-1-méthyl-2-oxo-2,3-dihydro-1H-1,4-benzodiazépin-5-yl)benzonitrile, 24d

En remplaçant dans l'exemple **24b** le 3-(6-bromo-7-hydroxy-8-méthoxy-2-oxo-2,3-dihydro-1*H*-1,4-benzodiazépin-5-yl)benzonitrile **23b** par le 3-(9-bromo-7-hydroxy-8-méthoxy-2-oxo-2,3-dihydro-1*H*-1,4-benzodiazèpin-5-yl)benzonitrile **23d,** on obtient de la même manière le produit titre. Rdt : 70%. RMN ¹H (CDCl₃, 300 MHz) : δ 3.31 (s, 3H, NC**H₃**), 3.79 (s, 3H, OC**H₃**), 3.99 (s, 3H, OC**H₃**),4.30 (system AB, Δδ = 1.0, J_{AB} = 10 Hz, 2H, NC**H₂**), 6.65 (s, **1H Ar**), 7.57-8.02 (m, 4H **Ar).**

### - Couplages Palladium, sur les benzodiazépinones, de type 25.

### 3-(7,8-Diméthoxy-1-methyl-2-oxo-6-phényl-2,3-dihydro-1H-1,4-benzodiazépin-5-yl)benzonitrile, 25b

Chauffer à 115°C pendant 16 heures, sous atmosphère inerte, un mélange de 67 mg (0.162 mmole) de 3-(6-bromo-7,8-diméthoxy-1-méthyl-2-oxo-2,3-dihydro-1*H*-1,4-benzodiazépin-5-yl)benzonitrile **24b,** 39mg (0.32 mmole) d'acide benzène boronique, 63 mg (0.30 mmole) de K₃PO₄, 19 mg (0.016 mmole) de tétrakis(triphénylphosphine) Pd(0) dans 1 mL de DMF. Laisser revenir à température ambiante puis diluer avec 10 volumes d'eau puis extraire 3X100 mL d'Et₂O. Sécher la phase organique sur Na₂SO₄, filtrer, évaporer à sec. Purifier par chromatographie sur silice (CH₂Cl₂/Et₂O: 1/1). Rdt : 40%. RMN ¹H (CDCl₃, 200 MHz) : δ 3.45 (s, 3H, NC**H₃**), 3.50 (s, 3H, OC**H₃**), 4.03 (s, 3H, OC**H₃**),4.50 (system AB, Δδ = 0.8, J_{AB} = 10 Hz, 2H, NC**H₂**), 6.87-7.51 (m, **7H Ar**), 7.33-7.80 (m, **3H Ar**).

### 3-(7,8-Diméthoxy-1-methyl-2-oxo-9-phényl-2,3-dihydro-1H-1,4-benzodiazépin-5-yl)benzonitrile, 25a

En remplaçant dans l'exemple **25b** le 3-(6-bromo-7,8-diméthoxy-1-méthyl-2-oxo-2,3-dihydro-1*H*-1,4-benzodiazépin-5-yl)benzonitrile **24b** par le 3-(9-iodo-7,8-diméthoxy-1-méthyl-2-oxo-2,3-dihydro-1*H*-1,4-benzodiazépin-5-yl)benzonitrile, **24d** on obtient de la même manière le produit titre. Rdt **:** 51%. RMN ¹H (CDCl₃, 300 MHz) : δ 2.44 (s, 3H, NC**H₃**), 3.63 (s, 3H, OC**H₃**), 3.80 (s, 3H, OC**H₃**), 4.45 (system AB, Δδ = 1.0, J_{AB} = 10 Hz, 2H, NC**H₂**), 6.69 (s, **1H Ar),** 7.41-8.27 (m, **9H Ar**).

### Tert-butyl 3-[5-(cyanophényl)-(7,8-diméthoxy-1-méthyl-2-oxo-2,3-dihydro-1H-1,4-benzodiazépin-9-yl)phényl]prop-2-ynylcarbamate,25c

En remplaçant dans l'exemple **6a,** la 5-(3-bromophényl)-7,8-diméthoxy-1-méthyl-1,3-dihydro-2*H*-1,4-benzodiazépin-2-one **4b,** par le 3-(9-iodo-7,8-diméthoxy-1-méthyl-2-oxo-2,3-dihydro-1*H*-1,4-benzodiazépin-5-yl)benzonitrile, **24a** on obtient de la même manière le produit titre. Rdt: 55%. RMN ¹H (CDCl₃, 200 MHz): δ 1.48 (s, 9H, C(C**H₃)₃**), 3.36 (s, 3H, NC**H₃**), 3.75 (s, 3H, OC**H₃**), 4.03 (s, 3H, OC**H₃**), 4.23-4.26 (d, 2H, NHC**H₂**), 4.32 (system AB, Δδ = 1.0, J_{AB} = 10 Hz, 2H, C**H₂**), 6.61 (s, **1H Ar**), 7.51-7.99 (m, **4H Ar**).

### Methyl (2E)-3-[5(cyanophényl)-7,8-diméthoxy-1-méthyl-2-oxo-2,3-dihydro-1H-1,4-benzodiazépin-9-yl)phényl]acrylate,25d

**Dans un tube scellé sous argon, dissoudre 92 mg (0.2 mmole) de** 3-(9-iodo-7,8-diméthoxy-1-méthyl-2-oxo-2,3-dihydro-1*H*-1,4-benzodiazépin-5-yl)benzonitrile, **24a** dans 0.5 ml de DMF. Ajouter 1 mg (4.4 µmole) d'acétate de palladium, 23 1 (0.25 mmole) d'acrylate de méthyle et 53 µl (0.22 mmole) de tributylamine. Irradier au micro-ondes pendant 10 min à 200 Watts. Ajouter de l'eau, extraire à l'AcOEt, sécher sur Na₂SO₄ et évaporer à sec. Purifier par chromatographie sur silice (CH₂Cl₂/AcOEt : 2/8). Rdt : 27%. RMN ¹H (CDCl₃, 200 MHz) : δ 3.13 (s, 3H, NC**H₃**), 3.78 (s, 3H, OC**H₃**), 3.84 (s, 3H, OC**H₃**), 3.93 (s, 3H, OC**H₃**), 4.37 (system AB, Δδ = 1.0, J_{AB} = 10 Hz, 2H, C**H₂**), 6.65-6.73 (m, **2H Ar**), 7.51-7.99 (m, **5H Ar**).

### Tert-butyl 3-[5-(cyanophényl)-(7,8-diméthoxy-1-méthyl-2-oxo-2,3-dihydro-1H-1,4-benzodiazépin-6-yl)phényl]prop-2-ynylcarbamate,25e

En remplaçant dans l'exemple **6a,** la 5-(3-bromophényl)-7,8-diméthoxy-1-méthyl-1,3-dihydro-2*H*-1,4-benzodiazépin-2-one **4b,** par le 3-(6-iodo-7,8-diméthoxy-1-méthyl-2-oxo-2,3-dihydro-1*H*-1,4-benzodiazépin-5-yl)benzonitrile, **24c** on obtient de la même manière le produit titre. Rdt: 59%. RMN ¹H (CDCl₃, 200 MHz): δ 1.48 (s, 9H, C(C**H₃)₃**), 3.36 (s, 3H, NC**H₃**), 3.75 (s, 3H, OC**H₃**), 4.02 (s, 3H, OC**H₃**), 4.23-4.26 (d, 2H, NH**CH₂**), 4.30 (system, AB, Δδ = 1.0, J_{AB} = 10 Hz, 2H, C**H₂**), 6.61 (s, **1H Ar),** 7.55-8.01 (m, **4H Ar**).

### [9-(3-aminoethynyl)-7,8-diméthoxy-1-méthyl-2-oxo-2,3-dihydro-1H-1,4-benzodiazépin-5-yl]benzonitrile,25f

Agiter 2 heures, sous atmosphère inerte à température ambiante, un mélange de 25 mg (0.05 mmole) de *tert*-butyl 3-[5-(cyanophényl)-(7,8-diméthoxy-1-méthyl-2-oxo-2,3-dihydro-1*H*-1,4-benzodiazépin-9-yl)phényl]prop-2-ynylcarbamate, **25c,** (40 L, 0.52 mmole) d'acide trifluoroacétique et 2 ml de CH₂Cl₂. Evaporer à sec puis cristalliser le produit dans l'AcOEt / Hex. Rdt : 97%. RMN ¹H (DMSO-d₆, 200 MHz) : δ 3.29 (s, 3H, NC**H₃**), 3.74 (s, 3H, OC**H₃**), 3.95 (s, 3H, OC**H₃**), 4.17 (m, 2H, C**H₂**), 4.26 (system AB, Δδ = 0.7, J_{AB} = 11 Hz, 2H, NC**H₂**), 6.90 (s, **1H Ar**), 7.66-8.07 (m, **4H Ar**), 8.41 (large s, 3H, N**H₂**).

### [6-(3-aminoethynyl)-7,8-diméthoxy-1-méthyl-2-oxo-2,3-dihydro-1H-1,4-benzodiazépin-5-yl]benzonitrile,25g

En remplaçant dans l'exemple **25f** *Tert*-butyl 3-[5-(cyanophényl)-(7,8-diméthoxy-1-méthyl-2-oxo-2,3-dihydro-1*H*-1,4-benzodiazépin-9-yl)phényl]prop-2-ynylcarbamate, 25c, par le *Tert*-butyl 3-[5-(cyanophényl)-(7,8-diméthoxy-1-méthyl-2-oxo-2,3-dihydro-1*H*-1,4-benzodiazépin-6-yl)phényl]prop-2-ynylcarbamate,**25e** on obtient de la même manière le produit titre. Rdt : 96%. RMN ¹H (DMSO-d₆, 200 MHz) : δ 3.33 (s, 3H, NC**H₃**), 3.75 (s, 3H, OC**H₃**), 4.01(s, 3H, OC**H₃**), 4.20 (m, 2H, C**H₂**), 4.25 (system AB, Δδ = 0.7, J_{AB} = 11 Hz, 2H, NC**H₂**), 6.63 (s, **1H** Ar), 7.53-7.99 (m, **4H Ar**), 8.52 (large s, 3H, NH₂).

### Synthèses des benzodiazépinones de type 29

### 4-Bromo-3,5-diméthoxyaniline 26a

Dissoudre 3.06g (20 mmoles) de 3,5-diméthoxyaniline dans 50 ml de CH₂Cl₂. Refroidir à -10°C et ajouter par petites spatules, sans que la température ne dépasse -5°C, 8.19g (20 mmoles) de 2,4,4,6-tetrabromocyclohexa-2,5-dienone. Laisser remonter à la température ambiante et agiter pendant 3h. Evaporer à sec et triturer dans l'éther. Filtrer et laver à l'éther. On obtient 3.20g (13.8 mmoles) de produit titre. Rdt : 69%. RMN ¹H (CDCl₃, 300 MHz) : δ 3.74 (s, 2H, N**H₂**), 3.84 (s, 6H, 2 x OC**H₃**), 5.96 (s, **2H Ar**).

### 3-(2-amino-4-méthoxybenzoyl)benzonitrile, 27a

En remplaçant dans l'exemple **2a** la 3,4-diméthoxyaniline par la 3-méthoxyaniline, on obtient de la même manière le produit titre. Rdt : 43%. RMN ¹H (CDCl₃, 300 MHz) : δ 3.82 (s, 3H, OC**H₃**), 6.17 (s, **1H Ar**), 6.46 (s, 2H échangeable N**H₂**), 6.21-6.24 (d, **1H Ar),** 7.54-7.59 (m, **1H Ar**), 7.75-7.95 (m, **5H Ar**).

### 3-(2-amino-5-méthoxybenzoyl)benzonitrile, 27b

En remplaçant dans l'exemple **2a** la 3,4-diméthoxyaniline par la 4-méthoxyaniline, on obtient de la même manière le produit titre. Rdt : 48%. RMN ¹H (CDCl₃, 200 MHz) : δ 3.70 (s, 3H, OC**H₃**), 5.88 (s, 2H échangeable N**H₂**), 6.75-6.84 (m, **2H Ar**), 7.05-7.11 (m, **1H Ar**), 7.59-7.73 (m, **2H Ar**), 7.83-7.99 (m, **2H Ar**).

### 3-(2-amino-6-méthoxybenzoyl)benzonitrile, 27c

Ce produit est obtenu en même temps que la 3-(2-amino-4-méthoxybenzoyl)benzonitrile **27a.** Rdt : 22%. RMN ¹H (CDCl₃, 300 MHz) : δ 3.49 (s, 3H, OC**H₃**), 5.05 (s, 2H échangeable N**H₂**), 6.23-6.25 (d, **1H Ar**), 6.38-6.42 (d, **1H Ar**), 7.20-7.28 (m, **1H Ar**), 7.50-7.55 (m, **2H Ar**), 7.74-7.77 (m, **1H Ar**), 7.90-7.96 (m, **1H Ar**),.

### (2-amino-4-méthoxyphényl)(phényl)méthanone, 27d

En remplaçant dans l'exemple **2a** l'isophtalonitrile par le benzonitrile, et la 3,4-diméthoxyaniline par la 3-méthoxyaniline, on obtient de la même manière le produit titre. Rdt : 68%. RMN ¹H (CDCl₃, 300 MHz) : δ 3.82 (s, 3H, OC**H₃**), 6.15-6.19 (m, **2H Ar**), 6.36 (s, 2H échangeable N**H₂**), 7.38-7.50 (m, **4H Ar),** 7.57-7.62 (m, **2H Ar**).

### (2-amino-6-méthoxyphényl)(phényl)méthanone, 27e

Ce produit est obtenu en même temps que la (2-amino-4-méthoxyphényl)(phényl)méthanone **27d,** Rdt : 16%. RMN ¹H (CDCl₃, 300 MHz) : δ 3.53 (s, 3H, OC**H₃**), 4.61 (s, 2H échangeable N**H₂**), 6.27-6.30 (d, **1H Ar**), 6.37-6.40 (d, **1H Ar),** 7.16-7.22 (t, **1H Ar**), 7.38-7.43 (m, **2H Ar**), 7.49-7.53 (m, **1H Ar**), 7.74-7.77 (m, **2H Ar**).

### (2-amino-3-bromo-4.5-diméthoxyphényl)(phényl)méthanone, 27f

Ajouter gouttes à gouttes et à 0°C à une solution de 900mg (3,5 mmole) de 2-amino-4,5-diméthoxybenzophénone **2c** dans 60 ml de DMSO, 15g d'HBr à 40% en poids dans l'eau. Chauffer à 60°C pendant 24h. Ajouter 400 ml d'H₂O et extraire avec 4 X 200 ml d'AcOEt ; sécher sur MgSO₄, évaporer l'AcOEt et purifier par chromatographie sur silice (AcOEt 1 / Hex 4). Rdt : 65%. ¹H-RMN (CDCl₃, 300MHz) : δ 3,67 (s, 3H, OC**H₃**), 3,97 (s, 3H, OC**H₃**), 6,59 (s, 2H, N**H₂**), 7,06 (s, 1**H Ar**), 7,47-7,65 (m, **5H Ar**). Masse : (M + H)⁺ = 335,98 + 337,98.

### 3-(2-amino-4,6-diméthoxybenzoyl)benzonitrile 27g

En remplaçant dans l'exemple **2a,** la 3,4-diméthoxyaniline par la 3,5-diméthoxyaniline, on obtient de la même manière le produit titre. Rdt : 55%. RMN ¹H (CDCl₃, 300 MHz) : δ 3.39 (s, 3H, OC**H₃**), 3.78 (s, 3H, OC**H₃**), 5.87(s, **1H Ar**), 6.08 (s, **1H Ar**), 6.36 (s, 2H échangeable N**H₂**), 7.61-7.97 (m, **4H Ar**).

### 3-(6-amino-3-bromo-2,4-diméthoxybenzoyl)benzonitrile 27h

En remplaçant dans l'exemple **2a** la 3,4-diméthoxyaniline par la 4-bromo-3,5-diméthoxyaniline **26a,** on obtient de la même manière le produit titre. Rdt : 59%. RMN ¹H (CDCl₃, 200 MHz) : δ 3.49 (s, 3H, OC**H₃**), 3.97 (s, 3H, OC**H₃**), 5.89(s, **1H Ar**), 6.09 (s, 2H échangeable N**H₂**), 7.51-7.96 (m, **4H Ar**).

### 3-(8-méthoxy-2-oxo-2,3-dihydro-1H-1,4-benzodiazépin-5-yl)benzonitrile, 28a

Chauffer à reflux pendant 36 h et sous atmosphère inerte un mélange de 3-(2-amino-4-méthoxybenzoyl)benzonitrile **27a** (4g, 15.9 mmole), de glycinate d'éthyle.HCl (4 g, 28.6 mmol), et 40 ml de pyridine anhydre. Ajouter deux fractions 2 g (14.3 mmol) de glycinate d'éthyle.HCl, toutes les dix heures. Laisser revenir à température ambiante. Evaporer à sec. Verser 200 ml d'eau. Extraire par 3 fois 200 ml de dichlorométhane. Sécher les fractions organiques sur Na₂SO₄ Purifier par chromatographie (AcOEt). Recristalliser dans EtOH/EtO₂. On obtient de produit titre sous forme de cristaux incolores. Rdt : 18%. RMN-¹H CDCl₃, 300 MHz): δ 3.90 (s, 3H, OC**H₃**), 4,35 (s 2H, C**H₂**), 6,65 (m, 1**H Ar**), 6.73-6.77 (m, 1**H Ar**), 7.15-7.18 (d, 1**H Ar**), 7.48-7.54 (t, 1**H Ar**), 7.72-7.75 (m, 1**H Ar**), 7.80-7.84 (m, 1**H Ar**), 7.86 (m, 1**H Ar**), 9,08 (s, 1H échangeable, -N**H**).

### 3-(6-méthoxy-2-oxo-2,3-dihydro-1H-1,4-benzodiazépin-5-yl)benzonitrile, 28b

En remplaçant dans l'exemple **28a** le 3-(2-amino-4-méthoxybenzoyl)benzonitrile **27a** par le 3-(2-amino-6-méthoxybenzoyl)benzonitrile **27c,** on obtient de la même manière le produit titre. Rdt : 12%. RMN ¹H (CDCl₃, 300 MHz) : δ 3.53 (s, 3H, OC**H₃**), 4.37 (system AB, Δδ = 0.87, J_{AB} = 11 Hz, 2H, C**H₂**), 6.73-6.80 (t, **2H Ar**), 7.42-7.53 (m, **2H Ar**), 7.64-7.68 (m, **1H Ar**), 7.72-7.75 (m, **2H Ar**), 8.42 (s, 1H échangeable, -**NH**).

### 3-(7-méthozy-2-oxo-2,3-dihydro-1H-1,4-benzodiazépin-5-yl)benzonitrile, 28c

En remplaçant dans l'exemple **28a** le 3-(2-amino-4-méthoxybenzoyl)benzonitrile **27a** par le 3-(2-amino-5-méthoxybenzoyl)benzonitrile **27b,** on obtient de la même manière le produit titre. Rdt : 35%. RMN ¹H (CDCl₃, 300 MHz) : δ 3.76 (s, 3H, OC**H₃**), 4.35 (s, 2H, C**H₂**), 6.70 (s, **1H Ar**), 7.12-7.13 (m, **1H** Ar), 7.50-7.55 (t, **1H Ar**), 7.73-7.76 (m, **1H Ar**), 7.84-7.87 (m, **1H Ar**), 7.92 (s, **1H** Ar), 8.51 (s, 1H échangeable, -**NH**).

### 6-méthoxy-5-phényl-1,3-dihydro-2H-1,4-benzodiazépin-2-one, 28d

En remplaçant dans l'exemple **28a** le 3-(2-amino-4-méthoxybenzoyl)benzonitrile **27a** par le (2-amino-6-méthoxyphényl)(phényl)méthanone **27e,** on obtient de la même manière le produit titre. Rdt : 68%. RMN ¹H (CDCl₃, 200 MHz) : δ 3.48 (s, 3H, OC**H₃**), 4.34 (system AB, Δδ = 0.85, J_{AB} = 11 Hz, 2H, C**H₂**), 6.69-6.80 (m, **2H Ar**), 7.26-7.48 **(m, 6H Ar**), 8.76 (s, 1H échangeable, -N**H**).

### 7-méthoxy-5-phényl-1,3-dihydro-2H-1,4-benzodiazépin-2-one, 28e

En remplaçant dans l'exemple **28a** le 3-(2-amino-4-méthoxybenzoyl)benzonitrile **27a** par le (2-amino-4-méthoxyphényl)(phényl)méthanone **27d,** on obtient de la même manière le produit titre. Rdt : 32%. RMN ¹H (CDCl₃, 200 MHz) : δ 3.72 (s, 3H, OC**H₃**), 4.32 (s, 2H, C**H₂**), 6.78 (s, **1H Ar**), 7.08 (m, **2H Ar**), 7.33-7.48 (m, **3H Ar**), 7.55-7.60 (m, **2H Ar**), 8.86 (s, 1H échangeable, -N**H**).

### 9-bromo-7,8-diméthoxy-5-phényl-1,3-dihydro-2H-1,4-benzodiazépin-2-one, 28f

En remplaçant dans l'exemple **28a** le 3-(2-amino-4-méthoxybenzoyl)benzonitrile **27a,** par la (2-amino-3-bromo-4.5-diméthoxyphényl)(phényl)méthanone **27f,** et le glycinate d'éthyl par le glycinate de méthyl on obtient de la même manière le produit titre. Rdt : 40%. ¹H-RMN (CDCl₃, 300MHz) : δ 3,74 (s, 3H, OC**H₃**), 3,98 (s, 3H, OC**H₃**), 4,32 (m, 2H, C**H₂**), 6,78 (s, 1**H Ar**), 7,41-7,67 (m, 6H, 1N**H** + **5H Ar**). Masse : (M+H)⁺ = 375,05 + 377,03.

### 3-(6,8-Dimethoxy-2-oxo-2,3-dihydro-1H-1,4-benzodiazepin-5-yl)benzonitrile, 28g

En remplaçant dans l'exemple **3a** le 3-(2-amino-4,5-diméthoxybenzoyl)benzonitrile **2a** par le 3-(2-amino-4,6-diméthoxybenzoyl)benzonitrile **27g,** on obtient de la même manière le produit titre. Rdt : 42%. ¹H-RMN (DMSO, 300MHz) : δ 3.46 (s, 3H, OC**H₃**), 3.86 (s, 3H, OC**H₃**), 4.18 (system AB, Δδ = 0.6, J_{AB} = 10 Hz, 2H, NC**H₂**), 6.45 (s, 1H, **1H Ar**), 6.49 (s, **1H Ar**), 7.58-7.89 (m, **4H Ar**), 10.44 (s, 1H, N**H**).

### 3-(7-Bromo-6,8-dimethoxy-2-oxo-2,3-dihydro-1H-1,4-benzodiazepin-5-yl)benzonitrile, 28h

En remplaçant dans l'exemple **3a** le 3-(2-amino-4,5-diméthoxybenzoyl)benzonitrile **2a** par le 3-(6-amino-3-bromo-2,4-diméthoxybenzoyl)benzonitrile **27h,** on obtient de la même manière le produit titre. Rdt : 24%. ¹H-RMN (DMSO, 200MHz) : δ 3.32 (s,2H, NC**H₂**), 3.61 (s, 3H, OC**H₃**), 3.98 (s, 3H, OC**H₃**), 6.41 (s, 1H, **1H Ar**), 7.49-8.16 (m, **4H** Ar), 9.80 (s, 1H, **NH**).

### 3-(8-méthoxy-1-méthyl-2-oxo-2,3-dihydro-1H-1,4-benzodiazépin-5-yl)benzonitrile, 29a

En remplaçant dans l'exemple **4k** le 3-(7,8-diméthoxy-2-oxo-2,3-dihydro-1H-1,4-benzodiazépin-5-yl)-benzonitrile **3a** par le 3-(8-méthoxy-2-oxo-2,3-dihydro-1H-1,4-benzodiazépin-5-yl)benzonitrile **28a,** on obtient de la même manière le produit titre. Rdt : 57%. RMN ¹H (CDCl₃, 200 MHz) : δ 3.40 (s, 3H, NC**H₃**), 3.91 (s, 3H, OC**H₃**), 4.30 (system AB, Δδ = 1.00 J_{AB} = 16 Hz, 2H, C**H₂**), 6.74-6.84 (m, **2H Ar**), 7.13-7.18 (d, **1H Ar**), 7.48-7.53 (t, **1H Ar**), 7.70-7.74 (d, **1H Ar**), 7.88 (m, **2H Ar**).

### 3-(6,8-Dimethoxy-1-methyl-2-oxo-2,3-dihydro-1H-1,4-benzodiazepin-5-yl)benzonitrile, 29b

En remplaçant dans l'exemple **4a** le 3-(7,8-diméthoxy-2-oxo-2,3-dihydro-1*H*-1,4-benzodiazépin-5-yl)-benzonitrile **3a** par la 3-(6,8-dimethoxy-2-oxo-2,3-dihydro-1*H*-1,4-benzodiazepin-5-yl)benzonitrile, **28g,** on obtient de la même manière le produit titre. Rdt : 77%. RMN ¹H (CDCl₃, 300 MHz) : δ 3.38 (s, 3H, NC**H₃**), 3.53 (s, 3H, OC**H₃**), 3.93 (s, 3H, OC**H₃**), 4.35 (system AB, Δδ = 1.00 J_{AB} = 15 Hz, 2H, C**H₂**), 6.34 (s, 1**H Ar**), 6.47 (s, **1H Ar**), 7.44-7.86 (m, **1H Ar**).

### 3-(7-Bromo-6,8-dimethoxy-1-methyl-2-oxo-2,3-dihydro-1H-1,4-benzodiazepin-5-yl)benzonitrile, 29c

En remplaçant dans l'exemple **4a** le 3-(7,8-diméthoxy-2-oxo-2,3-dihydro-1*H*-1,4-benzodiazépin-5-yl)-benzonitrile **3a** par la 3-(7-bromo-6,8-dimethoxy-2-oxo-2,3-dihydro-1*H*-1,4-benzodiazepin-5-yl)benzonitrile, **28h,** on obtient de la même manière le produit titre. Rdt : 20%. RMN ¹H (CDCl₃, 300 MHz) : δ 3.29 (s, 3H, NC**H₃**), 3.49 (s, 3H, OC**H₃**), 3.94 (s, 3H, OC**H₃**), 4.37 (s large, 2H, C**H₂**), 6.25 (s, **1H Ar**),.7.44-7.75 (m, **4H** Ar).

### Voie d'accès aux phényles méta carboxamides substitués de type 36.

### Acide 3-(2-amino-4-hydroxy-5-méthoxybenzoyl)benzoïque, 32a

Chauffer à 140°C pendant la nuit un mélange de 1,5g (2-amino 3,4-diméthoxybenzoyl)benzonitrile **2a** (5,31 mmoles), 3,13g de KOH (55,8 mmoles) dans25 ml d'éthylène glycol. Ajouter 150 ml d'H₂O glace et ajouter HCl 0,1 N jusqu'à obtenir un PH de 3-4 ; extraire avec 4 X 150 ml d'AcOEt ; sécher sur MgSO₄, évaporer l'AcOEt et purifier par chromatographie sur silice (AcOEt). Rdt : 70%. ¹H-RMN (CDCl₃, 300MHz) : δ 3,68 (s, 3H, OC**H₃**), 6,31 (s, 1**H Ar**), 6,82 (s, 1**H Ar**), 7,57-8,34 (m, **4H Ar**). Masse : (M + H)⁺ = 288,07.

### Acide 3-(7-méthoxy-8-hydroxy-2-oxo-2,3-dihydro-1H-1,4-benzodiazépin-5-yl) benzoïque, 33a

Chauffer à reflux pendant 36 h et sous atmosphère inerte un mélange de 300mg (1 mmole) d'acide 3-(2-amino-4-hydroxy-5-méthoxybenzoyl)benzoique **32a,** 150mg (2 mmoles) de glycinate d'éthyle.HCl, et 5 ml de pyridine anhydre. Ajouter quatres fractions de 100mg (0,79 mmole) de glycinate d'éthyle.HCl, toutes les six heures. Laisser revenir à température ambiante. Evaporer à sec. Verser 100 ml d'eau glace, filter puis rincer à l'H2O froide, à l'EtOH puis à l'Et₂O, sécher ; Rdt : 45%. L e produit est utilisé sans autre purification dans la réaction suivante

### Benzoate de 3-(7,8-diméthoxy-1-méthyl-2-oxo-2,3-dihydro-1H-1,4-benzodiazépin-5-yl)méthyl, 34a

Ajouter, à 0°C et sous atmosphère inerte, à une solution de 1 g (3,2 mmoles) d'acide 3-(7-méthoxy-8-hydroxy-2-oxo-2,3-dihydro-1H-1,4-benzodiazépin-5-yl) benzoïque **33a** dans 10 ml de DMF, 390mg (9,6 mmoles) de NaH à 60% dans l'huile. Laisser sous agitation à température ambiante pendant 1h. Ajouter goutte à goutte à 0°C, 600 µl d'iodométhane. Laisser sous agitation à température ambiante pendant la nuit. Ajouter 200 ml d'H₂O et extraire avec 3 X 200 ml d'AcOEt ; sécher sur MgSO₄, évaporer l'AcOEt et purifier par chromatographie sur silice (AcOEt puis AcOEt 5 / CH₂Cl₂ 4 / EtOH 1). Rdt : 50%.. ¹H-RMN (CDCl₃, 300MHz) : δ 3,42 (s, 3H, NC**H₃**), 3;74 (s, 3H, OC**H₃**),3,93 (s, 3H, COOC**H₃**), 4,00 (s, 3H, OC**H₃**), 4,33 (système AB, Δδ = 1,01, J_{AB} = 8,3, 2H, C**H₂**), 6,65 (s, 1**H Ar**), 6,81 (s, 1**H Ar**), 7,49-8,27 (m, 4**H Ar**). Masse : (M + H)⁺ = 369,1.

### Acide 3-(7,8-diméthoxy-1-méthyl-2-oxo-2,3-dihydro-1H-1,4-benzodiazépin-5-yl) benzoïque, 35a

Ajouter à 0°C à une solution de 1,8g de benzoate de 3-(7,8-diméthoxy-1-méthyl-2-oxo-2,3-dihydro-1H-1,4-benzodiazépin-5-yl)méthyl **34a** (4,8 mmoles) dans 75ml de MeOH et 25 ml d'H₂O, 300mg de pastille de KOH. Chauffer la solution à 60°C pendant 1h. Evaporer le MeOH, ajouter 100ml d'H₂O glace puis acidifier la solution à PH = 2-3 en additionnant HCl 1N goutte à goutte. Extraire avec 4 X 150 ml d'AcOEt, sécher sur MgSO₄, évaporer l'AcOEt. Rdt : 50%.. ¹H-RMN (CDCl₃, 300MHz) : δ 3,44 (s, 3H, NC**H₃**), 3;75 (s, 3H, OC**H₃**), 4,00 (s, 3H, OC**H₃**), 4,37 (système AB, Δδ = 1,07, J_{AB} = 10,9, 2H, C**H₂**), 6,66 (s, 1**H** Ar), 6,81 (s, 1**H Ar**), 7,50-8,39 (m, 4**H Ar**). Masse : (M + H)⁺ = 369,1.

### 3-(7,8-diméthoxy-1-méthyl-2-oxo-2,3-dihydro-1H-1,4-benzodiazépin-5-yl)N-isopropylbenzamide, 36a

Ajouter à une solution de 100mg d'acide 3-(7,8-diméthoxy-1-méthyl-1,3-dihydro-2H-1,4-benzodiazépin-5-yl)benzoïque **35a** (0,28 mmole) et de 17mg d'isopropylamine (0,29 mmole) dans 4 ml de DMF, 117mg de N-methylmorpholine (1,17 mmoles) suivi de 192mg de BOP (0,43 mmole). Laisser sous agitation à température ambiante pendant une nuit. Ajouter 100 ml d'H₂O et extraire avec 3 X 100 ml de CH₂Cl₂; sécher sur MgSO₄, évaporer le CH₂Cl₂ et purifier par chromatographie sur silice (AcOEt 3 / Hex 1 puis AcOEt). Rdt : 75%. ¹H-RMN (DMSO, 300MHz) : δ 1,27 (d, J₁₂ = 6,5, 6H, CH(**CH₃)₂**), 3,40 (s, 3H, NC**H₃**), 3,72 (s, 3H, OC**H₃**), 3,98 (s, 3H, OC**H₃**), 4,10 (m, J₂₁ = 6,5, J₂₃ = 7,2, 1H, C**H**), 4,32 (système AB, Δδ = 0,99, J_{AB} = 10,3, 2H, C**H₂**), 6,20 (d, J₃₂ =7,2, 1**H échangeable,** *i*prN**H**), 6,63 (s, 1**H Ar**), 6,78 (s, 1**H Ar**), 7,42-8,08 (m, **4H Ar**). Masse : (M+H)⁺ = 396,16.

### N-benzyl-3-(7,8-diméthoxy-1-méthyl-2-oxo-2,3-dihydro-1H-1,4-benzodiazépin-5-yl) benzamide, 36b

En remplaçant dans l'exemple **36a** l'isopropylamine par la benzylamine, on obtient de la même manière le produit titre. Rdt : 80%. ¹H-RMN (CDCl₃, 300MHz) : δ 3,35 (s, 3H, NC**H₃**), 3,69 (s, 3H, OC**H₃**), 3,95 (s, 3H, OC**H₃**), 4,23 (système AB, Δδ = 0,97, J_{AB} = 10,3, 2H, C**H₂**), 4,60 (m, 2H, PhC**H₂**), 6,60 (s, 1**H Ar**), 6,76 (s, 1**H Ar**), 7,15 (m, 1**H échangeable,** BnN**H**), 7,27-8,12 (m, 9**H Ar**). Masse : (M+H)⁺ = 444,20.

### N-(6-amino-6-oxohexyl)-3-(7,8-diméthoxy-1-méthyl-2-oxo-2,3-dihydro-1H-1,4-benzodiazépin-5-yl) benzamide, 36c

Ajouter à une solution de 100mg d'acide 3-(7,8-diméthoxy-1-méthyl-1,3-dihydro-2H-1,4-benzodiazépin-5-yl)benzoïque **35a** (0,28 mmole) et de 48mg de chlorhydrate de 5-aminopentylcarboxamide (0,29 mmole) dans 4 ml de DMF, 30mg de triéthylamine, 117mg de N-methylmorpholine (1,17 inmoles) suivi de 192mg de BOP (0,43 mmole). Laisser sous agitation à température ambiante pendant une nuit. Ajouter 100 ml d'H₂O et extraire avec 3 X 100 ml de CH₂Cl₂; sécher sur MgSO₄, évaporer le CH₂Cl₂ et purifier par chromatographie sur silice (AcOEt 5 / CH₂Cl₂ 4 / EtOH 1). Rdt : 75%. ¹H-RMN (CDCl₃, 200MHz) : δ 1,66-1,73 (m, 6H, (C**H₂**)₃), 2,28 (m, 2H, COC**H₂**), 3,4463,51 (m, 5H, NHC**H₂** + NC**H₃**), 3,77 (s, 3H, OC**H₃**), 4,02 (s, 3H, OC**H₃**), 4,33 (système AB, Δδ = 0,97, J_{AB} = 10,5, 2H, C**H₂**), 5,70 (slarge, 2**Héchangeable,** CON**H₂**), 6,69 (m, 2H, CON**H** + 1**H Ar**), 6,82 (s, 1**H Ar**), 7,45-8,20 (m, 4**H Ar**). Masse : (M+H)⁺ = 467,2.

### 3-(7,8-diméthoxy-1-méthyl-2-oxo-2,3-dihydro-1H-1,4,benzodiazépin,5-yl) N, N-diméthylbenzamide 36d

En remplaçant dans l'exemple **36a** l'isopropylamine par la diméthylamine, on obtient de la même manière le produit titre. Rdt : 90%. ¹H-RMN (CDCl₃, 300MHz) : δ 3,01 (s, 3H, N(C**H₃**)₂), 3,13 (s, 3H, N(C**H₃**)₂), 3,43 (s, 3H, NC**H₃**), 3,77-3,85 (m, 4H, 1HC**H₂** + OC**H₃**), 4,01 (s, 3H, OC**H₃**), 4,82 (m, 1HC**H₂**), 6,70 (s, 1**H Ar**), 6,81 (s, 1**H Ar**), 7,46-7,77 (m, 4**H Ar**). Masse : (M+H)⁺ = 382,20.

### 5-{3-[(4-benzylpypérazin-1-yl)carbonyl]phényl}7,8-diméthoxy-1-méthyl-2-oxo-2,3-dihydro-1H-1,4-benzodiazépin-2-one, 36e

En remplaçant dans l'exemple **36a,** l'isopropylamine par la N-benzylpypérazine, on obtient de la même manière le produit titre. Rdt : 70%. ¹H-RMN (CDCl₃, 300MHz) : δ 2,43-2,71 (m, 4H, 2C**H₂pyp**), 3,43-3,57 (m, 9H, PhC**H₂** + 2C**H₂pyp** + NC**H₃**), 3,77-3,85 (m, 4H, 1HC**H₂** + OC**H₃**), 4,01 (s, 3H, OC**H₃**), 4,83 (m, 1NC**H₂**), 6,70 (s, 1**H Ar**), 6,81 (s, 1**H Ar**), 7,30-7,77 (m, 9**H Ar**). Masse : (M+H)⁺ = 513,20.

### 3-(7,8-diméthoxy-1-méthyl-2-oxo-2,3-dihydro-1H-1,4-benzodiazépin-5-yl) N-(3-phénylpropyl)benzamide, 36f

En remplaçant dans l'exemple **36a** l'isopropylamine par la 3-phénylpropylamine, on obtient de la même manière le produit titre. Rdt : 95%. ¹H-RMN (CDCl₃, 300MHz) : δ 1,97-2,04 (m, 2H, PhC**H₂**), 2,72-2,80 (m, 2H, C**H₂**), 3,44-3,53 (m, 5H, NHC**H₂** + NC**H₃**), 3,76-3,86 (m, 4H, 1NC**H₂** + OC**H₃**), 4,01 (s, 3H, OC**H₃**), 4,83 (m, 1NC**H₂**), 6,28 (slarge, 1**Héchangeable**, CONH), 6,66 (s, 1**H Ar**), 6,82 (s, 1**H Ar**), 7,26-8,06 (m, 4**H Ar**). Masse : (M+H)⁺ = 472,20.

### - Synthèses des boroniques non commerciaux 37

### 2-hydroxy-5-iodobenzonitrile, 37a

Ajouter à une solution de 2g de 2-hydroxybenzonitrile (16,8 mmoles) dans 50 ml d'acétonitrile, sous atmosphère inerte et à -20°C, 1,65 ml d'acide trifluorométhane sulfonique suivi de 4,5g de N-iodosuccinimide (20,2 mmoles) par petite portion. Laisser sous agitation à température ambiante pendant 12h. Ajouter 200 ml d'H₂O et extraire avec 3 X 150 ml de CH₂Cl₂; sécher sur MgSO₄, évaporer le CH₂Cl₂ et purifier par chromatographie sur silice (AcOEt 1 / Hex 4). Rdt : 85%. ¹H-RMN (CDCl₃, 200MHz) : δ 6,78 (m, 1**H Ar**), 7,70-7,79 (m, 2**H Ar**), 8,17 (m, 1H échangeable, O**H**).

### 5-iodo-2-[(4-méthoxybenzyl)oxy]benzonitrile, 37b

Laisser sous agitation à température ambiante et sous atmosphère inerte un mélange de 2g de 2-hydroxy-5-iodobenzonitrile **37a** (8,16 mmoles), 4,5g de K₂CO₃ (32,64 mmoles), 295mg d'iodure de tetra *n*butyl ammonium (0,8 mmole), 1,4g de chlorure de 4-méthoxybenzyl (8,98 mmoles) dans 75 ml d'acétone anhydre pendant 12h. Evaporer l'acétone, Ajouter 150 ml d'H₂O et extraire avec 3 X 150 ml de CH₂Cl₂; sécher sur MgSO₄, évaporer le CH₂Cl₂. Triturer avec 20 ml d'AcOEt, filtrer, rincer avec un minimum d'AcOEt, sécher. Rdt : 75%. ¹H-RMN (CDCl₃, 300MHz) : δ 3,81 (s, 3H, OC**H₃**), 5,13 (s, 2H, C**H₂**Ph), 6,79 (m, 1**H Ar**), 6,92 (m, 2**HBn**), 7,35 (m, 2**HBn**), 7,72-7,82 (m, 2**H Ar**).

### Acide-3-cyano-4-[(4-méthoxybenzyl)oxy]phénylboronique, 37c

Ajouter par cannulation à -78°C et sous atmosphère inerte à une solution de 300mg de 5-iodo-2-[(4-méthoxybenzyl)oxy]benzonitrile **37b** (0,82 mmole) dans 10 ml de THF anhydre, 1 ml de *ter*BuLi 1,7M dans le pentane refroidi à -78°C . Laisser sous agitation 30min puis ajouter par cannulation une solution de 930 µl de triméthylborate dans 10 ml de THF anhydre refroidi à -78°C . Laisser revenir la solution à température ambiante pendant la nuit. Ajouter 50 ml d'H₂O glace et extraire avec 4 X 50 ml d'AcOEt ; sécher sur MgSO₄, évaporer l'AcOEt et purifier par chromatographie flash sur silice (AcOEt puis AcOEt 5 / CH₂Cl₂ 4 / EtOH 1). Rdt : 65%.

### EXEMPLE 2 : ACTIVITE PHARMACOLOGIQUE: INHIBITION DES PHOSPHODIESTERASES.

### 2.1. Isolement des phosphodiestérases du muscle lisse

Un segment de 3 g de média d'aorte bovine fragmenté à l'aide de ciseaux a été homogénéisé à l'aide d'un ultra-turrax puis d'un potter verre-verre dans 7 volumes/poids de tampon A contenant un cocktail d'inhibiteurs de protéases (20 mM Tris-HCl, 0,25 M saccharose, 2mM acétate de Mg, 1mM dithiothreitol, 5mM EGTA, 2000 U/ml aprotinine, 10 mg/l leupeptine et 10 mg/l d'inhibiteur trypsique de soja). L'homogénat a été centrifugé pendant 1h à 105000 g. Le surnageant a été déposé sur une colonne de DEAE-Sephacel (15 X 1,6 cm), pré-équilibrée avec le tampon B (tampon A dépourvu de saccharose, d'EGTA et d'inhibiteurs de protéases). La colonne a été lavée jusqu'à ce qu'aucune absorption ne puisse être détectée à 280 nm, puis éluée avec un gradient linéaire en NaCl (0-0,5M) dans le tampon B. Des fractions de 3ml ont été recueillies et les activités enzymatiques ont été déterminées suivant les conditions décrites ci-dessous pour localiser les différentes PDE1, PDE3, PDE4 et PDE5 qui ont été aliquotées et congélées à -80°C (Lugnier et col., Biochem. Phamacol., 35 (1986) 1746-1751). La PDE2 a été préparée selon les mêmes techniques à partir des cellules endothéliales bovines (Lugnier et Schini, Biochem. Pharmacol. 1990, 39 ; 75-84).

### 2.2. Protocole de mesure des Activités phosphodiestérasiques

L'activité de la phosphodiestérase des nucléotides cycliques a été déterminée à l'aide d'une méthode radioenzymatique en utilisant de l'AMP ou du GMP cyclique tritié (1µM) comme substrat (Lugnier et col., 1986). L'adénosine ou le guanosine monophosphate tritié formé par hydrolyse du nucléotide cyclique marqué a été, dans une seconde incubation avec une nucléotidase en excès, transformé en adénosine ou guanosine tritié. Le nucléoside formé a été séparé des nucléotides par chromatographie sur une résine échangeuse d'anions. La radioactivité du nucléoside a été déterminée par scintillation liquide. Les incubations enzymatiques ont été effectuées dans les conditions où il n'y a pas plus de 15% d'hydrolyse du substrat, chaque point étant en fait en double.

### 2.2.1. Détermination de l'inhibition de la PDE2.

La concentration en substance nécessaire pour inhiber 50% l'activité enzymatique (CI₅₀), et ceci en présence de 1µM d'AMP cyclique, a été déterminée par régression non linéaire à partir des valeurs expérimentales de vitesse d'hydrolyse (Prism, GraphPad).

### 2.2.2. Sélectivité

Une évaluation de l'activité des composés a été effectuée sur d'autres isoformes de phosphodiestérases, notamment la PDE1 du muscle lisse vasculaire à l'état basal ou activé par la calmoduline, la PDE3, la PDE4 et la PDE5 du muscle lisse vasculaire.

Les résultats obtenus sont présentés dans les Tableaux 1 et 2 ci-après, où les % représentent le % d'inhibition de l'activité enzymatique produit par 10 µmoles de la molécule testée.

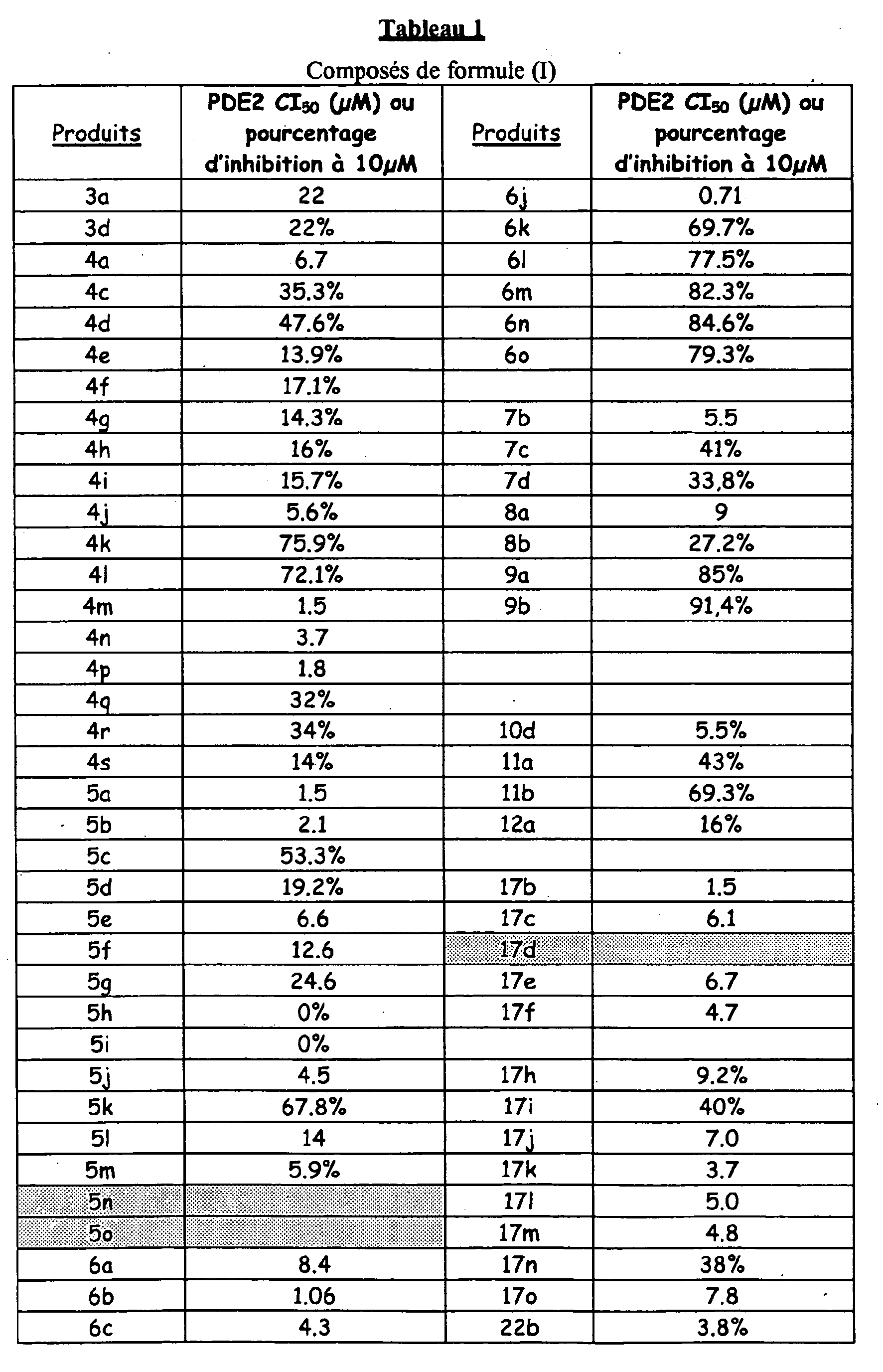

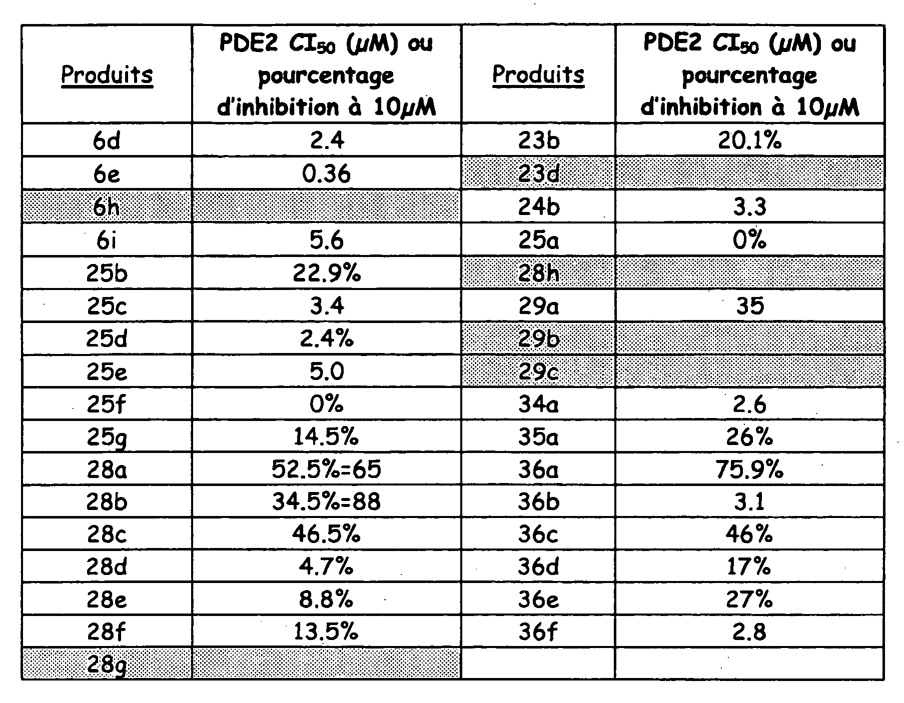

**Tableau 2**

| Sélectivité | | | | | |
|---|---|---|---|---|---|
| **produits** | **CI₅₀ (µM) ou pourcentage D'inhibition à 10µM** | | | | |
| | **PDE1** | **PDE2** | **PDE3** | **PDE4** | **PDE5** |
| 4m | | 1.5 | | | |
| 4p | 25% | 1.8 | 58% | 19% | 26% |
| 5a | 13.2% | 1.5 | 5% | 16.2% | 17.6% |
| 5b | | | 2.1 | | 21.6% |
| 6b | | 1.06 | | | |
| 6d | | 2.4 | | 55.7% | |
| 6e | | 0.36 | | | |
| 6j | | 0.71 | 5 | 2.8 | |
| 6m | | 82.3% | | 37.3% | |
| 6n | | 84.6% | | 58.7% | |
| 7a | | 3.13 | | 6.52 | |
| 9b | | 91.4% | | | |
| 17b | 10% | 1.5 | 36% | 8% | 14% |

L'ensemble des composés testés montre une forte activité inhibitrice de PDE2. Les molécules préférées selon l'invention présentent un excellent profil de puissance et de sélectivité vis-à-vis de la phosphodiestérase 2, dans la mesure où ces composés inhibent de manière plus faible les autres PDE, notamment la PDE3.

### EXEMPLE 3 : TESTS COMPORTEMENTAUX

Le composé 5a a été testé dans différents tests comportementaux.

### 3.1 Test du labyrinthe en croix surélevé (elevated plus maze)

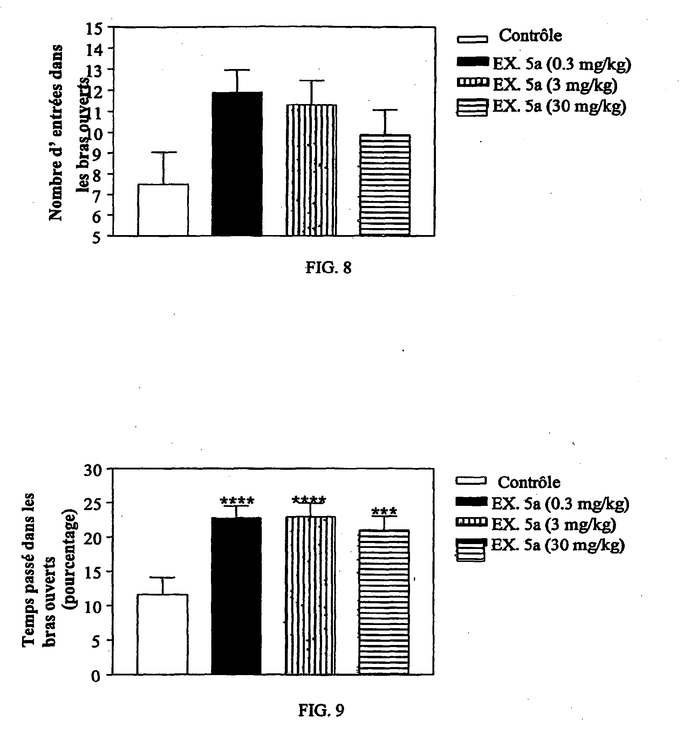

Ce test a été validé chez le rat par Pellow en 1986 et chez la souris en 1987 par Lister. Ce test est basé sur la peur de l'espace, ainsi les bras ouverts à l'espace soumises aux animaux représentent un stimulus d'anxiété ; au contraire les bras fermés représentent la sécurité. En enregistrant la fréquence de chaque bras, ce test permet d'évaluer l'effet anxiolytique d'une molécule en comparaison avec un composé de référence tel que la buspirone.
Les sujets sont des souris Balb/c ou Swiss de 10-11 semaines. De manière aléatoire, différents groupes de souris sont constitués, un groupe contrôle (traité avec le véhicule) et d'autres groupes traités par des composés.
L'appareil est un labyrinthe en PVC recouvert par un Plexiglas et divisé en quatre bras égaux exploratoires (45 X 10 cm), qui sont tous interconnectés par une petite plateforme (10 X 10 cm). L'appareil est placé à 66cm au dessus du sol. Les deux bras sont ouverts et les deux autres sont fermés avec une paroi (hauteur : 30 cm).
Après l'administration, la souris est placée sur la plateforme opposée au bras fermé. Le nombre d'entrées et le temps passé dans chaque bras ouvert sont enrégistrés pendant une période de 8 minutes.
Le traitement est administré 1 heure avant le test. Les composés sont adminitsrés per os à différentes doses. Les résultats sont donnés aux figures 8 et 9. N=10 ; *** p< 0,005 et **** p<0,001 (versus contrôle ; test de Dunnett).
Ainsi, une différence significative entre les groupes a été observée, notamment pour le pourcentage de temps passé dans les bras ouverts. Les souris traitées avec le composé de l'exemple 5a ont passé plus de temps dans les bras ouverts et ceci à toutes les doses testées.

### 3.2 Test de Porsolt (ou test de la nage forcée, swim test en anglais)

Ce test de la nage forcée est basé sur l'induction d'un comportement alternatif chez les rongeurs soumis à un stress aigü. Dans ce modèle, le rat ou la souris placé dans un cylindre rempli d'eau adopte un état particulier d'immobilité. Le début de cette immobilité est retardé par des médicaments anti-dépresseurs, administrés de façon aigue et répétée.

Les rats Wistar ou les souris Swiss sont utilisés. Les animaux après avoir été isolés pendant une semaine avec un cycle clair/obscure inversé, sont placés dans les cylindres remplis d'eau pendant 6 minutes.
La période totale d'immobilité est enrégistrée pendant les dernières 4 minutes.
Le traitement est administré 20 minutes avant le test. Pour 3 dosés, 4 groupes d'animaux sont nécessaires : un groupe contrôle traité avec le véhicule etles autres pour les différentes doses.

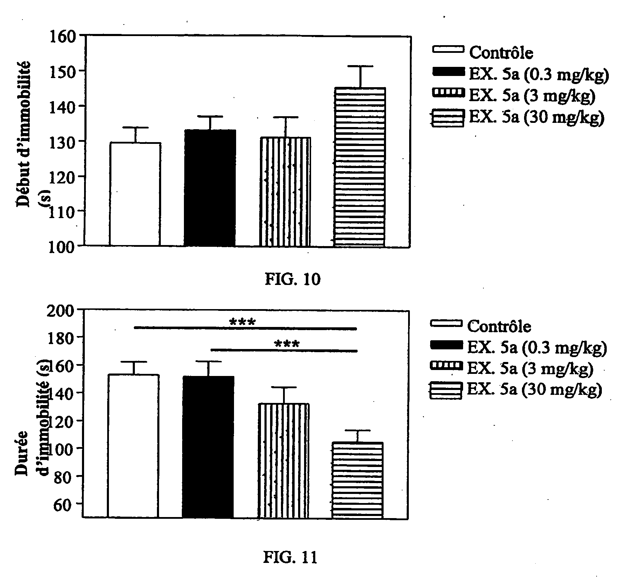

Les résultats sont donnés aux figures 10 et 11. Deux paramètres ont été enrégistrés : le début de l'immobilité et la durée de l'immobilité. N= 10 ; *** p<0,005 (test de Dunnett).

Les analyses statistiques ont révélé une différence significative entre les groupes pour la durée totale d'immobilité (p = 0,007). Les souris traitées avec le composé de l'exemple 5a à 3 et à 30 mg/kg ont montré une durée plus faible d'immobilité que les groupes contrôle et de l'ex. 5a à 0,3 mg/kg.

### 3.3 Test clair/obscure (light/dark test en anglais)

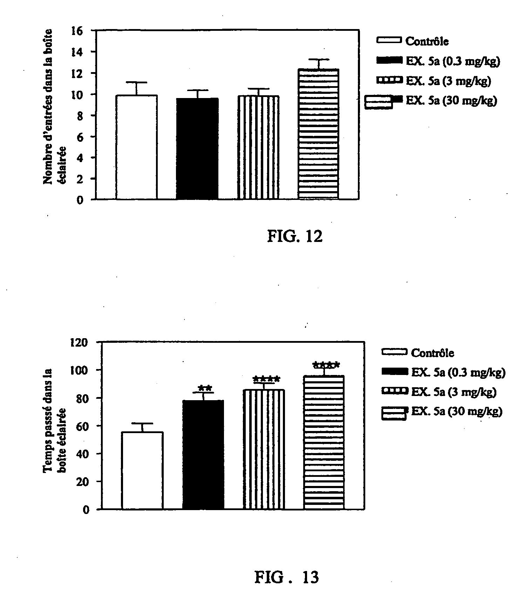

Ce test de choix du clair/obscure est basé sur la tendance naturelle des rongeurs à préférer un environnement obscure et permet ainsi d'évaluer les réponses émotionnelles des animaux dans une situation de lumière intense. Cette procédure est adéquate pour révéler l'état d'anxiété qui est évoqué par des stiuli d'anxiété (équipe de Lister en 1990). Les souris confrontées à cet appareil, même si elles montrent une préférence pour le compartiment obscure, montrent également quelques incursions dans celui éclairé. Cette procédure a été validée par Misslin et al. en 1990 qui ont démontré que les propriétés anxiolytiques et anxiogènes de différentes substances agissent sur le système serotoninergqiue ou le complexe récepteur GABA-benzodiazépines.
Des souris Balb/c ou Swiss de 10-11 semaines sont utilisées. De manière aléatoire, différents groupes de souris sont constitués, un groupe contrôle (traité avec le véhicule) et d'autres groupes traités par des composés.
L'appareil consiste en deux boîtes PVC (20 X 20 X 14 cm) recouvertes de Plexiglas. Une de ces boîtes est obscure. Une lumière d'une ampoule de 100W est placée 15 cm au dessus de l'autre boîte et produit ainsi la seule lumière de la pièce (d'environ 4400 Lux). Un tunnel opaque en plastique sépare la boîte obscure de celle éclairée.
Les animaux sont placés dans la boîte éclairée, avec la tête dirigée vers le tunnel. La durée passée dans la boîte éclairée et le nombre d'entrées dans la boîte éclairée sont enrégistrés pendant 5 minutes après la première entrée de l'anmal dans la boîte obscure.
Le composé ou le contrôle est administré per os 1 heure avant le test.
Les résultats sont donnés aux figures 12 et 13. N = 10 ; ** p<0,01 ; **** p<0,001 (test de Dunnett versus contrôle).
Une différence significative a été constatée entre les groupes testés pour le temps passé dans la boîte éclairée (p<0,001). Les souris traitées avec le composé de l'exemple 5a à 0,3 ; 3 et 30 mg/kg ont passé un temps significativement plus long dans la boîte éclairée en comparaison aux souris contrôle (p<0,01, contrôle versus test de Dunnett).
L'ensemble de ces résultats confirme l'effet anxiolytique et anti-dépresseur des composés selon l'invention et en particulier du composé de l'exemple 5a, notamment aux doses testées.

## Revendications

1. Composé de formule générale (I) : 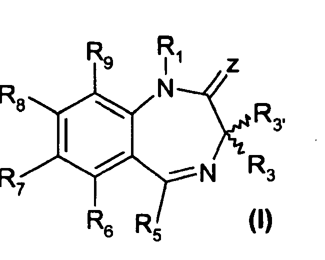 dans laquelle :
. Z représente un atome d'oxygène, de soufre ou un radical NR₂,
. R₁ est l'atome d'hydrogène ou un groupe (C₁-C₆) alkyle non substitué, ,
. R₂ est un atome d'hydrogène, un groupe (C₁-C₆) alkyle, un groupe (C₆-C₁₈)aryle ou un groupe (C₁-C₆)alkyl(C₆-C₁₈)aryle ou (C₆-C₁₈)aryl(C₁-C₄)alkyle,
R₁ et R₂ pouvant éventuellement former ensemble une chaîne hydrocarbonée linéaire ou ramifiée ayant de 2 à 6 atomes de carbone, comportant éventuellement une autre ou plusieurs autres doubles liaisons et/ou éventuellement interrompue par un atome d'oxygène, de soufre ou d'azote,
. R₃ et R_{3·}, identiques ou différents, représentent l'atome d'hydrogène, un groupe (C₁-C₁₂) alkyle, (C₃-C₆) cycloalkyle, (C₆-C₁₈) aryle, (C₆-C₁₈)aryl(C₁-C₄)alkyle, (C₁-C₁₂)alkyl(C₆-C₁₈)aryle ou un hétérocycle en (C₅-C₁₈), aromatique ou non, comportant 1 à 3 hétéroatomes, un groupe NO₂, CF₃, CN, NR'R", SR', OR', COOR', CONR'R" ou NHCOR'R", R' et R", indépendamment l'un de l'autre, étant choisis parmi l'atome d'hydrogène, un groupe (C₁-C₆) alkyle, (C₃-C₆) cycloalkyle, (C₆-C₁₂) aryle, et un hétérocycle en (C₅-C₁₂), aromatique ou non, comportant 1 à 3 hétéroatomes ;
. R₅ représente un groupe phényle au moins substitué en position 3, un groupe naphtyle, un hétérocycle en (C₅-C₁₈), aromatique ou non, comportant 1 à 3 hétéroatomes, choisi parmi le radical pyridyle, isoquinolyle, quinolyle et piperazinyle, avec les conditions que lorsque R₅ est un groupe naphtyle substitué en position 6, ce dernier n'est alors pas lié au reste de la molécule en position 2, ou lorsque R5 est un groupement pyridyle, il n'est alors pas lié au reste de la molécule en position 4, ou lorsque R5 est un groupement tetrahydro 1,2,3,4-isoquinolyle, il n'est alors pas lié au reste de la molécule en position 2,
lorsque R₅ représente un groupe phényle au moins substitué en position 3, ledit substituant étant choisi parmi : un radical alkyle, halogénoalkyle, cycloalkyle, alkényle, alkynyle, aralkyle, aryle, hétérocycle, hétérocycloalkyle, un groupe OH, =O NO₂, NH₂, CN, CF₃, COR', COOR', (C₁-C₆)alkoxy, (di)(C₁-C₆)alkylamino, NHCOR', CONR'R", dans lesquels R' et R" sont tels que définis ci-avant, CHO, CONH2, phényle éventuellement substitué, notamment par un radical acétyle, par un atome d'halogène (Cl), par un groupe CONH2 ou par un groupe CN, prop-1-ynyl éventuellement substitué, notamment par un radical benzyloxy ou tert-butyl carbamate, hex-1-ynyl éventuellement substitué, notamment par un groupe CN ou NH2, pentyle éventuellement substitué, notamment par un groupe CONH2, hexyl, pipéridinyle éventuellement substitué, notamment par un radical prop-1-ynyle, benzylaminométhyl, acétamide (CH3CONH), aminométhyl, NH2CS-, 4-phényl-1, 3-thiazol-2-yl, -CONHBenzyl, -COOEthyl, -CONHiPropyl, -CONH-(CH2)n-CONH2 (n représentant un entier de 1 à 6), -CONR'R", avec R' et R", identiques ou différents, représentant un radical alkyle de C1-C6 ou un atome d'hydrogène, -(4-benzylpypérazin-1-yl)carbonyl, -CONH-(CH2)n-phényl (n représentant un entier de 1 à 6), imidazolyle, pipérazinyle éventuellement substitué, notamment par un radical phényle,
. R₇ et R₈, indépendamment l'un de l'autre, représentent un groupe OR₁₀ dans lequel R₁₀ est un groupe (C₁-C₆) alkyle, de préférence un groupe éthyle ou méthyle, avantageusement méthyle, ou R₇ et R₈ représentent tous deux un groupe éthoxy ou méthoxy, avantageusement méthoxy, ou l'un représente un atome d'hydrogène et l'autre un groupe éthoxy ou méthoxy, avantageusement méthoxy,
. R₆ est choisi parmi l'atome d'hydrogène, un atome d'halogène, un radical alkyle, cycloalkyle, alkényle, alkynyle, un groupe aryle, aralkyle, hétérocycle, hétérocycloalkyle et un groupe OR₁₀, R₁₀ étant tel que défini ci-avant,
le groupe alkyle, cycloalkyle, alkényle, alkynyle, aralkyle, aryle, phényle, naphtyle, hétérocycle, hétérocycloalkyle ou la chaîne hydrocarbonée définie ci-dessus étant éventuellement substitué par un ou plusieurs substituants, identiques ou différents, choisis de préférence parmi un atome d'halogène, un radical alkyle, halogénoalkyle, cycloalkyle, alkényle, alkynyle, aralkyle, aryle, hétérocycle, hétérocycloalkyle, un groupe OH, =O, NO₂, NH₂, CN, CF₃, COR', COOR', (C₁-C₆)alkoxy, (di)(C₁-C₆)alkylamino, NHCOR' et CONR'R", dans lesquels R' et R" sont tels que définis ci-avant, les substituants pouvant être également substitués,
R₉ est un atome d'hydrogène,
et les sels du composé de formule (I),
à l'exclusion des composés de formule (I) dans laquelle .
- R1 est un radical alkyle, R3 et R'3 sont des atomes d'hydrogène, R6 est un atome d'hydrogène, R5 est un radical phényle au moins substitué en position 3 par un groupement méthoxy,
- R1 est un radical alkyle ou un atome d'hydrogène, R3 et R'3 sont des atomes d'hydrogène, R6 est un atome d'hydrogène, R5 est un radical phényle uniquement substitué en position 3 par un atome de chlore ou de brome,
- R1 est un radical alkyle, R3 et R'3 sont des atomes d'hydrogène, R6 est un atome d'hydrogène, R5 est un radical phényle au moins substitué en position 3 par un groupement CH2OH,
- R1 est un atome d'hydrogène, R3 et R'3 sont des atomes d'hydrogène, R6 est un atome d'hydrogène, R5 est un radical phényle uniquement substitué en position 3 par un groupement CF3,
- R1 est un radical alkyle, R3 et R'3 sont des atomes d'hydrogène, R6 est un atome d'hydrogène, R5 est un radical phényle substitué en positions 3 et 5 par un groupement CF3,
- R1 est un radical alkyle, R3 et R'3 sont des atomes d'hydrogène, R6 est un atome d'hydrogène, R7 et R8 sont des radicaux methoxy, R5 est un radical phényle substitué en position 3 par un groupement phényle,
- R1 est un radical alkyle, R3 et R'3 sont des atomes d'hydrogène, R6 est un atome d'hydrogène, R7 et R8 sont des radicaux methoxy, R5 est un radical phényle substitué en positions 3 par un groupement phényléthynyle.

2. Composé de formule (I) selon la revendication 1, dans laquelle R₅ est un radical phényle au moins substitué en position 3.

3. Composé de formule (I) selon la revendication 2, dans laquelle les groupes substituants peuvent être choisis parmi : CHO, CN, CONH2, NO2, CF3, NH2, atome d'halogène (Cl), (C1-C6)alkyle, phényle éventuellement substitué, notamment par un radical acétyle, par un atome d'halogène (Cl), par un groupe CONH2 ou par un groupe CN, prop-1-ynyl éventuellement substitué, notamment par un radical benzyloxy ou tert-butyl carbamate, hex-1-ynyl éventuellement substitué, notamment par un groupe CN ou NH2, pentyle éventuellement substitué, notamment par un groupe CONH2, hexyl, pipéridinyle éventuellement substitué, notamment par un radical prop-1-ynyle, benzylaminométhyl, acétamide (CH3CONH), aminométhyl, NH2CS-, 4-phényl-1, 3-thiazol-2-yl, -CONHBenzyl, -COOEthyl, - CONHiPropyl, -CONH-(CH2)n-CONH2 (n représentant un entier de 1 à 6), -CONR'R", avec R' et R", identiques ou différents, représentant un radical alkyle de C1-C6 ou un atome d'hydrogène, -(4-benzylpypérazin-1-yl)carbonyl, -CONH-(CH2)n-phényl (n représentant un entier de 1 à 6), imidazolyle, pipérazinyle éventuellement substitué, notamment par un radical phényle.

4. Composé de formule (I) selon l'une des revendications 1 à 3, dans laquelle R5 est un radical phényle substitué en positions 3 et 4, notamment par une chaîne hydrocarbonée comportant éventuellement au moins un hétéroatome, tel que l'oxygène, comme la chaîne méthylènedioxy (-O-CH2-O-) formant un cycle avec le radical phényl auquel il est rattaché.

5. Composé de formule (I) selon la revendication 1, dans laquelle R5 est le radical 3-pyridyle, 4-isoquinolyle, pipérazinyle éventuellement substitué, notamment en position 4 par un groupement aryle, tel que le phényl.

6. Composé de formule (I) selon la revendication 1, dans laquelle Z représente un atome de soufre ou -NR2, avec en particulier R2 formant un cycle avec R1 de type imidazole.

7. Composé de formule (I) selon la revendication 1, 2 ou 3, dans laquelle :
- Z est l'atome d'oxygène et/ou
- R₆ représente l'atome d'hydrogène, un atome d'halogène, un radical phényle, un groupe (C₁-C₆) alkyle ou un groupe OR₁₀ dans lequel R₁₀ est un groupe (C₁-C₆) alkyle, de préférence un groupe éthyle ou méthyle, et/ou
- R₃ et R_{3'}, identiques ou différents, représentent un atome d'hydrogène, et/ou
- R₁ est un groupe (C₁-C₆)alkyle non substitué.

8. Composé de formule (I) selon l'une des revendications 1 à 7, dans laquelle R₁ représente un atome d'hydrogène ou un groupe (C₁-C₃) alkyle non substitué.

9. Composé de formule (I) selon l'une des revendications 1 à 8, dans laquelle R₅ est un groupe phényle substitué par :
(a) un ou plusieurs groupes OR', en particulier méthoxy ou éthoxy, ou
(b) un groupe COR', en particulier acétyle ou aldéhyde, ou
(c) un groupe CONR'R", en particulier CONH2,ou
(d) un groupe CN, ou
(e) un groupe trifluorométhyle, ou
(f) un groupe alkyle, par exemple méthyle, ou alkynyle, par exemple hexynyle ou propynyle, ou
(g) un groupe aryle ou hétérocycle, notamment un groupe phényle, furyle, pyridyle, pipéridine, thiazole ou thiényle, ledit aryle ou hétérocycle étant lui-même éventuellement substitué par un ou plusieurs groupes choisis de préférence parmi les groupes (a)-(g).

10. Composé de formule (I) selon l'une des revendications 1 à 9, dans laquelle au moins un des groupements R7 et R8, avantageusement les deux, représente un radical méthoxy.

11. Composé selon la revendication 1, le composé étant choisi parmi les composés suivants : '
3-(7,8-Diméthoxy-2-oxo-2,3-dihydro-1H-1,4-benzodiazépin-5-yl)benzonitrile , **3a**
3-(7,8-diméthoxy-1-méthyl-2-oxo-2,3-dihydro-1*H*-1,4-benzodiazépin-5-yl)-benzonitrile, **4a**
3-(1-éthyl-7,8-dimethoxy-2-oxo-2,3-dihydro-1H-1,4-benzodiazépin-5-yl)benzonitrile, **4k**
3-(7,8-diméthoxy-1-propyl-2-oxo-2,3-dihydro-1H-1,4-benzodiazépin-5-yl)benzonitrile, **4l**
7,8-Diméthoxy-1-méthyl-5-[(3-trifluorométhyl)phényl]-1,3-dihydro-2*H*-1,4-benzodiazépin-2-one **4p**
7,8-diméthoxy-1-éthyl-5-[3-(trifluorométhyl)phényl]-1,3-dihydro-2H-1,4-benzodiazépin-2-one **4q**
5-[3-(trifluorométhyl)phényl]-7,8-diméthoxy-1-*n*-propyl-1,3-dihydro-1,4-benzodiazépin-2-one, **4r**
3-(7,8-Diméthoxy-1-méthyl-2-oxo-2,3-dihydro-1*H*-1,4-benzodiazépin-5-yl)-benzamide **5a**
3-(6-Bromo-7,8-diméthoxy-1-méthyl-2-oxo-2,3-dihydro-1*H*-1,4-benzodiazépin-5-yl)benzamide, **5b**
3-(7,8-Diméthoxy-1-methyl-2-oxo-6-phényt-2,3-dihydro-1*H*-1,4-benzodiazépin-5-yl)benzamide, **5c**
3-(7,8-diméthoxy-2-oxo-2,3-dihydro-1H-1,4-benzodiazépin-5-yl)benzamide, **5e**
3-(7,8-diméthoxy-1-propyl-2-oxo-2,3-dihydro-1H-1,4-benzodiazépin-5-yl)benzamide, **5f**
3-(1-éthyl-7,8-diméthoxy-2-oxo-2,3-dihydro-1H-1,4-benzodiazépin-5-yl)benzamide, **5g**
3-(7,8-diméthoxy-1,3-diméthyl-2-oxo-2,3-dihydro-1H-1,4-benzodiazépin-5-yl) benzamide, **5j**
3-[3-(3,4-dichlorobenzyl)-7,8-diméthoxy-1-méthyl-2-oxo-2,3-dihydro-1H-1,4-benzodiazépin-5-yl]benzamide, **5k**
3-(8-méthoxy-1-méthyl-2-oxo-2,3-dihydro-1H-1,4-benzodiazépin-5-yl)benzamide, **5l**
3-(6,8-Dimethoxy-2-oxo-2,3-dihydro-1*H*-1,4-benzodiazepin-5-yl)benzamide, **5n**
3-(6,8-Dimethoxy-1-méthyl-2-oxo-2,3-dihydro-1*H*-1,4-benzodiazépin-5-yl)benzamide, **5o**
*Tert*-butyl 3-[3-(7,8-diméthoxy-1-méthyl-2-oxo-2,3-dihydro-1*H*-1,4-benzodiazépin-5-yl)phényl]propynylcarbamate, **6a**
7,8-Diméthoxy-5-(3'-hex-1-ynylphényl)-1-*N*-méthyl-1,3-dihydro-2*H*-1,4-benzodiazépin-2-one, **6b**
7,8-Diméthoxy-1-méthyl-5-[3-(3-pipéridin-1-ylprop-1-ynyl)phényl]-1,3-dihydro-2*H*-1,4-benzodiazépin-2-one, **6c**
6-[3-(7,8-Diméthoxy-1-méthyl-2-oxo-2,3-dihydro-1*H*-1,4-benzodiazépin-5-yl)phényl]hex-5-ynenitrile, **6d**
7,8-Diméthoxy-5-(3'-hexylphényl)-1-*N*-méthyl-1,3-dihydro-2*H*-1,4-benzodiazépin-2-one, **6e**
5-[3-(3-aminopropyl)phényl-7,8-diméthoxy-1-méthyl-1,3-dihydro-2H-1,4-benzodiazépin-2-one trifluoroacétate, **6h**
6-[3-(7,8-diméthoxy-1-méthyl-2-oxo-2,3-dihydro-1*H*-1,4-benzodiazépin-5-yl)phényl]hexanamide, **6i**
5-(4'-chloro-1,1'-biphényl-3-yl)-7,8-diméthoxy-1-méthyl-1,3-dihydro-2H-1,4-benzodiazépin-2-one, **6j**
5-{3-[3-(benzyloxy)prop-1-ynyl]phényl}-1-éthyl-7,8-diméthoxy-1,3-dihydro-2H-1,4-benzodiazépin-2-one, **6k**
3'-(7,8-diméthoxy-1-méthyl-2-oxo-2,3-dihydro-1H-1,4-benzodiazépin-5-yl)-1,1'-biphényl-3-carbonitrile, **6l**
3'-(7,8-diméthoxy-1-méthyl-2-oxo-2,3-dihydro-1H-1,4-benzodiazépin-5-yl)-1,1'-biphényl-4-carbonitrile, **6m**
3'-(7,8-diméthoxy-1-méthyl-2-oxo-2,3-dihydro-1H-1,4-benzodiazépin-5-yl)-1,1'-biphényl-4-carboxamide, **6n**
3'-(7,8-diméthoxy-1-méthyl-2-oxo-2,3-dihydro-1H-1,4-benzodiazépin-5-yl)-1,1'-biphényl-3-carboxamide, **6o**
3-[3-(3,4-dichlorobenzyl)-7,8-diméthoxy-1-méthyl-2-oxo-2,3-dihydro-1H-1,4-benzodiazépin-5-yl]benzonitrile, **7b**
7,8-diméthoxy-1,3-diméthyl-5-(3-trifluorométhylphényl)-1,3-dihydro-2H-1,4-benzodiazépin-2-one, **7c**
3-(7,8-diméthoxy-1,3-diméthyl-2-oxo-2,3-dihydro-1H-1,4-benzodiazépin-5-yl)benzonitrile, **7d**
5-[3-(aminométhyl)phényl]-7,8-diméthoxy-1-méthyl-1,3-dihydro-2H-1,4-benzodiazépin-2-one, **8a**
N-[3-(7,8-diméthoxy-1-méthyl-2-oxo-2,3-dihydro-1H-1,4-benzodiazépin-5-yl)benzyl]acétamide, **8b**
3-(7,8-diméthoxy-1-méthyl-2-oxo-2,3-dihydro-1H-1,4-benzodiazépin-5-yl)thiobenzamide, **9a**
7,8-diméthoxy-1-méthyl-5-[3-(4-phényl-1, 3-thiazol-2-yl)phényl]-1,3-dihydro-2H-1,4-benzodiazépin-2-one, **9b**
5-(3-cyanophényl)-7,8-diméthoxy-1,3-dihydro-2H-1,4-benzodiazépin-2-thione, **10d**
3-(7,8-diméthoxy-2-méthylamino-1,3-dihydro-3H-1,4-benzodiazépin-5-yl)benzonitrile, **12a**
7,8-diméthoxy-1-méthyl-5-(3-pyridyl)-1,3-dihydro-1,4-benzodiazépin-2-one, **17b**
7,8-diméthoxy-1-méthyl-5-(3-nitrophényl)-1,3-dihydro-1,4-benzodiazépin-2-one, **17c**
5-(7,8-diméthoxy-1-méthyl-2-oxo-2,3-dihydro-1H-1,4-benzodiazépin-5-yl)-2-benzonitrile, **17d**
5-(3-acétylphényl)-7,8-diméthoxy-1-méthyl-1,3-dihydro-1,4-benzodiazépin-2-one, **17e**
5-(4-isoquinoléinyl)-7,8-diméthoxy-1-méthyl-1,3-dihydro-1,4-benzodiazépin-2-one, **17f**
7,8-diméthoxy-5-(3-hydroxyméthylphényl)-1-méthyl-3-propyl-1,3-dihydro-2H-1,4-benzodiazépin-2-one, **17h**
5-(3-aminophényl)-7,8-diméthoxy-1-méthyl-1,3-dihydro-2H-1,4-benzodiazépin-2-one, **17i**
5-(3,4-dichlorophényl)-7,8-diméthoxy-1-méthyl-l ,3-dihydro-1,4-benzodiazépin-2-one, **17j**
7,8-diméthoxy-1-méthyl-5-(3-méthylphényl)-1,3-dihydro-1,4-benzodiazépin-2-one, **17k.**
5-(3-formylphényl)-7,8-diméthoxy-1-méthyl-1,3-dihydro-1,4-benzodiazépin-2-one, **17l**
Chlorhydrate de la 5-[3-(benzylaminométhyl)phényl]-7,8-diméthoxy-1-méthyl-1,3-dihydro-2H-1,4-benzodiazépin-2-one, **17m**
N-[3-(7,8-diméthoxy-1-méthyl-2-oxo-2,3-dihydro-1H-1,4-benzodiazépin-5-yl) phényl]acétamide, **17n**
7,8-diméthoxy-1-méthyl-5-(3,4-méthylènedioxyphényl)-1,3-dihydro-2H-1,4-benzodiazépin-2-one, **17o**
3-(6-Bromo-7,8-diméthoxy-1-méthyl-2-oxo-2,3-dihydro-1*H*-1,4-benzodiazépin-5-yl)benzonitrile, **24b**
3-(7,8-Diméthoxy-1-methyl-2-oxo-6-phényl-2,3-dihydro-1*H*-1,4-benzodiazépin-5-yl)benzonitrile, **25b**
*Tert*-butyl 3-[5-(3-cyanophényl)-(7,8-diméthoxy-1-méthyl-2-oxo-2,3-dihydrp-1*H*-1,4-benzodiazépin-6-yl)]prop-2-ynylcarbamate, **25e**
3-[6-(3-aminoethynyl)-7,8-diméthoxy-1-méthyl-2-oxo-2,3-dihydro-1*H*-1,4-benzodiazépin-5-yl]benzonitrile, **25g**
3-(8-méthoxy-2-oxo-2,3-dihydro-1H-1,4-benzodiazépin-5-yl)benzonitrile, **28a**
3-(7-méthoxy-2-oxo-2,3-dihydro-1H-1,4-benzodiazépin-5-yl)benzonitrile, **28c**
3-(6,8-Dimethoxy-2-oxo-2,3-dihydro-1*H*-1,4-benzodiazepin-5-yl)benzonitrile, **28g**
3-(8-méthoxy-1-méthyl-2-oxo-2,3-dihydro-1H-1,4-benzodiazépin-5-yl)benzonitrile, **29a**
3-(6,8-Dimethoxy-1-methyl-2-oxo-2,3-dihydro-1*H*-1,4-benzodiazepin-5-yl)benzonitrile, **29b**
Benzoate de 3-(7,8-diméthoxy-1-méthyl-2-oxo-2,3-dihydro-1H-1,4-benzodiazépin-5-yl)méthyl, **34a**
Acide 3-(7,8-diméthoxy-1-méthyl-2-oxo-2,3-dihydro-1H-1,4-benzodiazépin-5-yl) benzoïque, **35a**
3-(7,8-diméthoxy-1-méthyl-2-oxo-2,3-dihydro-1H-1,4-benzodiazépin-5-yl)N-isopropylbenzamide, **36a**
N-benzyl-3-(7,8-diméthoxy-1-méthyl-2-oxo-2,3-dihydro-1H-1,4-benzodiazépin-5-yl) benzamide, **36b**
N-(6-amino-6-oxohexyl)-3-(7,8-diméthoxy-1-méthyl-2-oxo-2,3-dihydro-1H-1,4-benzodiazépin-5-yl) benzamide, **36c**
3-(7,8-diméthoxy-1-méthyl-2-oxo-2,3-dihydro-1H-1,4-benzodiazépin-5-yl) N, N-diméthylbenzamide **36d**
5-{3-[(4-benzylpypérazin-1-yl)carbonyl]phényl}7,8-diméthoxy-1-méthyl-2-oxo-2,3-dihydro-1H-1,4-benzodiazépin-2-one, **36e**
3-(7,8-diméthoxy-1-méthyl-2-oxo-2,3-dihydro-1H-1,4-benzodiazépin-5-yl) N-(3-phénylpropyl)benzamide, **36f.**

12. Composé selon la revendication 1, le composé étant choisi parmi les composés suivants :
7,8-Diméthoxy-l-méthyl-5-[(3-trifluorométhyl)phényl]-1,3-dihydro-2*H*-1,4-benzodiazépin-2-one **4p**
3-(7,8-Diméthoxy-1-méthyl-2-oxo-2,3-dihydro-1*H*-1,4-benzodiazépin-5-yl)-benzamide **5a**
3-(6-Bromo-7,8-diméthoxy-1-méthyl-2-oxo-2,3-dihydro-1*H*-1,4-benzodiazépin-5-yl)benzamide, **5b**
*Tert*-butyl 3-[3-(7,8-diméthoxy-1-méthyl-2-oxo-2,3-dihydro-1*H*-1,4-benzodiazépin-5-yl)phényl]propynylcarbamate, **6a**
7,8-Diméthoxy-5-(3'-hex-1-ynylphényl)-1-*N*-méthyl-1,3-dihydro-2*H*-1,4-benzodiazépin-2-one, **6b**
6-[3-(7,8-Diméthoxy-1-méthyl-2-oxo-2,3-dihydro-1*H*-1,4-benzodiazépin-5-yl)phényl]hex-5-ynenitrile, **6d**
7,8-Diméthoxy-5-(3'-hexylphényl)-1-*N*-méthyl-1,3-dihydro-2*H*-1,4-benzodiazépin-2-one, **6e**
5-(4'-chloro-1,1'-biphényl-3-yl)-7,8-diméthoxy-1-méthyl-1,3-dihydro-2H-1,4-benzodiazépin-2-one, **6j**
3'-(7,8-diméthoxy-1-méthyl-2-oxo-2,3-dihydro-1H-1,4-benzodiazépin-5-yl)-1,1'-biphényl-4-carbonitrile, **6m**
3'-(7,8-diméthoxy-1-méthyl-2-oxo-2,3-dihydro-1H-1,4-benzodiazépin-5-yl)-1,1'-biphényl-4-carboxamide, **6n**
7,8-diméthoxy-1-méthyl-5-[3-(4-phényl-1, 3-thiazol-2-yl)phényl]-1,3-dihydro-2H-1,4-benzodiazépin-2-one, **9b**
7,8-diméthoxy-1-méthyl-5-(3-pyridyl)-1,3-dihydro-1,4-benzodiazépin-2-one, **17b.**

13. Composé selon la revendication 1, le composé étant le suivant :
3-(7,8-Diméthoxy-1-méthyl-2-oxo-2,3-dihydro-1*H*-1,4-benzodiazépin-5-yl)-benzamide **5a.**

14. Composition pharmaceutique comprenant au moins un composé tel que défini dans l'une des revendications 1 à 13 et un véhicule ou un excipient acceptable sur le plan pharmaceutique.

15. Utilisation d'un composé tel que défini selon l'une des revendications 1 à 13 pour la préparation d'une composition pharmaceutique destinée à traiter des pathologies concernant le système nerveux central.

16. Utilisation selon la revendications précédente, **caractérisée en ce que** la pathologie est choisie parmi la dépression, la schizophrénie, l'anxiété, le désordre bipolaire, les désordres de défaut d'attention, les troubles du sommeil, les troubles obsessionnelles compulsifs, la fibromyalgie, syndrome de Tourette, pharmacodépendance, l'épilepsie, la maladie d'Alzheimer, la maladie de Parkinson, la sclérose latérale amyotrophique, la sclérose multiple, l'obésité et la démence des corps de Lewy.

17. Utilisation d'un composé tel que défini selon l'une des revendications 1 à 13 pour la préparation d'une composition pharmaceutique destinée à traiter des pathologies impliquant le système nerveux périphérique, les pathologies dues ou impliquant des dysfonstionnements de la libération de la prolactine, choisies parmi le syndrome de la jambe sans repos, ou les désordres rhumatismaux, allergiques ou autoinflammatoires, choisis parmi l'arthrite rhumatoïde, la rhinite et l'asthme.

18. Utilisation d'un composé tel que défini selon l'une des revendications 1 à 13 pour la préparation d'une composition pharmaceutique destinée au traitement de désordres du système nerveux, central ou périphérique, de nature chronique ou aiguë.

19. Utilisation d'un composé tel que défini selon l'une des revendications 1 à 13 pour la préparation d'une composition pharmaceutique pour le traitement des troubles de la mémoire ou de troubles cognitifs, en particulier troubles cognitifs modérés.

20. Utilisation d'un composé tel que défini selon l'une des revendications 1 à 13 pour la préparation d'une composition pharmaceutique pour le traitement de pathologies neuro-dégénératives.

21. Utilisation d'un composé tel que défini selon l'une des revendications 1 à 13 pour la préparation d'une composition pharmaceutique pour le traitement de l'obésité.

## Claims

1. A compound represented by general formula (I) : 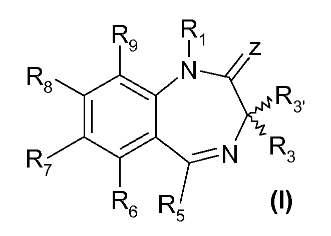 in which :
. Z represents an oxygen or sulfur atom or a NR₂ group,
. R₁ is the hydrogen atom or an unsubsituted (C₁-C₆) alkyl group,
. R₂ is a hydrogen atom, a (C₁-C₆) alkyl group, a (C₆-C₁₈)aryl group or a (C₁-C₆)alkyl(C₆-C₁₈)aryl or (C₆-C₁₈)aryl(C₁-C₄)alkyl group,
R₁ and R₂ taken together can optionally form a linear or branched hydrocarbon chain having from 2 to 6 carbon atoms, possibly containing one or several other double bonds and/or possibly interrupted by an oxygen, sulfur or nitrogen atom,
. R₃ and R_{3'}, which are the same or different, represent the hydrogen atom, a (C₁-C₁₂) alkyl, (C₃-C₆) cycloalkyl, (C₆-C₁₈) aryl, (C₆-C₁₈)aryl(C₁-C₄)alkyl, (C₁-C₁₂)alkyl(C₆-C₁₈)aryl group or a (C₅-C₁₈) heterocycle, aromatic or not, containing 1 to 3 heteroatoms, a NO₂, CF₃, CN, NR'R", SR', OR', COOR', CONR'R" or NHCOR'R" group, R' and R", independently of each another, being selected in the group consisting of the hydrogen atom, a (C₁-C₆) alkyl, (C₃-C₆) cycloalkyl, (C₆-C₁₂) aryl group and a (C₅-C₁₂) heterocycle, aromatic or not, containing 1 to 3 heteroatoms;
. R₅ represents a phenyl group substituted at least in position 3, a naphthyl group, a (C₅-C₁₈) heterocycle, aromatic or not, containing 1 to 3 heteroatoms, selected in the group consisting of the pyridyl, isoquinolyl, quinolyl and piperazinyl group, provided that when R₅ is a naphthyl group substituted in position 6, then the latter is not attached to the rest of the molecule in position 2, or when R₅ is a pyridyl group, then it is not attached to the rest of the molecule in position 4, or when R₅ is a tetrahydro 1,2,3,4-isoquinolyl group, then it is not attached to the rest of the molecule in position 2,
when R₅ represents a phenyl group substituted at least in position 3, said substituent being selected in the group consisting of: an alkyl, halogenoalkyl, cycloalkyl, alkenyl, alkynyl, aralkyl, aryl, heterocycle, heterocycloalkyl group, a OH, =O, NO₂, NH₂, CN, CF₃, COR', COOR', (C₁-C₆)alkoxy, (di)(C₁-C₆)alkylamino, NHCOR', CONR'R" group, in which R' and R" are such as defined hereinabove, CHO, CONH2, phenyl optionally substituted, in particular by an acetyl group, by a halogen atom (Cl), by a CONH2 group or by a CN, prop-1-ynyl optionally substituted, in particular by a benzyloxy or tert-butyl carbamate, hex-1-ynyl optionally substituted, in particular by a CN or NH2, pentyl optionally substituted, in particular by a CONH2, hexyl, piperidinyl group optionally substituted, in particuar by a prop-1-ynyl, benzylaminomethyl, acetamide (CH3CONH), aminomethyl, NH2CS-, 4-phenyl-1, 3-thiazol-2-yl, -CONHBenzyl, -COOEthyl, -CONHiPropyl, -CONH-(CH2)n-CONH2 (n representing a whole number from 1 to 6), -CONR'R" group, with R' and R", which are the same or different, representing a C1-C6 alkyl group or a hydrogen atom, -(4-benzylpyperazin-1-yl)carbonyl, -CONH-(CH2)n-phenyl (n representing a whole number from 1 to 6), imidazolyl, piperazinyl optionally substituted, in particular by a phenyl group,
. R₇ and R₈, independently of each other, are a OR₁₀ group in which R₁₀ represents a (C₁-C₆) alkyl, preferably an ethyl or methyl group, advantageously methyl group, or R₇ and R₈ both represent the ethoxy or methoxy group, preferably methoxy group, or one represents the hydrogen atom and the other represents the ethoxy or methoxy group, advantageously methoxy group,
. R₆ is selected in the group consisting of the hydrogen atom, a halogen atom, an alkyl, cycloalkyl, alkenyl, alkynyl group, an aryl, aralkyl, heterocycle, heterocycloalkyl group and a OR₁₀ group, R₁₀ being such as defined hereinabove,
the alkyl, cycloalkyl, alkenyl, alkynyl, aralkyl, aryl, phenyl, naphthyl, heterocycle, heterocycloalkyl group or the hydrocarbon chain defined hereinabove being optionally substituted by one or more substituents, which are the same or different, preferably selected in the group consisting of a halogen atom, an alkyl, halogenoalkyl, cycloalkyl, alkenyl, alkynyl, aralkyl, aryl, heterocycle, heterocycloalkyl group, a OH, =O, NO₂, NH₂, CN, CF₃, COR', COOR', (C₁-C₆)alkoxy, (di)(C₁-C₆)alkylamino, NHCOR' and CONR'R" group, in which R' and R" are such as defined hereinabove, the substitutents also being optionally substituted,
. R₉ is the hydrogen atom,
and one of the salts thereof,
with the exception of compounds represented by formula (I) in which .
- R1 is an alkyl group, R3 and R'3 are hydrogen atoms, R6 and R9 are hydrogen atoms, R5 is a phenyl group substituted at least in position 3 by a methoxy group,
- R1 is an alkyl group or a hydrogen atom, R3 and R'3 are hydrogen atoms, R6 and R9 are hydrogen atoms, R5 is a phenyl group substituted only in position 3 by a chlorine or bromine atom,
- R1 is an alkyl group, R3 and R'3 are hydrogen atoms, R6 and R9 are hydrogen atoms, R5 is a phenyl group substituted at least in position 3 by a CH2OH group,
- R1 is a hydrogen atom, R3 and R'3 are hydrogen atoms, R6 and R9 are hydrogen atoms, R5 is a phenyl group substituted only in position 3 by a CF3 group,
- R1 is an alkyl group, R3 and R'3 are hydrogen atoms, R6 and R9 are hydrogen atoms, R5 is a phenyl group substituted in positions 3 and 5 by a CF3 group,
- R1 is an alkyl group, R3 and R'3 are hydrogen atoms, R6 and R9 are hydrogen atoms, R7 and R8 are methoxy groups, R5 is a phenyl group substituted in positions 3 by a phenyl group,
- R1 is an alkyl group, R3 and R'3 are hydrogen atoms, R6 and R9 are hydrogen atoms, R7 and R8 are methoxy groups, R5 is a phenyl group substituted in positions 3 by a phenylethynyl group.

2. The compound represented by formula (I) according to claim 1, in which R₅ is a phenyl group substituted at least in position 3.

3. The compound represented by formula (I) according to claim 2, wherein the substituents are selected in the group consisting of : CHO, CN, CONH2, N02, CF3, NH2, halogen atom (C1), (C1-C6) alkyl, phenyl optionally substituted, in particular by an acetyl group, by a halogen atom (C1), by a CONH2 group or by a CN, prop-1-ynyl group optionally substituted, in particular by a benzyloxy or tert-butyl carbamate, hex-1-ynyl group optionally substituted, in particular by a CN or NH2 group, pentyl optionally substituted, in particular by a CONH2, hexyl, piperidinyl optionally substituted, in particular by a prop-1-ynyl, benzylaminomethyl, acetamide (CH3CONH), aminomethyl, NH2CS-, 4-phenyl-1, 3-thiazol-2-yl, -CONHBenzyl, -COOEthyl, -CONHiPropyl, -CONH-(CH2)n-CONH2 (n representing a whole number from 1 to 6), -CONR'R" group, with R' and R", which are the same or different, representing a C1 to C6 alkyl group or a hydrogen atom, -(4-benzylpyperazin-1-yl)carbonyl, -CONH-(CH2)n-phenyl (n representing a whole number from 1 to 6), imidazolyl, piperazinyl group optionally substituted, in particular by a phenyl group.

4. The compound represented by formula (I) according to anyone of claims 1 to 3, in which R5 is a phenyl group substituted in positions 3 and 4, in particular by a hydrocarbon chain possibly containing at least one heteroatom, such as oxygen, like the methylenedioxy (-O-CH2-O-) chain forming a ring with the phenyl group to which it is attached.

5. The compound represented by formula (I) according to claim 1, in which R5 is the 3-pyridyl, 4-isoquinolyl, piperazinyl group optionally substituted, in particular in position 4 by an aryl group, such as phenyl.

6. The compound represented by formula (I) according to claim 1, in which Z represents a sulfur atom or -NR2, with in particular R2 forming a ring of the imidazole type with R1.

7. The compound represented by formula (I) according to claim 1, 2 or 3, in which :
- Z is the oxygen atom, and/or
- R₆ represents the hydrogen atom, a halogen atom, a phenyl group, a (C₁-C₆) alkyl group or a OR₁₀ group in which R₁₀ is a (C₁-C₆) alkyl group, preferably an ethyl or methyl group, and/or
- R₃ and R₃', which are the same or different, represent a hydrogen atom, and/or
- R₁ is an unsubstituted (C1-C6) alkyl.

8. The compound represented by formula (I) according to anyone of claims 1 to 7, in which R₁ represents a hydrogen atom or an unsubstituted (C₁-C₃) alkyl.

9. The compound represented by formula (I) according to anyone of claims 1-8, in which R₅ is a phenyl group substituted by:
(a) one or more OR' groups, in particular methoxy or ethoxy, or
(b) a COR' group, in particular acetyl or aldehyde, or
(c) a CONR'R" group, in particular CONH2, or
(d) a CN group, or
(e) a trifluoromethyl group, or
(f) an alkyl group, for example methyl, or alkynyl group, for example hexynyl or propynyl, or
(g) an aryl or heterocycle group, in particular phenyl, furyl, pyridyl, piperidine, thiazole or thienyl, said aryl or heterocycle itself being optionally substituted by one or more groups preferably selected from groups (a)-(g).

10. The compound represented by formula (I) according to one of claims 1 to 9, in which at least one group, preferably both, R₇ and R₈ represents the methoxy group.

11. The compound according to claim 1, wherein said compound is selected from the following compounds:
3-(7,8-dimethoxy-2-oxo-2,3-dihydro-1H-1,4-benzodiazepin-5-yl)benzonitrile, **3a**
3-(7,8-dimethoxy-1-methyl-2-oxo-2,3-dihydro-1*H*-1,4-benzodiazepin-5-yl)-benzonitrile, **4a**
3-(1-ethyl-7,8-dimethoxy-2-oxo-2,3-dihydro-1H-1,4-benzodiazepin-5-yl)benzonitrile, **4k**
3-(7,8-dimethoxy-1-propyl-2-oxo-2,3-dihydro-1H-1,4-benzodiazepin-5-yl)benzonitrile, **4l**
7,8-dimethoxy-1-methyl-[5-(3-trifluoromethyl)phenyl]-1,3-dihydro-2*H*-1,4-benzodiazepin-2-one, **4p**
7,8-dimethoxy-1-ethyl-5-[3-(trifluoromethyl)phenyl]-1,3-dihydro-2H-1,4-benzodiazepin-2-one, **4q**
5-[3-(trifluoromethyl)phenyl]-7,8-dimethoxy-1-*n*-propyl-1,3-dihydro-1,4-benzodiazepin-2-one, **4r**
3-(7,8-dimethoxy-1-methyl-2-oxo-2,3-dihydro-1*H*-1,4-benzodiazepin-5-yl)-benzamide **5a**
3-(6-bromo-7,8-dimethoxy-1-methyl-2-oxo-2,3-dihydro-1*H*-1,4-benzodiazepin-5-yl)benzamide, **5b**
3-(7,8-dimethoxy-1-methyl-2-oxo-6-phenyl-2,3-dihydro-1*H*-1,4-benzodiazepin-5-yl)benzamide, **5c**
3-(7,8-dimethoxy-2-oxo-2,3-dihydro-1H-1,4-benzodiazepin-5-yl)benzamide, **5e**
3-(7,8-dimethoxy-1-propyl-2-oxo-2,3-dihydro-1H-1,4-benzodiazepin-5-yl)benzamide, **5f**
3-(1-ethyl-7,8-dimethoxy-2-oxo-2,3-dihydro-1H-1,4-benzodiazepin-5-yl)benzamide, **5g**
3-(7,8-dimethoxy-1,3-dimethyl-2-oxo-2,3-dihydro-1H-1,4-benzodiazepin-5-yl) benzamide, **5j**
3-[3-(3,4-dichlorobenzyl)-7,8-dimethoxy-1-methyl-2-oxo-2,3-dihydro-1H-1,4-benzodiazepin-5-yl]benzamide, **5k**
3-(8-methoxy-1-methyl-2-oxo-2,3-dihydro-1H-1,4-benzodiazepin-5-yl)benzamide, **5l**
3-(6,8-dimethoxy-2-oxo-2,3-dihydro-1*H*-1,4-benzodiazepin-5-yl)benzamide, **5n**
3-(6,8-dimethoxy-1-methyl-2-oxo-2,3-dihydro-1*H*-1,4-benzodiazepin-5-yl)benzamide, **5o**
*tert*-butyl-3-[3-(7,8-dimethoxy-1-methyl-2-oxo-2,3-dihydro-1*H*-1,4-benzodiazepin-5-yl)phenyl]propynylcarbamate, **6a**
7,8-dimethoxy-5-(3'-hex-1-ynylphenyl)-1-*N*-methyl-1,3-dihydro-2*H*-1,4-benzodiazepin-2-one, **6b**
7,8-dimethoxy-1-methyl-5-[3-(3-piperidin-1-ylprop-1-ynyl)phenyl]-1,3-dihydro-2*H*-1,4-benzodiazepin-2-one, **6c**
6-[3-(7,8-dimethoxy-1-methyl-2-oxo-2,3-dihydro-1*H*-1,4-benzodiazepin-5-yl)phenyl]hex-5-ynenitrile, **6d**
7,8-dimethoxy-5-(3'-hexylphenyl)-1-*N*-methyl-1,3-dihydro-2*H*-1,4-benzodiazepin-2-one, **6e**
5-[3-(3-aminopropyl)phenyl-7,8-dimethoxy-1-methyl-1,3-dihydro-2H-1,4-benzodiazepin-2-one trifluoroacetate, **6h**
6-[3-(7,8-dimethoxy-1-methyl-2-oxo-2,3-dihydro-1*H*-1,4-benzodiazepin-5-yl)phenyl]hexanamide, **6i**
5-(4'-chloro-1,1'-biphenyl-3-yl)-7,8-dimethoxy-1-methyl-1,3-dihydro-2H-1,4-benzodiazepin-2-one, **6j**
5- {3-[3-(benzyloxy)prop-1-ynyl]phenyl}-1-ethyl-7,8-dimethoxy-1,3-dihydro-2H-1,4-benzodiazepin-2-one, **6k**
3'-(7,8-dimethoxy-1-methyl-2-oxo-2,3-dihydro-1H-1,4-benzodiazepin-5-yl)-1,1'-biphenyl-3-carbonitrile, **6l**
3'-(7,8-dimethoxy-1-methyl-2-oxo-2,3-dihydro-1H-1,4-benzodiazepin-5-yl)-1,1'-biphenyl-4-carbonitrile, **6m**
3'-(7,8-dimethoxy-1-methyl-2-oxo-2,3-dihydro-1H-1,4-benzodiazepin-5-yl)-1,1'-biphenyl-4-carboxamide, **6n**
3'-(7,8-dimethoxy-1-methyl-2-oxo-2,3-dihydro-1H-1,4-benzodiazepin-5-yl)-1,1'-biphenyl-3-carboxamide, **6o**
3-[3-(3,4-dichlorobenzyl)-7,8-dimethoxy-1-methyl-2-oxo-2,3-dihydro-1H-1,4-benzodiazepin-5-yl]benzonitrile, **7b**
7,8-dimethoxy-1,3-dimethyl-5-(3-trifluoromethylphenyl)-1,3-dihydro-2H-1,4-benzodiazepin-2-one, **7c**
3-(7,8-dimethoxy-1,3-dimethyl-2-oxo-2,3-dihydro-1H-1,4-benzodiazepin-5-yl)benzonitrile, **7d**
5-[3-(aminomethyl)phenyl]-7,8-dimethoxy-1-methyl-1,3-dihydro-2H-1,4-benzodiazepin-2-one, **8a**
N-[3-(7,8-dimethoxy-1-methyl-2-oxo-2,3-dihydro-1H-1,4-benzodiazepin-5-yl)benzyl]acetamide, **8b**
3-(7,8-dimethoxy-1-methyl-2-oxo-2,3-dihydro-1H-1,4-benzodiazepin-5-yl)thiobenzamide, **9a**
7,8-dimethoxy-1-methyl-5-[3-(4-phenyl-1,3-thiazol-2-yl)phenyl]-1,3-dihydro-2H-1,4-benzodiazepin-2-one, **9b**
5-(3-cyanophenyl)-7,8-dimethoxy-1,3-dihydro-2H-1,4-benzodiazepin-2-thione, **10d**
3-(7,8-dimethoxy-2-methylamino-1,3-dihydro-3H-1,4-benzodiazepin-5-yl)benzonitrile, **12a**
7,8-dimethoxy-1-methyl-5-(3-pyridyl)-1,3-dihydro-1,4-benzodiazepin-2-one, **17b**
7,8-dimethoxy-1-methyl-5-(3-nitrophenyl)-1,3-dihydro-1,4-benzodiazepin-2-one, **17c**
5-(7,8-dimethoxy-1-methyl-2-oxo-2,3-dihydro-1H-1,4-benzodiazepin-5-yl)-2-benzonitrile, **17d**
5-(3-acetylphenyl)-7,8-dimethoxy-1-methyl-1,3-dihydro-1,4-benzodiazepin-2-one, **17e**
5-(4-isoquinolinyl)-7,8-dimethoxy-1-methyl-1,3-dihydro-1,4-benzodiazepin-2-one, **17f**
7,8-dimethoxy-5-(3-hydroxymethylphenyl)-1-methyl-3-propyl-1,3-dihydro-2H-1,4-benzodiazepin-2-one, **17h**
5-(3-aminophenyl)-7,8-dimethoxy-1-methyl-1,3-dihydro-2H-1,4-benzodiazepin-2-one, **17i**
5-(3,4-dichlorophenyl)-7,8-dimethoxy-1-methyl-1,3-dihydro-1,4-benzodiazepin-2-one, **17j**
7,8-dimethoxy-1-methyl-5-(3-methylphenyl)-1,3-dihydro-1,4-benzodiazepin-2-one, **17k.**
5-(3-formylphenyl)-7,8-dimethoxy-1-methyl-1,3-dihydro-1,4-benzodiazepin-2-one, **17l**
5-[3-(benzylaminomethyl)phenyl]-7,8-dimethoxy-1-methyl-1,3-dihydro-2H-1,4-benzodiazepin-2-one hydrochloride, **17m**
N-[3-(7,8-dimethoxy-1-methyl-2-oxo-2,3-dihydro-1H-1,4-benzodiazepin-5-yl) phenyl]acetamide, **17n**
7,8-dimethoxy-1-methyl-5-(3,4-methylenedioxyphenyl)-1,3-dihydro-2H-1,4-benzodiazepin-2-one, **17o**
3-(6-bromo-7,8-dimethoxy-1-methyl-2-oxo-2,3-dihydro-1*H*-1,4-benzodiazepin-5-yl)benzonitrile, **24b**
3-(7,8-dimethoxy-1-methyl-2-oxo-6-phenyl-2,3-dihydro-1*H*-1,4-benzodiazepin-5-yl)benzonitrile, **25b**
*tert*-butyl-3-[5-(cyanophenyl)-(7,8-dimethoxy-1-methyl-2-oxo-2,3-dihydro-1*H*-1,4-benzodiazepin-6-yl)phenyl]prop-2-ynylcarbamate, **25e**
[6-(3-aminoethynyl)-7,8-dimethoxy-1-methyl-2-oxo-2,3-dihydro-1*H*-1,4-benzodiazepin-5-yl]benzonitrile, **25g**
3-(8-methoxy-2-oxo-2,3-dihydro-1H-1,4-benzodiazepin-5-yl)benzonitrile, **28a**
3-(7-methoxy-2-oxo-2,3-dihydro-1H-1,4-benzodiazepin-5-yl)benzonitrile, **28c**
3-(6,8-dimethoxy-2-oxo-2,3-dihydro-1*H*-1,4-benzodiazepin-5-yl)benzonitrile, **28g**
3-(8-methoxy-1-methyl-2-oxo-2,3-dihydro-1H-1,4-benzodiazepin-5-yl)benzonitrile, **29a**
3-(6,8-dimethoxy-1-methyl-2-oxo-2,3-dihydro-1*H*-1,4-benzodiazepin-5-yl)benzonitrile, **29b**
3-(7,8-dimethoxy-1-methyl-2-oxo-2,3-dihydro-1H-1,4-benzodiazepin-5-yl)methyl benzoate, **34a**
3-(7,8-dimethoxy-1-methyl-2-oxo-2,3-dihydro-1H-1,4-benzodiazepin-5-yl)benzoic acid, **35a**
3-(7,8-dimethoxy-1-methyl-2-oxo-2,3-dihydro-1H-1,4-benzodiazepin-5-yl)N-isopropylbenzamide, **36a**
N-benzyl-3-(7,8-dimethoxy-1-methyl-2-oxo-2,3-dihydro-1H-1,4-benzodiazepin-5-yl) benzamide, **36b**
N-(6-amino-6-oxohexyl)-3-(7,8-dimethoxy-1-methyl-2-oxo-2,3-dihydro-1H-1,4-benzodiazepin-5-yl)benzamide, **36c**
3-(7,8-dimethoxy-1-methyl-2-oxo-2,3-dihydro-1H-1,4-benzodiazepin-5-yl)-N,N-dimethylbenzamide **36d**
5- {3-[(4-benzylpyperazin-1-yl)carbonyl]phenyl}7,8-dimethoxy-1-methyl-2-oxo-2,3-dihydro-1H-1,4-benzodiazepin-2-one, **36e**
3-(7,8-dimethoxy-1-methyl-2-oxo-2,3-dihydro-1H-1,4-benzodiazepin-5-yl)-N-(3-phenylpropyl)benzamide, **36f.**

12. The compound according to claim 1, wherein said compound is selected from the following compounds :
7,8-dimethoxy-1-methyl-[5-(3-trifluoromethyl)phenyl]-1,3-dihydro-2*H*-1,4-benzodiazepin-2-one, **4p**
3-(7,8-dimethoxy-1-methyl-2-oxo-2,3-dihydro-1*H*-1,4-benzodiazepin-5-yl)benzamide, **5a**
3-(6-bromo-7,8-dimethoxy-1-methyl-2-oxo-2,3-dihydro-1*H*-1,4-benzodiazepin-5-yl)benzamide, **5b**
*tert*-butyl-3-[3-(7,8-dimethoxy-1-methyl-2-oxo-2,3-dihydro-1*H*-1,4-benzodiazepin-5-yl)phenyl]propynylcarbamate, **6a**
7,8-dimethoxy-5-(3'-hex-1-ynylphenyl)-1-*N*-methyl-1,3-dihydro-2*H*-1,4-benzodiazepin-2-one, **6b**
6-[3-(7,8-dimethoxy-1-methyl-2-oxo-2,3-dihydro-1*H*-1,4-benzodiazepin-5-yl)phenyl]hex-5-ynenitrile, **6d**
7,8-dimethoxy-5-(3'-hexylphenyl)-1-*N*-methyl-1,3-dihydro-2*H*-1,4-benzodiazepin-2-one, **6e**
5-(4'-chloro-1,1'-biphenyl-3-yl)-7,8-dimethoxy-1-methyl-1,3-dihydro-2H-1,4-benzodiazepin-2-one, **6j**
3'-(7,8-dimethoxy-1-methyl-2-oxo-2,3-dihydro-1H-1,4-benzodiazepin-5-yl)-1,1'-biphenyl-4-carbonitrile, **6m**
3'-(7,8-dimethoxy-1-methyl-2-oxo-2,3-dihydro-1H-1,4-benzodiazepin-5-yl)-1,1'-biphenyl-4-carboxamide, **6n**
7,8-dimethoxy-1-methyl-5-[3-(4-phenyl-1, 3-thiazol-2-yl)phenyl]-1,3-dihydro-2H-1,4-benzodiazepin-2-one, **9b**
7,8-dimethoxy-1-methyl-5-(3-pyridyl)-1,3-dihydro-1,4-benzodiazepin-2-one, **17b.**

13. The compound according to claim 1, wherein said compound is the following
3-(7,8-dimethoxy-1-methyl-2-oxo-2,3-dihydro-1*H*-1,4-benzodiazepin-5-yl)benzamide, **5a**

14. A pharmaceutical composition comprising at least one compound such as defined in one of claims 1 to 13 and a pharmaceutically acceptable vehicle or excipient.

15. Use of a compound such as defined in any one of claims 1 to 13 for preparing a pharmaceutical composition for treating pathologies involving the central nervous system.

16. Use according to the previous claim, wherein the pathology is selected in the group consisting of depression, schizophrenia, anxiety, bipolar disorder, attention deficit disorders, sleep disorders, obsessive compulsive disorder, fibromyalgia, Tourette's syndrome, pharmacodependence, epilepsy, Alzheimer's disease, Parkinson's disease, amyotrophic lateral sclerosis, multiple sclerosis, obesity and Lewy body dementia.

17. Use of a compound such as defined in any one of claims 1 to 13 for preparing a pharmaceutical composition for treating pathologies involving the peripheral nervous system, pathologies due to or involving dysfunctions of prolactin secretion, selected from restless legs syndrome, or rheumatismal, allergic or auto-inflammatory disorders, selected from rheumatoid arthritis, rhinitis and asthma.

18. Use of a compound such as defined in any one of claims 1 to 13 for preparing a pharmaceutical composition for treating disorders of the central or peripheral nervous system, of chronic or acute nature.

19. Use of a compound such as defined in any one of claims 1 to 13 for preparing a pharmaceutical composition for treating memory impairment or cognitive impairment, in particular mild cognitive impairment.

20. Use of a compound such as defined in any one of claims 1 to 13 for preparing a pharmaceutical composition for treating neurodegenerative diseases.

21. Use of a compound such as defined in any one of claims 1 to 13 for preparing a pharmaceutical composition for treating obesity.

## Patentansprüche

1. Verbindung der allgemeinen Formel (I): 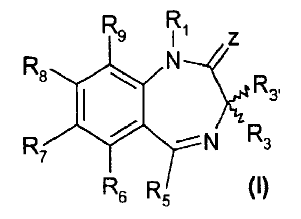 in der:
- Z für ein Sauerstoffatom, Schwefelatom oder einen NR₂-Rest steht,
- R₁ ein Wasserstoffatom oder eine nicht substituierte (C₁-C₆)-Alkylgruppe ist,
- R₂ ein Wasserstoffatom, eine (C₁-C₆)-Alkylgruppe, eine (C₆-C₁₈)-Arylgruppe oder eine (C₁-C₆)-Alkyl-(C₆-C₁₈)-Aryl- oder (C₆-C₁₈)-Aryl-(C₁-C₄)-Alkylgruppe ist,
wobei R₁ und R₂ gegebenenfalls zusammen eine unverzweigte oder verzweigte Kohlenwasserstoffkette mit 2 bis 6 Kohlenstoffatomen bilden können, die gegebenenfalls eine weitere oder mehrere weitere Doppelbindungen umfasst und/oder gegebenenfalls von einem Sauerstoff-, Schwefel- oder Stickstoffatom unterbrochen ist,
- R₃ und R₃, identisch oder verschieden sind und für ein Wasserstoffatom, eine (C₁-C₁₂)-Alkyl-, (C₃-C₆)-Cycloalkyl-, (C₆-C₁₈)-Aryl-, (C₆-C₁₈)-Aryl-(C₁-C₄)-Alkyl-, (C₁-C₁₂)-Alkyl-(C₆-C₁₈)-Arylgruppe oder einen 1 bis 3 Heteroatome umfassenden aromatischen oder nichtaromatischen Heterocyclus mit (C₅-C₁₈), eine NO₂-, CF₃-, CN-, NR'R"-, SR'-, OR'-, COOR'-, CONR'R"- oder NHCOR'R"-Gruppe stehen, wobei R' und R" unabhängig voneinander aus einem Wasserstoffatom, einer (C₁-C₆)-Alkyl-, (C₃-C₆)-Cycloalkyl-, (C₆-C₁₂)-Arylgruppe und einem 1 bis 3 Heteroatome umfassenden aromatischen oder nichtaromatischen Heterocyclus mit (C₅-C₁₂), ausgewählt sind;
- R₅ für eine Phenylgruppe, die wenigstens in Position 3 substituiert ist, eine Naphtylgruppe, einen 1 bis 3 Heteroatome umfassenden aromatischen oder nichtaromatischen Heterocyclus mit (C₅-C₁₈) steht, der aus einem Pyridyl-, Isochinolyl-, Chinolyl- und Piperazinylrest ausgewählt ist, unter den Bedingungen, dass, wenn R₅ eine in Position 6 substituierte Naphtylgruppe ist, letztere dann nicht an den Rest des Moleküls in Position 2 gebunden ist, oder wenn R₅ eine Pyridylgruppe ist, sie dann nicht an den Rest des Moleküls in Position 4 gebunden ist, oder wenn R₅ eine 1,2,3,4-Tetrahydroisochinolylgruppe ist, sie dann nicht an den Rest des Moleküls in Position 2 gebunden ist,
wenn R₅ für eine Phenylgruppe steht, die wenigstens in Position 3 substituiert ist, dann ist besagter Substituent ausgewählt aus: einem Alkyl-, Halogenalkyl-, Cycloalkyl-, Alkenyl-, Alkinyl-, Aralkyl-, Arylrest, Heterocyclus, Heterocycloalkylrest, einer OH-, =O-, NO₂-, NH₂-, CN-, CF₃-, COR'-, COOR'-, (C₁-C₆)-Alkoxy-, (Di)(C₁-C₆)-Alkylamino-, NHCOR'-, CONR'R"-Gruppe, in denen R' und R" wie oben definiert sind, einer CHO-, CONH₂-, Phenylgruppe, gegebenenfalls substituiert, insbesondere mit einem Acetylrest, mit einem Halogenatom (Cl), mit einer CONH₂-Gruppe oder mit einer CN-Gruppe, einer Prop-1-inylgruppe, gegebenenfalls substituiert, insbesondere mit einem Benzyloxy- oder tert.-Butylcarbamatrest, einer Hex-1-inylgruppe, gegebenenfalls substituiert, insbesondere mit einer CN- oder NH₂-Gruppe, einer Pentylgruppe, gegebenenfalls substituiert, insbesondere mit einer CONH₂-Gruppe, einer Hexyl-, Piperidinylgruppe, gegebenenfalls substituiert, insbesondere mit einem Prop-1-inylrest, einer Benzylaminomethyl-, Acetamid (CH₃CONH)-, Aminomethyl-, NH₂CS-, 4-Phenyl-1-, 3-Thiazol-2-yl-, -CONHBenzyl-, -COOEthyl-, -CONHiPropyl-, -CONH-(CH₂)n-CONH₂- (wobei n für eine ganze Zahl von 1 bis 6 steht), -CONR'R"-Gruppe, wobei R' und R", die identisch oder verschieden sind, für einen (C₁-C₆)-Alkylrest oder ein Wasserstoffatom stehen, -(4-Benzylpiperazin-1-yl)carbonyl-, -CONH-(CH₂)n-Phenyl- (wobei n für eine ganze Zahl von 1 bis 6 steht), einer Imidazolyl-, Piperazinylgruppe, gegebenenfalls substituiert, insbesondere mit einem Phenylrest,
- R₇ und R₈, unabhängig voneinander, für eine OR₁₀-Gruppe stehen, in der R₁₀ eine (C₁-C₆)-Alkylgruppe, vorzugsweise eine Ethyl- oder Methylgruppe, vorteilhafterweise Methylgruppe, ist oder R₇ und R₈ beide für eine Ethoxy- oder Methoxygruppe, vorteilhafterweise Methoxygruppe, stehen oder der eine Rest für ein Wasserstoffatom und der andere für eine Ethoxy- oder Methoxygruppe, vorteilhafterweise Methoxygruppe, steht,
- R₆ aus einem Wasserstoffatom, einem Halogenatom, einem Alkyl-, Cycloalkyl-, Alkenyl-, Alkinylrest, einer Aryl-, Aralkylgruppe, einem Heterocyclus, einer Heterocycloalkyl- und OR₁₀-Gruppe ausgewählt ist, wobei R₁₀ wie oben definiert ist,
wobei die Alkyl-, Cycloalkyl-, Alkenyl-, Alkinyl-, Aralkyl-, Aryl-, Phenyl-, Naphthylgruppe, der Heterocyclus, die Heterocycloalkylgruppe oder die oben definierte Kohlenwasserstoffkette mit einem oder mehreren identischen oder verschiedenen Substituenten gegebenenfalls substituiert ist, die vorzugsweise aus einem Halogenatom, einer Alkyl-, Halogenalkyl-, Cycloalkyl-, Alkenyl-, Alkinyl-, Aralkylrest, Heterocyclus, Heterocycloalkylrest, einer OH-, =O-, NO₂-, NH₂-, CN-, CF₃-, COR'-, COOR'-, (C₁-C₆)-Alkoxy-, (Di)(C₁-C₆)-Alkylamino-, NHCOR'- und CONR'R"-Gruppe ausgewählt sind, in denen R' und R" wie oben definiert sind, wobei die Substituenten ebenfalls substituiert sein können,
- R₉ ein Wasserstoffatom ist,
und die Salze der Verbindung der Formel (I),
außer den Verbindungen der Formel (I), in der
- R₁ ein Alkylrest ist, R₃ und R'₃ Wasserstoffatome sind, R₆ ein Wasserstoffatom ist, R₅ ein Phenylrest ist, der wenigstens in Position 3 mit einer Methoxygruppe substituiert ist,
- R₁ ein Alkylrest oder ein Wasserstoffatom ist, R₃ und R'₃ Wasserstoffatome sind, R₆ ein Wasserstoffatom ist, R₅ ein Phenylrest ist, der nur in Position 3 mit einem Chlor- oder Bromatom substituiert ist,
- R₁ ein Alkylrest ist, R₃ und R'₃ Wasserstoffatome sind, R₆ ein Wasserstoffatom ist, R₅ ein Phenylrest ist, der wenigstens in Position 3 mit einer CH₂OH-Gruppe substituiert ist,
- R₁ ein Wasserstoffatom ist, R₃ und R'₃ Wasserstoffatome sind, R₆ ein Wasserstoffatom ist, R₅ ein Phenylrest ist, der nur in Position 3 mit einer CF₃-Gruppe substituiert ist,
- R₁ ein Alkylrest ist, R₃ und R'₃ Wasserstoffatome sind, R₆ ein Wasserstoffatom ist, R₅ ein Phenylrest ist, der in Positionen 3 und 5 mit einer CF₃-Gruppe substituiert ist,
- R₁ ein Alkylrest ist, R₃ und R'₃ Wasserstoffatome sind, R₆ ein Wasserstoffatom ist, R₇ und R₈ Methoxyreste sind, R₅ ein Phenylrest ist, der in Position 3 mit einer Phenylgruppe substituiert ist,
- R₁ ein Alkylrest ist, R₃ und R'₃ Wasserstoffatome sind, R₆ ein Wasserstoffatom ist, R₇ und R₈ Methoxyreste sind, R₅ ein Phenylrest ist, der in Position 3 mit einer Phenylethinylgruppe substituiert ist.

2. Verbindung der Formel (I) gemäß Anspruch 1, in der R₅ ein Phenylrest ist, der wenigstens in Position 3 substituiert ist.

3. Verbindung der Formel (I) gemäß Anspruch 2, in der die substituierenden Gruppen ausgewählt sein können aus: CHO, CN, CONH₂, CF₃, NH₂, Halogenatom (Cl), (C₁-C₆)-Alkyl, Phenyl, gegebenenfalls substituiert, insbesondere mit einem Acetylrest, mit einem Halogenatom (Cl), mit einer CONH₂-Gruppe oder mit einer CN-Gruppe, Prop-1-inyl, gegebenenfalls substituiert, insbesondere mit einem Benzyloxy- oder tert.-Butylcarbamatrest, Hex-1-inyl, gegebenenfalls substituiert, insbesondere mit einer CN- oder NH₂-Gruppe, Pentyl, gegebenenfalls substituiert, insbesondere mit einer CONH₂-Gruppe, Hexyl, Piperidinyl, gegebenenfalls substituiert, insbesondere mit einem Prop-1-inylrest, Benzylaminomethyl, Acetamid (CH₃CONH), Aminomethyl, NH₂CS-, 4-Phenyl-1, 3-Thiazol-2-yl, -CONHBenzyl, -COOEthyl, -CONHiPropyl, -CONH-(CH₂)n-CONH₂ (wobei n für eine ganze Zahl von 1 bis 6 steht), -CONR'R", wobei R' und R", die identisch oder verschieden sind, für einen (C₁-C₆)-Alkylrest oder ein Wasserstoffatom stehen, -(4-Benzylpiperazin-1-yl)carbonyl, -CONH-(CH₂)n-Phenyl (wobei n für eine ganze Zahl von 1 bis 6 steht), Imidazolyl, Piperazinyl, gegebenenfalls substituiert, insbesondere mit einem Phenylrest.

4. Verbindung der Formel (I) gemäß einem der Ansprüche 1 bis 3, in der R₅ ein Phenylrest ist, der in den Positionen 3 und 4 substituiert ist, insbesondere mit einer Kohlenwasserstoffkette, umfassend gegebenenfalls wenigstens ein Heteroatom, wie beispielsweise Sauerstoff, wie die Methylendioxykette (-O-CH2-O-), die einen Ring mit dem Phenylrest bildet, an den sie gebunden ist.

5. Verbindung der Formel (I) gemäß Anspruch 1, in der R₅ der 3-Pyridyl-, 4-Isochinolyl-, Piperazinylrest ist, der gegebenenfalls insbesondere in der Position 4 mit einer Arylgruppe, wie Phenyl, substituiert ist.

6. Verbindung der Formel (I) gemäß Anspruch 1, in der Z für ein Schwefelatom oder -NR₂ steht, wobei insbesondere R₂ mit R₁ einen Imidazolring bildet.

7. Verbindung der Formel (I) gemäß Anspruch 1, 2 oder 3, in der:
- Z ein Sauerstoffatom ist und/oder
- R₆ für ein Wasserstoffatom, ein Halogenatom, einen Phenylrest, eine (C₁-C₆)-Alkylgruppe oder eine OR₁₀-Gruppe steht, in der R₁₀ eine (C₁-C₆)-Alkylgruppe, vorzugsweise eine Ethyl- oder Methylgruppe, ist und/oder
- R₃ und R₃', identisch oder verschieden sind und für ein Wasserstoffatom stehen, und/oder
- R₁ eine nicht substituierte (C₁-C₆)-Alkylgruppe ist.

8. Verbindung der Formel (I) gemäß einem der Ansprüche 1 bis 7, in der R₁ für ein Wasserstoffatom oder eine nicht substituierte (C₁-C₃)-Alkylgruppe steht.

9. Verbindung der Formel (I) gemäß einem der Ansprüche 1 bis 8, in der R₅ eine Phenylgruppe ist und substituiert ist mit:
(a) einer oder mehreren OR'-Gruppen, insbesondere Methoxy- oder Ethoxygruppe, oder
(b) einer COR'-Gruppe, insbesondere Acetyl- oder Aldehydgruppe, oder
(c) einer CONR'R"-Gruppe, insbesondere CONH₂-Gruppe, oder
(d) einer CN-Gruppe oder
(e) einer Trifluormethylgruppe oder
(f) einer Alkylgruppe, zum Beispiel Methylgruppe, oder Alkinylgruppe, zum Beispiel Hexinyl- oder Propinylgruppe, oder
(g) einer Arylgruppe oder einem Heterocyclus, insbesondere einer Phenyl-, Furyl-, Pyridyl-, Piperidin-, Thiazol- oder Thienylgruppe, wobei besagtes Aryl oder besagter Heterocyclus gegebenenfalls selbst mit einer oder mehreren Gruppen substituiert ist, die vorzugsweise aus den Gruppen (a)-(g) ausgewählt sind.

10. Verbindung der Formel (I) gemäß einem der Ansprüche 1 bis 9, in der wenigstens eine der Gruppen R₇ und R₈, vorteilhafterweise beide, für einen Methoxyrest steht.

11. Verbindung gemäß Anspruch 1, wobei die Verbindung aus den folgenden Verbindungen ausgewählt ist:
3-(7,8-Dimethoxy-2-oxo-2,3-dihydro-1H-1,4-benzodiazepin-5-yl)benzonitril, **3a**
3-(7,8-Dimethoxy-1-methyl-2-oxo-2,3-dihydro-1*H*-1,4-benzodiazepin-5-yl)benzonitril, **4a**
3-(1-Ethyl-7,8-dimethoxy-2-oxo-2,3-dihydro-1H-1,4-benzodiazepin-5-yl)benzonitril, **4k**
3-(7,8-Dimethoxy-1-propyl-2-oxo-2,3-dihydro-1H-1,4-benzodiazepin-5-yl)benzonitril, **4l**
7,8-Dimethoxy-1-methyl-5-[(3-trifluormethyl)phenyl]-1,3-dihydro-2*H*-1,4-benzodiazepin-2-on, **4p**
7,8-Dimethoxy-1-ethyl-5-[3-(trifluormethyl)phenyl]-1,3-dihydro-2H-1,4-benzodiazepin-2-on, **4q**
5-[3-(Trifluormethyl)phenyl]-7,8-dimethoxy-1-*n*-propyl-1,3-dihydro-1,4-benzodiazepin-2-on, **4r**
3-(7,8-Dimethoxy-1-methyl-2-oxo-2,3-dihydro-1*H*-1,4-benzodiazepin-5-yl)benzamid, **5a**
3-(6-Brom-7,8-dimethoxy-1-methyl-2-oxo-2,3-dihydro-1*H*-1,4-benzodiazepin-5-yl)benzamid, **5b**
3-(7,8-Dimethoxy-1-methyl-2-oxo-6-phenyl-2,3-dihydro-1*H*-1,4-benzodiazepin-5-yl)benzamid, **5c**
3-(7,8-Dimethoxy-2-oxo-2,3-dihydro-1H-1,4-benzodiazepin-5-yl)benzamid, **5e**
3-(7,8-Dimethoxy-1-propyl-2-oxo-2,3-dihydro-1H-1,4-benzodiazepin-5-yl)benzamid, **5f**
3-(1-Ethyl-7,8-dimethoxy-2-oxo-2,3-dihydro-1H-1,4-benzodiazepin-5-yl)benzamid, **5g**
3-(7,8-Dimethoxy-1,3-dimethyl-2-oxo-2,3-dihydro-1H-1,4-benzodiazepin-5-yl)benzamid, **5j**
3-[3-(3,4-Dichlorbenzyl)-7,8-dimethoxy-1-methyl-2-oxo-2,3-dihydro-1H-1,4-benzodiazepin-5-yl]benzamid, **5k**
3-(8-Methoxy-1-methyl-2-oxo-2,3-dihydro-1H-1,4-benzodiazepin-5-yl)benzamid, **5l**
3-(6,8-Dimethoxy-2-oxo-2,3-dihydro-1*H*-1,4-benzodiazepin-5-yl)benzamid, **5n**
3-(6,8-Dimethoxy-1-methyl-2-oxo-2,3-dihydro-1*H*-1,4-benzodiazepin-5-yl)benzamid, **5o**
*tert*.-Butyl-3-[3-(7,8-dimethoxy-1-methyl-2-oxo-2,3-dihydro-1*H*-1,4-benzodiazepin-5-yl)phenyl]propinylcarbamat, **6a**
7,8-Dimethoxy-5-(3'-hex-1-inylphenyl)-1-*N*-methyl-1,3-dihydro-2*H*-1,4-benzodiazepin-2-on, **6b**
7,8-Dimethoxy-1-methyl-5-[3-(3-piperidin-1-ylprop-1-inyl)phenyl]-1,3-dihydro-2*H*-1,4-benzodiazepin-2-on, **6c**
6-[3-(7,8-Dimethoxy-1-methyl-2-oxo-2,3-dihydro-1*H*-1,4-benzodiazepin-5-yl)phenyl]hex-5-innitril, **6d**
7,8-Dimethoxy-5-(3'-hexylphenyl)-1-*N*-methyl-1,3-dihydro-2*H*-1,4-benzodiazepin-2-on, **6e**
5-[3-(3-Aminopropyl)phenyl-7,8-dimethoxy-1-methyl-1,3-dihydro-2H-1,4-benzodiazepin-2-on-trifluoracetat, **6h**
6-[3-(7,8-Dimethoxy-1-methyl-2-oxo-2,3-dihydro-1*H*-1,4-benzodiazepin-5-yl)phenyl]hexanamid, **6i**
5-(4'-Chlor-1,1'-biphenyl-3-yl)-7,8-dimethoxy-1-methyl-1,3-dihydro-2H-1,4-benzodiazepin-2-on, **6j**
5- {3-[3-(Benzyloxy)prop-1-inyl]phenyl}-1-ethyl-7,8-dimethoxy-1,3-dihydro-2H-1,4-benzodiazepin-2-on, **6k**
3'-(7,8-Dimethoxy-1-methyl-2-oxo-2,3-dihydro-1H-1,4-benzodiazepin-5-yl)-1,1'-biphenyl-3-carbonitril, **6l**
3'-(7,8-Dimethoxy-1-methyl-2-oxo-2,3-dihydro-1H-1,4-benzodiazepin-5-yl)-1,1'-biphenyl-4-carbonitril, **6m**
3'-(7,8-Dimethoxy-1-methyl-2-oxo-2,3-dihydro-1H-1,4-benzodiazepin-5-yl)-1,1'-biphenyl-4-carboxamid, **6n**
3'-(7,8-Dimethoxy-1-methyl-2-oxo-2,3-dihydro-1H-1,4-benzodiazepin-5-yl)-1,1'-biphenyl-3-carboxamid, **6o**
3-[3-(3,4-Dichlorbenzyl)-7,8-dimethoxy-1-methyl-2-oxo-2,3-dihydro-1H-1,4-benzodiazepin-5-yl]benzonitril, **7b**
7,8-Dimethoxy-1,3-dimethyl-5-(3-trifluormethylphenyl)-1,3-dihydro-2H-1,4-benzodiazepin-2-on, **7c**
3-(7,8-Dimethoxy-1,3-dimethyl-2-oxo-2,3-dihydro-1H-1,4-benzodiazepin-5-yl)benzonitril, **7d**
5-[3-(Aminomethyl)phenyl]-7,8-dimethoxy-1-methyl-1,3-dihydro-2H-1,4-benzodiazepin-2-on, **8a**
N-[3-(7,8-Dimethoxy-1-methyl-2-oxo-2,3-dihydro-1H-1,4-benzodiazepin-5-yl)benzyl]acetamid, **8b**
3-(7,8-Dimethoxy-1-methyl-2-oxo-2,3-dihydro-1H-1,4-benzodiazepin-5-yl)thiobenzamid, **9a**
7,8-Dimethoxy-1 -methyl-5-[3-(4-phenyl-1,3-thiazol-2-yl)phenyl]-1,3-dihydro-2H-1,4-benzodiazepin-2-on, **9b**
5-(3-Cyanophenyl)-7,8-dimethoxy-1,3-dihydro-2H-1,4-benzodiazepin-2-thion, **10d**
3-(7,8-Dimethoxy-2-methylamino-1,3-dihydro-3H-1,4-benzodiazepin-5-yl)benzonitril, **12a**
7,8-Dimethoxy-1-methyl-5-(3-pyridyl)-1,3-dihydro-1,4-benzodiazepin-2-on, **17b**
7,8-Dimethoxy-1-methyl-5-(3-nitrophenyl)-1,3-dihydro-1,4-benzodiazepin-2-on, **17c**
5-(7,8-Dimethoxy-1-methyl-2-oxo-2,3-dihydro-1H-1,4-benzodiazepin-5-yl)-2-benzonitril, **17d**
5-(3-Acetylphenyl)-7,8-dimethoxy-1-methyl-1,3-dihydro-1,4-benzodiazepin-2-on, **17e**
5-(4-Isochinolyl)-7,8-dimethoxy-1-methyl-1,3-dihydro-1,4-benzodiazepin-2-on, **17f**
7,8-Dimethoxy-5-(3-hydroxymethylphenyl)-1-methyl-3-propyl-1,3-dihydro-2H-1,4-benzodiazepin-2-on, **17h**
5-(3-Aminophenyl)-7,8-dimethoxy-1-methyl-1,3-dihydro-1H-1,4-benzodiazepin-2-on, **17i**
5-(3,4-Dichlorphenyl)-7,8-dimethoxy-1-methyl-1,3-dihydro-1,4-benzodiazepin-2-on, **17j**
7,8-Dimethoxy-1-methyl-5-(3-methylphenyl)-1,3-dihydro-1,4-benzodiazepin-2-on, **17k**
5-(3-Formylphenyl)-7,8-dimethoxy-1-methyl-1,3-dihydro-1,4-benzodiazepin-2-on, **17l**
5-[3-(Benzylaminomethyl)phenyl]-7,8-dimethoxy-1-methyl-1,3-dihydro-2H-1,4-benzodiazepin-2-on Hydrochlorid, **17m**
N-[3-(7,8-Dimethoxy-1-methyl-2-oxo-2,3-dihydro-1H-1,4-benzodiazepin-5-yl)phenyl]acetamid, **17n**
7,8-Dimethoxy-1-methyl-5-(3,4-methylendioxyphenyl)-1,3-dihydro-2H-1,4-benzodiazepin-2-on, **17o**
3-(6-Brom-7,8-dimethoxy-1-methyl-2-oxo-2,3-dihydro-1*H*-1,4-benzodiazepin-5-yl)benzonitril, **24b**
3-(7,8-Dimethoxy-1-methyl-2-oxo-6-phenyl-2,3-dihydro-1*H*-1,4-benzodiazepin-5-yl)benzonitril, **25b**
*tert*.-Butyl-3-[5-(3-cyanophenyl)-(7,8-dimethoxy-1-methyl-2-oxo-2,3-dihydro-1*H*-1,4-benzodiazepin-6-yl)]prop-2-inylcarbamat, **25e**
3-[6-(3-Aminoethinyl)-7,8-dimethoxy-1-methyl-2-oxo-2,3-dihydro-1*H*-1,4-benzodiazepin-5-yl]benzonitril, **25g**
3-(8-Methoxy-2-oxo-2,3-dihydro-1H-1,4-benzodiazepin-5-yl)benzonitril, **28a**
3-(7-Methoxy-2-oxo-2,3-dihydro-1H-1,4-benzodiazepin-5-yl)benzonitril, **28c**
3-(6,8-Dimethoxy-2-oxo-2,3-dihydro-1*H*-1,4-benzodiazepin-5-yl)benzonitril, **28g**
3-(8-Methoxy-1-methyl-2-oxo-2,3-dihydro-1H-1,4-benzodiazepin-5-yl)benzonitril, **29a**
3-(6,8-Dimethoxy-1-methyl-2-oxo-2,3-dihydro-1*H*-1,4-benzodiazepin-5-yl)benzonitril, **29b**
3-(7,8-Dimethoxy-1-methyl-2-oxo-2,3-dihydro-1H-1,4-benzodiazepin-5-yl)methylbenzoat, **34a**
3-(7,8-Dimethoxy-1-methyl-2-oxo-2,3-dihydro-1H-1,4-benzodiazepin-5-yl)benzoesäure, **35a**
3-(7,8-Dimethoxy-1-methyl-2-oxo-2,3-dihydro-1H-1,4-benzodiazepin-5-yl)N-isopropylbenzamid, **36a**
N-Benzyl-3-(7,8-dimethoxy-1-methyl-2-oxo-2,3-dihydro-1H-1,4-benzodiazepin-5-yl)benzamid, **36b**
N-(6-Amino-6-oxohexyl)-3-(7,8-dimethoxy-1-methyl-2-oxo-2,3-dihydro-1H-1,4-benzodiazepin-5-yl)benzamid, **36c**
3-(7,8-Dimethoxy-1-methyl-2-oxo-2,3-dihydro-1H-1,4-benzodiazepin-5-yl)-N,N-dimethylbenzamid, **36d**
5- {3-[(4-Benzylpiperazin-1-yl)carbonyl]phenyl}7,8-dimethoxy-1-methyl-2-oxo-2,3-dihydro-1H-1,4-benzodiazepin-2-on, **36e**
3-(7,8-Dimethoxy-1-methyl-2-oxo-2,3-dihydro-1H-1,4-benzodiazepin-5-yl)-N-(3-phenylpropyl)benzamid, **36f.**

12. Verbindung gemäß Anspruch 1, wobei die Verbindung aus den folgenden Verbindungen ausgewählt ist:
7,8-Dimethoxy-1-methyl-5-[(3-trifluormethyl)phenyl]-1,3-dihydro-2*H*-1,4-benzodiazepin-2-on, **4p**
3-(7,8-Dimethoxy-1-methyl-2-oxo-2,3-dihydro-1*H*-1,4-benzodiazepin-5-yl)benzamid, **5a**
3-(6-Brom-7,8-dimethoxy-1-methyl-2-oxo-2,3-dihydro-1*H*-1,4-benzodiazepin-5-yl)benzamid, **5b**
*tert*.-Butyl-3-[3-(7,8-dimethoxy-1-methyl-2-oxo-2,3-dihydro-1*H*-1,4-benzodiazepin-5-yl)phenyl]propinylcarbamat, **6a**
7,8-Dimethoxy-5-(3'-hex-1-inylphenyl)-1-*N*-methyl-1,3-dihydro-2*H*-1,4-benzodiazepin-2-on, **6b**
6-[3-(7,8-Dimethoxy-1-methyl-2-oxo-2,3-dihydro-1*H*-1,4-benzodiazepin-5-yl)phenyl]hex-5-innitril, **6d**
7,8-Dimethoxy-5-(3'-hexylphenyl)-1-*N*-methyl-1,3-dihydro-2*H*-1,4-benzodiazepin-2-on, **6e**
5-(4'-Chlor-1,1'-biphenyl-3-yl)-7,8-dimethoxy-1-methyl-1,3-dihydro-2H-1,4-benzodiazepin-2-on, **6j**
3'-(7,8-Dimethoxy-1-methyl-2-oxo-2,3-dihydro-1H-1,4-benzodiazepin-5-yl)-1,1'-biphenyl-4-carbonitril, **6m**
3'-(7,8-Dimethoxy-1-methyl-2-oxo-2,3-dihydro-1H-1,4-benzodiazepin-5-yl)-1,1'-biphenyl-4-carboxamid, **6n**
7,8-Dimethoxy-1-methyl-5-[3-(4-phenyl-1,3-thiazol-2-yl)phenyl]-1,3-dihydro-2H-1,4-benzodiazepin-2-on, **9b**
7,8-Dimethoxy-1-methyl-5-(3-pyridyl)-1,3-dihydro-1,4-benzodiazepin-2-on, **17b.**

13. Verbindung gemäß Anspruch 1, wobei die Verbindung die folgende ist:
3-(7,8-Dimethoxy-1-methyl-2-oxo-2,3-dihydro-1*H*-1,4-benzodiazepin-5-yl)benzamid, **5a**

14. Pharmazeutische Zusammensetzung, umfassend wenigstens eine Verbindung, wie in einem der Ansprüche 1 bis 13 definiert, und ein pharmazeutisch verträgliches Vehikulum oder einen pharmazeutisch verträglichen Arzneimittelträger.

15. Verwendung einer Verbindung, wie gemäß einem der Ansprüche 1 bis 13 definiert, für die Herstellung einer pharmazeutischen Zusammensetzung für die Behandlung von Krankheiten, die das Zentralnervensystem betreffen.

16. Verwendung gemäß dem vorhergehenden Anspruch, **dadurch gekennzeichnet, dass** die Krankheit aus Depression, Schizophrenie, Angst, bipolarer Störung, Aufmerksamkeitsdefizitstörungen, Schlafstörungen, Zwangsstörungen, Fibromyalgie, Tourette Syndrom, Arzneimittelabhängigkeit, Epilepsie, Alzheimer Krankheit, Parkinson Krankheit, amyotropher Lateralsklerose, multipler Sklerose, Obesität und Lewy-Körperchen-Demenz ausgewählt ist.

17. Verwendung einer Verbindung, wie gemäß einem der Ansprüche 1 bis 13 definiert, für die Herstellung einer pharmazeutischen Zusammensetzung für die Behandlung von Krankheiten, die das periphere Nervensystem mit einbeziehen, Krankheiten, die von Dysfunktionen der Prolaktin-Freisetzung hervorgerufen sind oder diese implizieren, ausgewählt aus dem Restless-Legs-Syndrom oder den rheumatischen, allergischen oder autoinflammatorischen Störungen, ausgewählt aus rheumatoider Arthritis, Rhinitis und Asthma.

18. Verwendung einer Verbindung, wie gemäß einem der Ansprüche 1 bis 13 definiert, für die Herstellung einer pharmazeutischen Zusammensetzung für die Behandlung von chronischen oder akuten Störungen des zentralen oder peripheren Nervensystems.

19. Verwendung einer Verbindung, wie gemäß einem der Ansprüche 1 bis 13 definiert, für die Herstellung einer pharmazeutischen Zusammensetzung für die Behandlung von Gedächtnisstörungen oder kognitiven Störungen, insbesondere von mäßigen kognitiven Störungen.

20. Verwendung einer Verbindung, wie gemäß einem der Ansprüche 1 bis 13 definiert, für die Herstellung einer pharmazeutischen Zusammensetzung für die Behandlung von neurodegenerativen Krankheiten.

21. Verwendung einer Verbindung, wie gemäß einem der Ansprüche 1 bis 13 definiert, für die Herstellung einer pharmazeutischen Zusammensetzung für die Behandlung der Obesität.
